(19) Europäisches Patentamt / European Patent Office / Office européen des brevets

(11) **EP 4 253 411 A1**

(12) **EUROPEAN PATENT APPLICATION**
published in accordance with Art. 153(4) EPC

(43) Date of publication:
04.10.2023 Bulletin 2023/40

(21) Application number: 21896926.9

(22) Date of filing: 23.11.2021

(51) International Patent Classification (IPC):
C07K 16/18 (2006.01)     C12N 15/13 (2006.01)
G01N 33/574 (2006.01)     A61K 39/395 (2006.01)
A61P 35/00 (2006.01)

(86) International application number:
PCT/CN2021/132218

(87) International publication number:
WO 2022/111425 (02.06.2022 Gazette 2022/22)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 24.11.2020 CN 202011332200

(71) Applicant: Sanyou Biopharmaceuticals Co., Ltd.
Shanghai 201114 (CN)

(72) Inventors:
• TAN, Yongcong
  Shanghai 201114 (CN)
• LANG, Guojun
  Shanghai 201114 (CN)
• LIU, Chanjuan
  Shanghai 201114 (CN)
• YAN, Xintian
  Shanghai 201114 (CN)

(74) Representative: Reitstötter Kinzebach
Patentanwälte
Sternwartstrasse 4
81679 München (DE)

Remarks:
The complete document including Reference
Table(s) and the Sequence Listing(s) can be
downloaded from the EPO website

(54) **MOLECULE SPECIFICALLY BINDING TO CLDN18.2**

(57) Provided are a molecule specifically binding to CLDN18.2, the molecule comprising an immunoglobulin single variable domain, a nucleic acid molecule encoding the molecule specifically binding to CLDN18.2, and an expression vector and a host cell for expressing the molecule specifically binding to CLDN18.2; further provided are a method for producing the molecule specifically binding to CLDN18.2 and use thereof for treating or preventing conditions associated with CLDN18.2.

FIG. 6a

EP 4 253 411 A1

**Description**

The present application claims priority to Chinese Application No. 202011332200.9 filed on November 24, 2020 and entitled with "MOLECULE SPECIFICALLY BINDING TO CLD18A2", the content of which is incorporated herein by reference in its entirety.

**Sequence Listing**

The present application contains a Sequence Listing, which is incorporated herein by reference in its entirety.

**Technical Field**

In general, the present application relates to antibodies. More specifically, the present application relates to a molecule specifically binding to CLDN18.2, a preparation method therefor and the use thereof.

**Background Art**

Cell junctions are structures connecting cells and are an important basis for the inter-association and interaction between adjacent cells in a multicellular organism. In general, there are four types of animal cell junctions: tight junctions, adherens junctions, gap junctions, and desmosomes/hemidesmosomes. Tight junctions, also referred to as occluding junctions, are structures formed between endothelial cells or epithelial cells and can prevent substances between tissues from diffusing through the intercellular spaces, and accordingly, the substances are allowed to enter the cells only by means of active transport. Structures of tight junctions are formed by means of intracellular and intercellular protein interactions among dozens of Claudin proteins, and the expression of these proteins has a certain tissue specificity. CLDN18 is an important one among the Claudin proteins present in tight junctions.

CLDN18 is a membrane protein having four transmembrane regions and contains two extracellular domains. There are two variants of CLDN18 in the human body, which are CLDN18.1 and CLDN18.2 (also called "CLD18A2"), respectively. The two are distributed in different tissues. The former is mainly expressed in pulmonary epithelial cells, and the latter is specifically expressed in gastric epithelial cells and is not expressed in gastric stem cells. CLDN18.1 and CLDN18.2 have an extremely high similarity in protein sequences, and the main difference between the two is in the N-terminus; the two differ by only eight amino acids in the first extracellular domain, and the C-terminal sequences are completely identical.

Antibody therapy is becoming one of the most promising methods for treating cancer in patients. CLDN18.2 has currently become a very potential action target for antibody drugs due to its expression specificity in tumour cells and normal tissues. However, since CLDN18.1 and CLDN18.2 have an extremely high sequence similarity, it is extremely difficult to develop antibodies that only target CLDN18.2 but not CLDN18.1. The German company Ganymed, as an early developer of antibody drugs against CLDN18.2 targets, develops IMAB362 which is a monoclonal antibody composed of two heavy chains and two light chains. However, so far, there have been no reports on single domain antibody drugs that target CLDN18.2 undergoing clinical studies.

Single domain antibody (sdAb for short) is an antibody composed of a single monomeric variable antibody domain (e.g., a heavy chain variable domain of an antibody). Like an intact antibody (e.g., IgG), a single domain antibody can selectively bind to a specific antigen. However, the molecular weight of a single domain antibody is much smaller than that of a common antibody consists of two heavy chains and two light chains. The first single domain antibody is engineered from a heavy chain antibody found in camelids (Hamers-Casterman C, Atarhouch T, Muyldermans S, Robinson G, Hamers C, Songa EB, Bendahman N, Hamers R (1993) Naturally occurring antibodies devoid of light chains. Nature 363(6428): 446-448.). The heavy chain antibody found in these camelids is also referred to as a VHH fragment. At present, most of the researches on single domain antibodies are based on heavy chain variable domains.

Although there are already monoclonal antibody drugs that target CLDN18.2 undergoing clinical studies, an urgent need still exists to continue with the development of antibodies that target CLDN18.2 as therapeutic agents. It is desirable in the art to develop a new antibody that targets CLDN18.2, particularly a single domain antibody that only specifically recognizes CLDN18.2 but does not recognize CLDN18.1.

**Summary of the Invention**

Broadly speaking, the present disclosure provides a novel compound of an antibody, a preparation method, a composition and a product thereof. The benefits provided by the present disclosure are widely applicable to the fields of treatment and diagnosis with antibodies. More particularly, the present disclosure provides a single domain antibody that targets CLDN18.2, a method for preparing the antibody, an expression vector and a host cell for expressing the antibody, etc. The antibody of the present disclosure provides a method for treating or preventing a condition associated with Claudin proteins, particularly CLDN18.2 and the use thereof.

The inventors have discovered a molecule specifically binding to CLDN18.2, i.e., a CLDN18.2 binding molecule that can specifically bind to extracellular domain 1 (ECD1) of human CLDN18.2 (e.g., a single domain antibody targeting CLDN18.2).

The present disclosure comprises at least the following embodiments, which are arranged sequentially and enumerated in the manner of "N" (wherein "N" represents a number), respectively. The following enumerative list is non-exhaustive, and those skilled in the art can combine different technical solutions.

1. A CLDN18.2 binding molecule, comprising at least one immunoglobulin single variable domain, wherein the immunoglobulin single variable domain comprises CDR1, CDR2 and CDR3, and wherein

(1) an amino acid sequence of CDR1 differs from a sequence as shown in SEQ ID NO: 1, 4 or 7 by amino acid addition, deletion or substitution of no more than 2 amino acids; an amino acid sequence of CDR2 differs from a sequence as shown in SEQ ID NO: 2, 5 or 8 by amino acid addition, deletion or substitution of no more than 2 amino acids; and/or an amino acid sequence of CDR3 differs from a sequence as shown in SEQ ID NO: 3, 6 or 9 by amino acid addition, deletion or substitution of no more than 2 amino acids; or

(2) an amino acid sequence of CDR1 differs from a sequence as shown in SEQ ID NO: 30, 37 or 45 by amino acid addition, deletion or substitution of no more than 2 amino acids; an amino acid sequence of CDR2 differs from a sequence as shown in SEQ ID NO: 31, 38 or 46 by amino acid addition, deletion or substitution of no more than 2 amino acids; and/or an amino acid sequence of CDR3 differs from a sequence as shown in SEQ ID NO: 32, 39 or 47 by amino acid addition, deletion or substitution of no more than 2 amino acids.

2. The CLDN18.2 binding molecule of embodiment 1, wherein the CLDN18.2 binding molecule is an antibody or an antigen-binding fragment thereof against CLDN18.2.

3. The CLDN18.2 binding molecule of embodiment 1 or 2, wherein the immunoglobulin single variable domain is VHH, such as VHH from camelids (e.g., an alpaca).

4. The CLDN18.2 binding molecule of any one of embodiments 1-3, wherein the immunoglobulin single variable domain comprises:

i) CDR1 having an amino acid sequence as shown in SEQ ID NO: 1, 4 or 7;
ii) CDR2 having an amino acid sequence as shown in SEQ ID NO: 2, 5 or 8; and
iii) CDR3 having an amino acid sequence as shown in SEQ ID NO: 3, 6 or 9.

4a. The CLDN18.2 binding molecule of any one of embodiments 1-3, wherein the immunoglobulin single variable domain comprises:

i) CDR1 having an amino acid sequence as shown in SEQ ID NO: 1, 4, 7, 30, 34, 37, 40, 41, 45 or 48;
ii) CDR2 having an amino acid sequence as shown in SEQ ID NO: 2, 5, 8, 31, 35, 38, 43 or 46; and
iii) CDR3 having an amino acid sequence as shown in SEQ ID NO: 3, 6, 9, 32, 33, 36, 39, 42, 44 or 47.

5. The CLDN18.2 binding molecule of any one of embodiments 1-4, wherein the immunoglobulin single variable domain comprises:

(a) CDR1 having an amino acid sequence as shown in SEQ ID NO: 1, CDR2 having an amino acid sequence as shown in SEQ ID NO: 2 and CDR3 having an amino acid sequence as shown in SEQ ID NO: 3;
(b) CDR1 having an amino acid sequence as shown in SEQ ID NO: 4, CDR2 having an amino acid sequence as shown in SEQ ID NO: 5 and CDR3 having an amino acid sequence as shown in SEQ ID NO: 6; or
(c) CDR1 having an amino acid sequence as shown in SEQ ID NO: 7, CDR2 having an amino acid sequence as shown in SEQ ID NO: 8 and CDR3 having an amino acid sequence as shown in SEQ ID NO: 9.

5a. The CLDN18.2 binding molecule of any one of embodiments 1-4, wherein the immunoglobulin single variable domain comprises:

(d) CDR1 having an amino acid sequence as shown in SEQ ID NO: 30, CDR2 having an amino acid sequence as shown in SEQ ID NO: 31 and CDR3 having an amino acid sequence as shown in SEQ ID NO: 32;
(e) CDR1 having an amino acid sequence as shown in SEQ ID NO: 30, CDR2 having an amino acid sequence as shown in SEQ ID NO: 31 and CDR3 having an amino acid sequence as shown in SEQ ID NO: 33;

(f) CDR1 having an amino acid sequence as shown in SEQ ID NO: 34, CDR2 having an amino acid sequence as shown in SEQ ID NO: 35 and CDR3 having an amino acid sequence as shown in SEQ ID NO: 36;

(g) CDR1 having an amino acid sequence as shown in SEQ ID NO: 37, CDR2 having an amino acid sequence as shown in SEQ ID NO: 38 and CDR3 having an amino acid sequence as shown in SEQ ID NO: 39;

(h) CDR1 having an amino acid sequence as shown in SEQ ID NO: 40, CDR2 having an amino acid sequence as shown in SEQ ID NO: 38 and CDR3 having an amino acid sequence as shown in SEQ ID NO: 39;

(i) CDR1 having an amino acid sequence as shown in SEQ ID NO: 41, CDR2 having an amino acid sequence as shown in SEQ ID NO: 38 and CDR3 having an amino acid sequence as shown in SEQ ID NO: 39;

(j) CDR1 having an amino acid sequence as shown in SEQ ID NO: 37, CDR2 having an amino acid sequence as shown in SEQ ID NO: 38 and CDR3 having an amino acid sequence as shown in SEQ ID NO: 42;

(k) CDR1 having an amino acid sequence as shown in SEQ ID NO: 37, CDR2 having an amino acid sequence as shown in SEQ ID NO: 43 and CDR3 having an amino acid sequence as shown in SEQ ID NO: 44;

(l) CDR1 having an amino acid sequence as shown in SEQ ID NO: 45, CDR2 having an amino acid sequence as shown in SEQ ID NO: 46 and CDR3 having an amino acid sequence as shown in SEQ ID NO: 47;

(m) CDR1 having an amino acid sequence as shown in SEQ ID NO: 48, CDR2 having an amino acid sequence as shown in SEQ ID NO: 46 and CDR3 having an amino acid sequence as shown in SEQ ID NO: 47.

6. The CLDN18.2 binding molecule of any one of embodiments 1-5, wherein the immunoglobulin single variable domain comprises or consists of the following sequences:

(A) an amino acid sequence as shown in SEQ ID NO: 10, 11, 12, 13, 14 or 15;
(B) an amino acid sequence at least 80%, 85%, 90%, 95%, 98% or 99% identical to the amino acid sequence as shown in SEQ ID NO: 10, 11, 12, 13, 14 or 15; or
(C) an amino acid sequence having amino acid addition, deletion and/or substitution of one or more (e.g., 1, 2, 3, 4, 5, 6, 7, 8, 9 or 10) amino acids in comparison with the amino acid sequence as shown in SEQ ID NO: 10, 11, 12, 13, 14 or 15, wherein preferably, the addition, deletion and/or substitution do/does not occur in a CDR region.

6a. The CLDN18.2 binding molecule of any one of embodiments 1-5, wherein the immunoglobulin single variable domain comprises or consists of the following sequences:

(A) an amino acid sequence as shown in SEQ ID NO: 10, 11, 12, 13, 14, 15, 23, 24, 25, 26, 27, 28 or 29;
(B) an amino acid sequence that is at least 80%, 85%, 90%, 95%, 98% or 99% identical to the amino acid sequence as shown in SEQ ID NO: 10, 11, 12, 13, 14, 15, 23, 24, 25, 26, 27, 28 or 29; or
(C) an amino acid sequence having amino acid addition, deletion and/or substitution of one or more (e.g., 1, 2, 3, 4, 5, 6, 7, 8, 9 or 10) amino acids in comparison with the amino acid sequence as shown in SEQ ID NO: 10, 11, 12, 13, 14, 15, 23, 24, 25, 26, 27, 28 or 29, wherein preferably, the addition, deletion and/or substitution do/does not occur in a CDR region.

7. The CLDN18.2 binding molecule of any one of embodiments 1-6, wherein the CLDN18.2 binding molecule is a single domain antibody, such as a heavy chain single domain antibody; a chimeric antibody; or a humanized antibody.

8. The CLDN18.2 binding molecule of any one of embodiments 1-7, wherein the immunoglobulin single variable domain is fused to an additional molecule, and the additional molecule is, for example, an Fc domain of an immunoglobulin (e.g., IgG) or a fluorescent protein.

9. The CLDN18.2 binding molecule of embodiment 8, wherein the CLDN18.2 binding molecule is a chimeric antibody comprising VHH from camelids and an Fc domain of human IgG (e.g., human IgG1 or IgG4).

10. The CLDN18.2 binding molecule of embodiment 9, wherein the CLDN18.2 binding molecule is a chimeric antibody comprising a VHH from alpaca and an Fc domain of human IgG1.

11. The CLDN18.2 binding molecule of any one of embodiments 1-10, wherein the CLDN18.2 binding molecule binds to extracellular domain 1 (ECD1) of human CLDN18.2.

12. A CLDN18.2 binding molecule, competing with the CLDN18.2 binding molecule of any one of embodiments 1-11 for the same epitope.

13. The CLDN18.2 binding molecule of any one of embodiments 1-12, specifically binding to CLDN18.2, but not binding to CLDN18.1.

14. An isolated nucleic acid molecule, encoding the CLDN18.2 binding molecule of any one of embodiments 1-13.

15. An expression vector, comprising the isolated nucleic acid molecule of embodiment 14.

16. A host cell, comprising the expression vector of embodiment 15.

17. The host cell of embodiment 16, wherein the host cell is a bacterial cell (e.g., *E. coli*), a fungal cell (e.g., a yeast), or a mammalian cell.

18. A pharmaceutical composition, comprising at least one CLDN18.2 binding molecule of any one of embodiments 1-13 and a pharmaceutically acceptable carrier.

19. A method for preparing the CLDN18.2 binding molecule of any one of embodiments 1-13, comprising the following steps:

- expressing the CLDN18.2 binding molecule of any one of embodiments 1-13 in the host cell of embodiment 16 or 17; and
- isolating the CLDN18.2 binding molecule from the host cell.

20. A method for treating a condition associated with CLDN18.2 in a subject, comprising: administering a therapeutically effective amount of the CLDN18.2 binding molecule of any one of embodiments 1-13 to the subject.

21. The method of embodiment 20, wherein the condition associated with CLDN18.2 includes a disease related to or associated with cells expressing CLDN18.2.

22. The method of embodiment 20 or 21, wherein the condition associated with CLDN18.2 includes cancer.

23. The method of embodiment 22, wherein the cancer includes bone cancer, blood cancer, lung cancer, liver cancer, pancreatic cancer, skin cancer, head and neck cancer, cutaneous or intraocular melanoma, uterine cancer, ovarian cancer, rectal cancer, cancer of the anal region, gastric cancer, colon cancer, breast cancer, prostate cancer, uterine cancer, cancer of sexual organs and reproductive organs, Hodgkin's disease, oesophageal cancer, small intestine cancer, cancer of endocrine system, thyroid cancer, parathyroid cancer, adrenal cancer, soft tissue sarcomas, bladder cancer, renal cancer, renal cell carcinoma, renal pelvis cancer, central nervous system (CNS) tumour, neuroectodermal cancer, spinal axis tumour, glioma, meningioma and pituitary adenoma.

24. The method of embodiment 23, wherein the cancer is gastric cancer.

25. Use of the CLDN18.2 binding molecule of any one of embodiments 1-13 in the preparation of a medicament for treating or preventing a condition associated with CLDN18.2.

26. A kit for treating or diagnosing a condition associated with CLDN18.2, comprising a container, wherein the container comprises the CLDN18.2 binding molecule of any one of embodiments 1-13.

The content stated above is a general description, and may include simplification, summary and omission of details if necessary. Therefore, those skilled in the art will recognize that the general description is only for illustrative purposes and not intended to be limiting in any way. Other aspects, features and advantages of the method, composition and/or device and/or other subjects described herein will become apparent from the teaching set forth herein. General descriptions are provided to briefly introduce some selected concepts, and these general descriptions will be further described in the following detailed description. The general descriptions above are not intended to determine the key features or basic features of the claimed subject matter, nor are they intended to be used as an auxiliary means to determine the scope of the claimed subject matter. In addition, the content of all references, patents, and published patent applications cited throughout the present application is incorporated herein by reference in their entirety.

## Brief Description of the Drawings

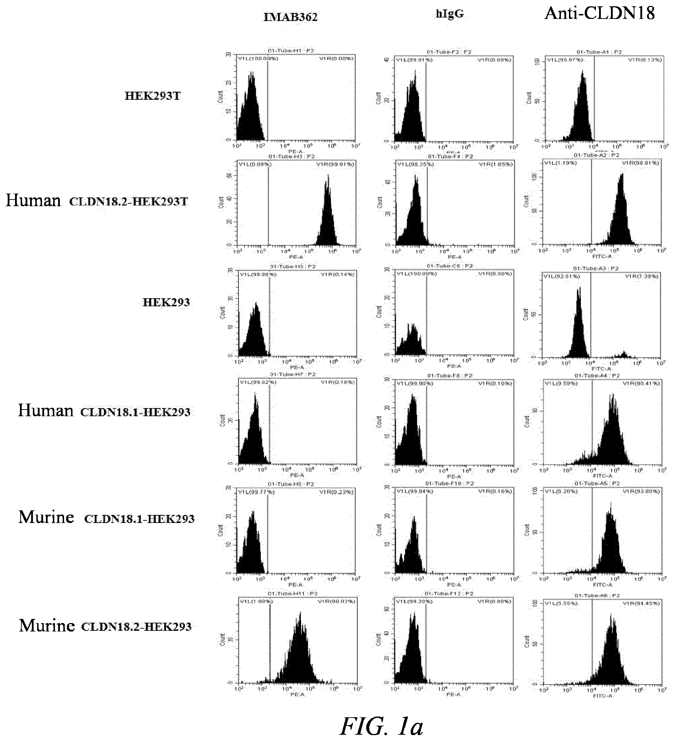

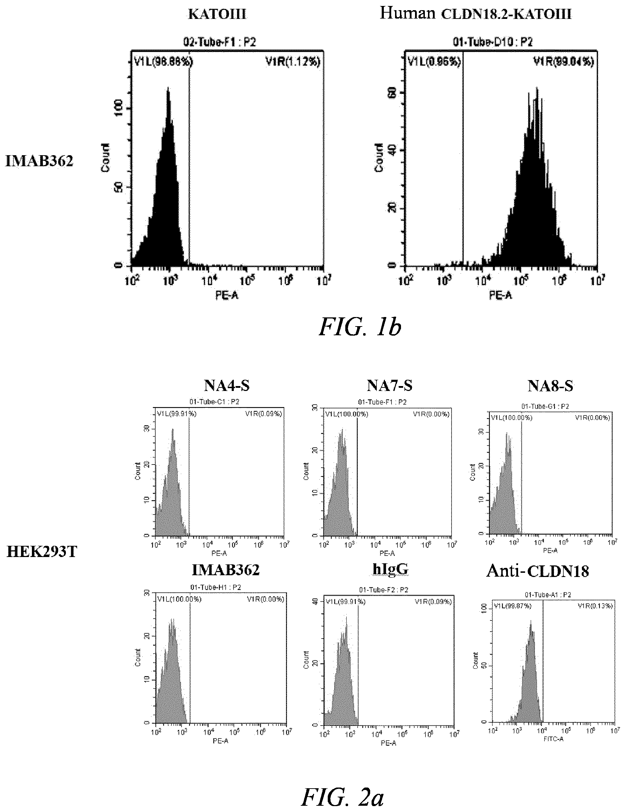

FIGs. 1a and 1b show flow cytometry identification results of overexpressing cell lines and tumour cell lines treated with IMAB362, hIgG and an anti-CLDN18 antibody in Example 1. FIG. 1a shows flow cytometry identification results of human CLDN18.2-HEK293T, human CLDN18.1-HEK293, murine CLDN18.2-HEK293 and murine CLDN18.1-HEK293 overexpressing cell lines. FIG. 1b shows flow cytometry identification results of human CLDN18.2-KATOIII tumour cell lines.

FIGs. 2a-2f show experimental results of the cross-binding of candidate antibodies NA4-S, NA7-S and NA8-S to human CLDN18.2-HEK293T, human CLDN18.1-HEK293, murine CLDN18.2-HEK293 and murine CLDN18.1-HEK293 overexpressing cell lines.

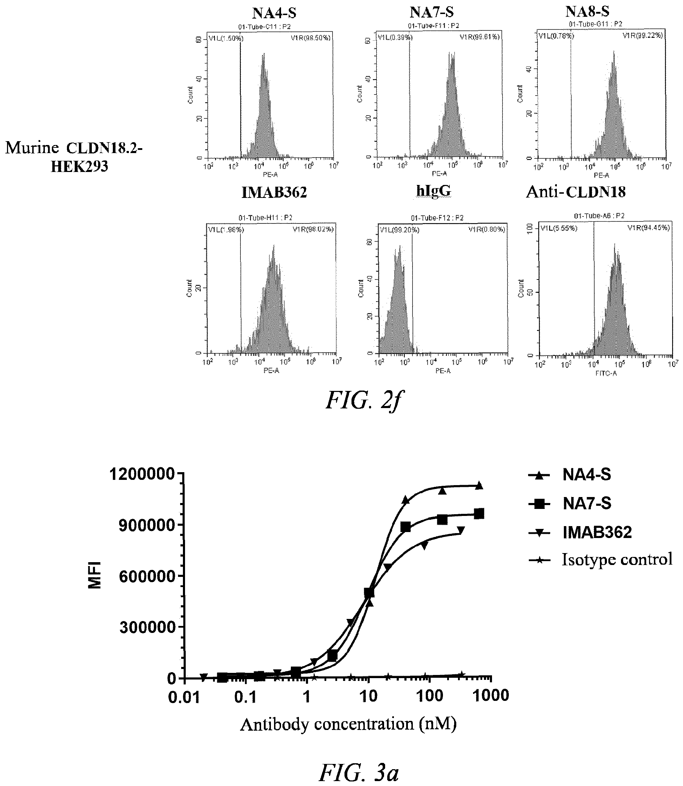

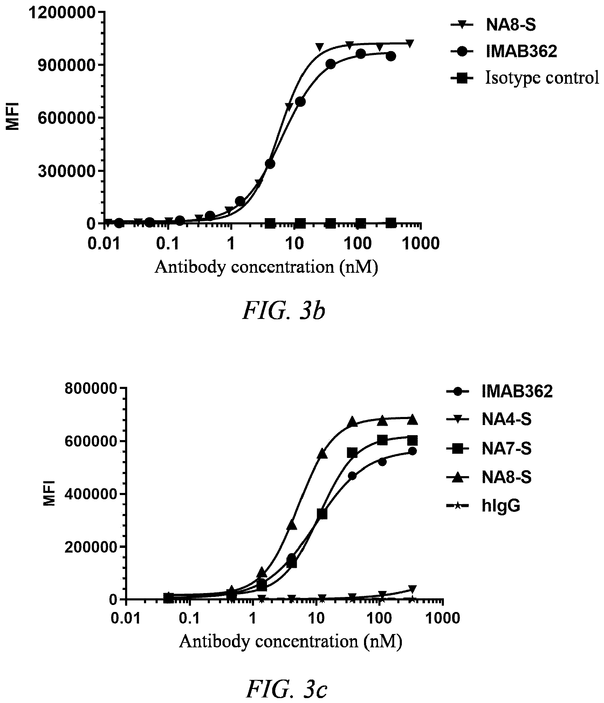

FIGs. 3a, 3b and 3c show comparative experimental results of the binding of candidate antibodies NA4-S, NA7-S and NA8-S and control antibodies at a cellular level. FIG. 3a shows comparative experimental results of the level detected by flow cytometry of antibodies NA4-S and NA7-S on human CLDN18.2-HEK293T overexpressing cells. FIG. 3b shows comparative experimental results of the level detected by flow cytometry of the antibody NA8-S on human CLDN18.2-HEK293T overexpressing cells. FIG. 3c shows comparative experimental results of the level detected by flow cytometry of antibodies NA4-S, NA7-S and NA8-S and control antibodies on human CLDN18.2-KATOIII tumour cell lines.

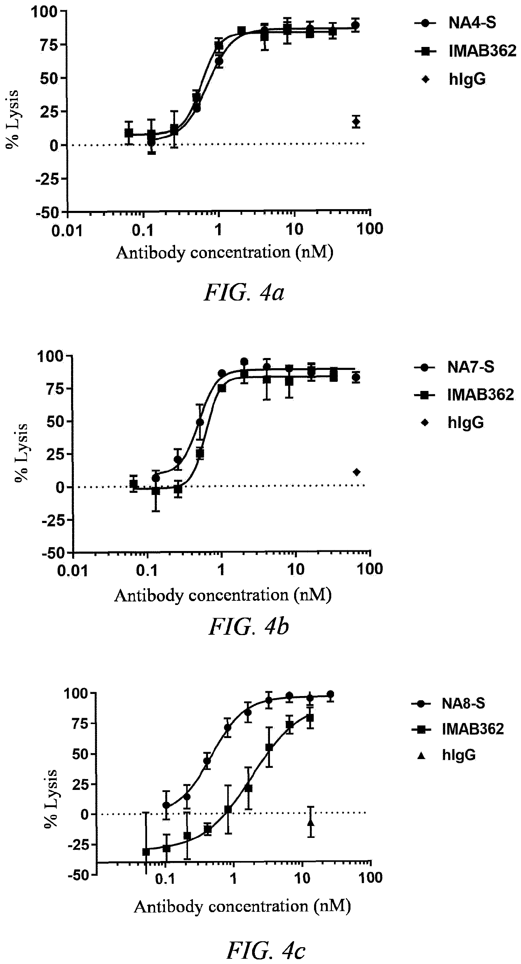

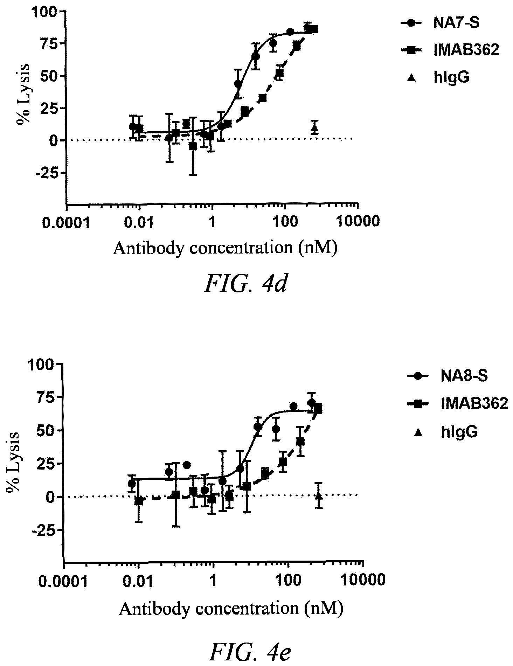

FIGs. 4a-4e show complement-dependent cytotoxicity (CDC) mediated by candidate antibodies NA4-S, NA7-S and NA8-S on human CLDN18.2-HEK293T overexpressing cell lines and human CLDN18.2-KATOIII tumour cell lines. FIGs. 4a, 4b and 4c show CDC mediated by antibodies NA4-S, NA7-S and NA8-S on human CLDN18.2-HEK293T, respectively. FIGs. 4d and 4e show CDC mediated by antibodies NA7-S and NA8-S on human CLDN18.2-KATOIII tumour cell lines, respectively.

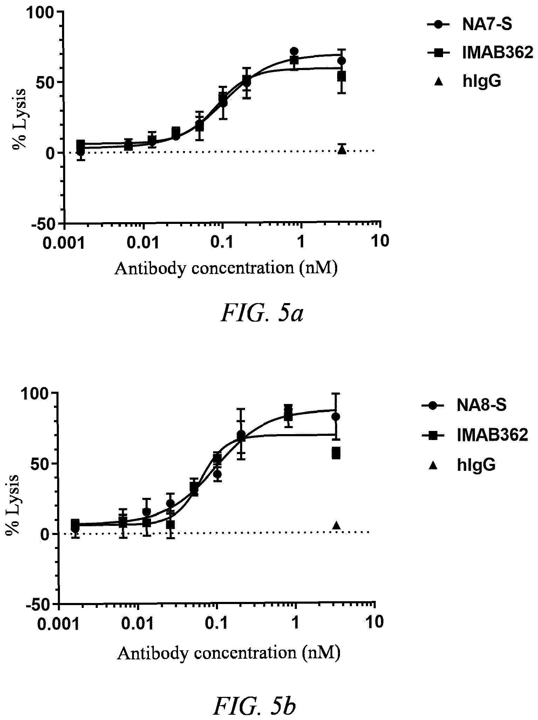

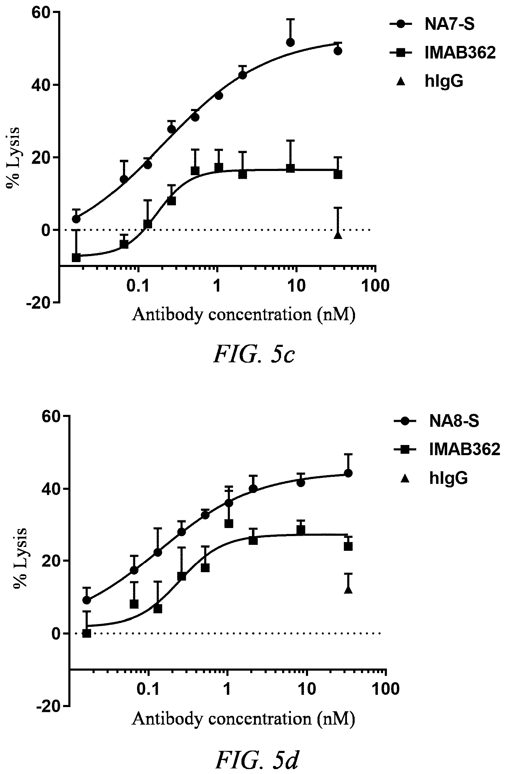

FIGs. 5a, 5b, 5c and 5d show antibody-dependent cell-mediated cytotoxicity (ADCC) mediated by candidate antibodies NA7-S and NA8-S on human CLDN18.2-HEK293T overexpressing cell lines and human CLDN18.2-KATOIII tumour cell lines. FIGs. 5a and 5b show ADCC mediated by antibodies NA7-S and NA8-S on human CLDN18.2-HEK293T overexpressing cell lines. FIGs. 5c and 5d show ADCC mediated by antibodies NA7-S and NA8-S on human CLDN18.2-KATOIII tumour cell lines.

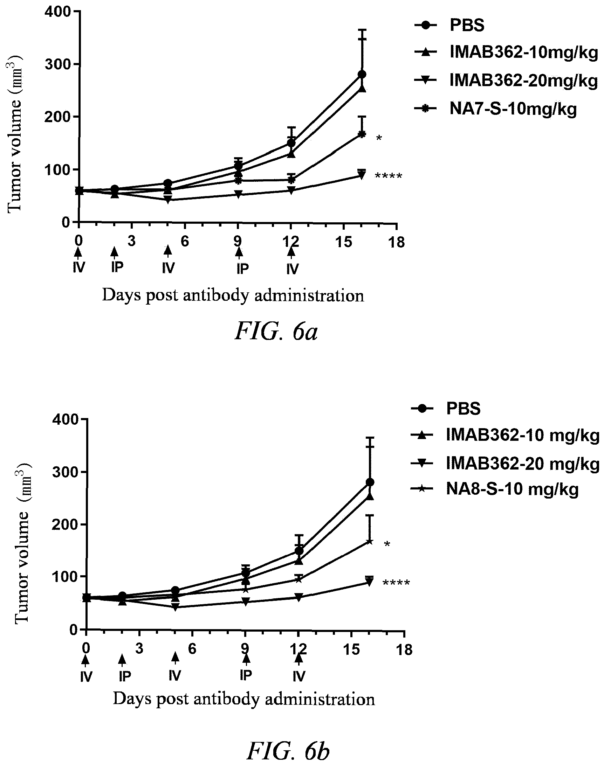

FIGs. 6a and 6b show tumour inhibition effects of candidate antibodies NA7-S and NA8-S in a human CLDN18.2-HEK293T xenograft model. FIG. 6a shows tumour inhibition effects of the candidate antibody NA7-S in a human CLDN18.2-HEK293T xenograft model. FIG. 6b shows tumour inhibition effects of the candidate antibody NA8-S in a human CLDN18.2-HEK293T xenograft model, wherein mice with immunodeficiency (SCID) are used; the administration dosages of NA7-S and NA8-S and IMAB362 are calculated according to the mass concentration per kilogram of body weight; and the mode of administration is crossover administration at the tail vein and the abdominal cavity.

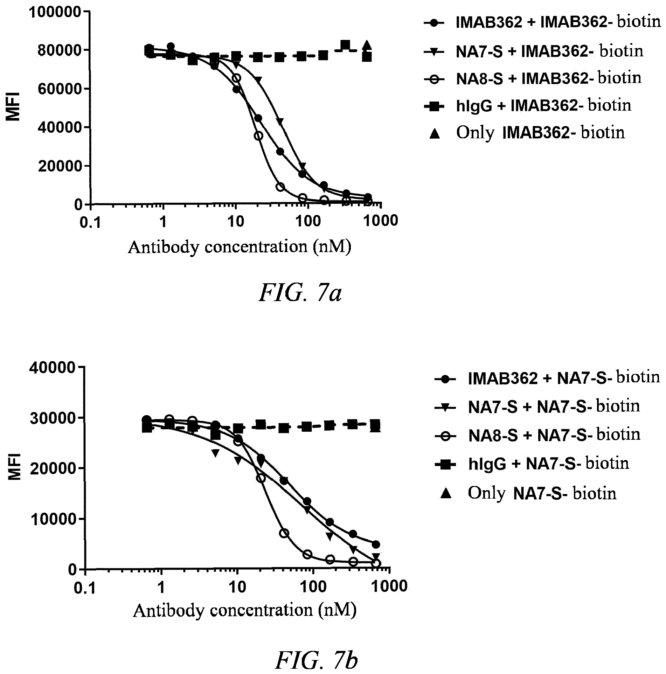

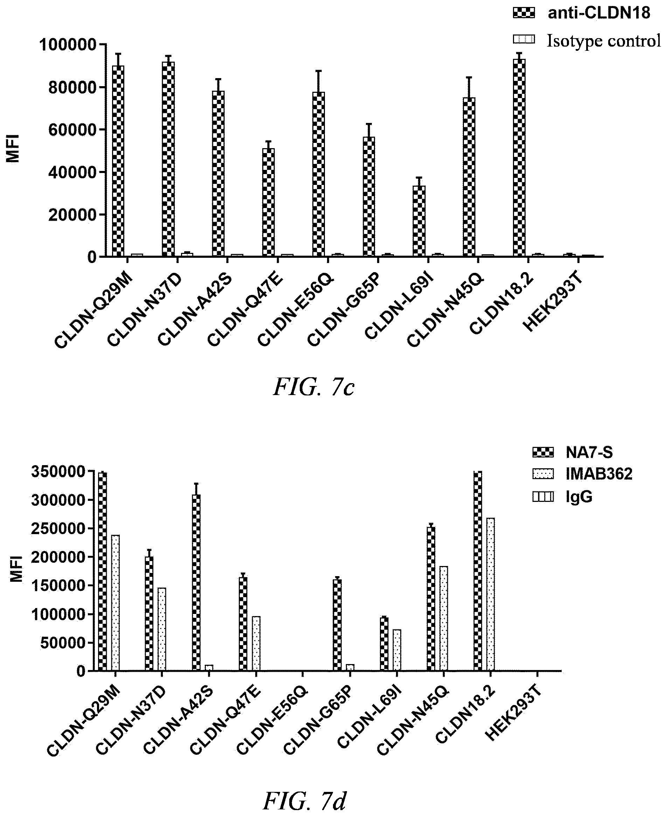

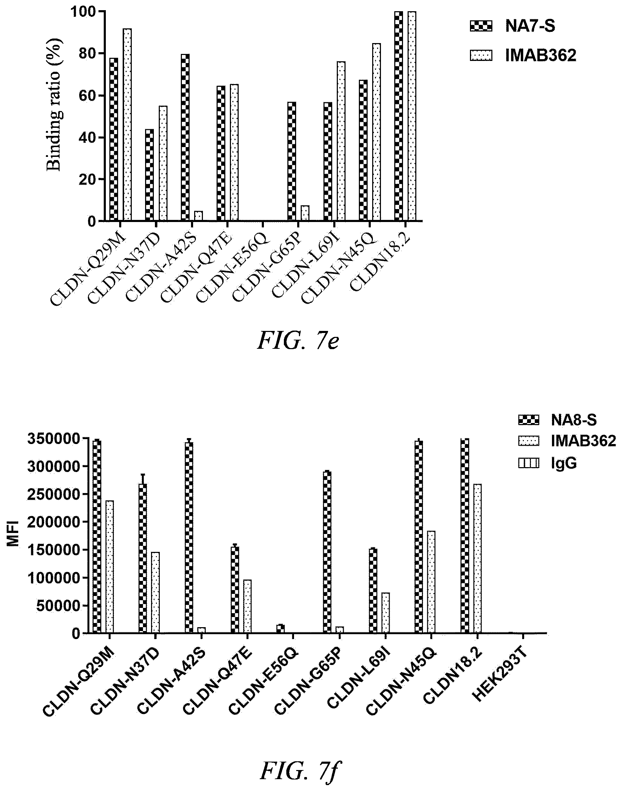

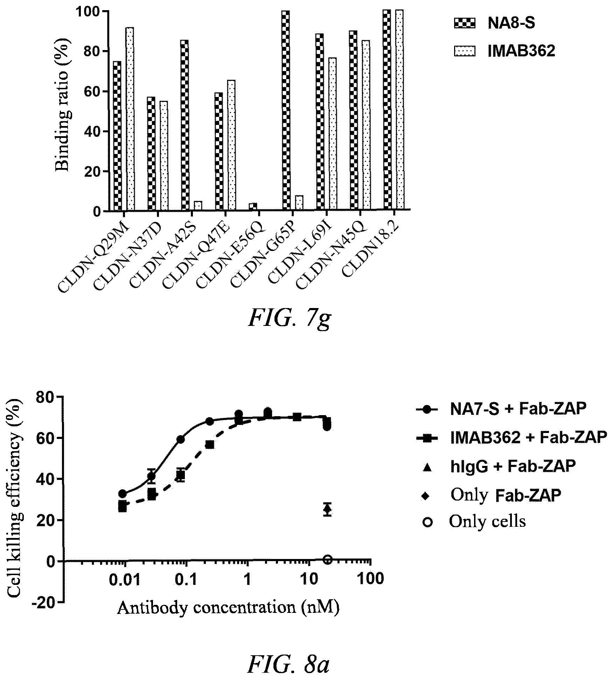

FIGs. 7a-7g show binding epitopes of candidate antibodies NA7-S, NA8-S and IMAB362 on a CLDN18.2 protein and key amino acid sites for binding. FIG. 7a shows competitive binding assays of candidate antibodies NA7-S and NA8-S with IMAB362 on human CLDN18.2-HEK293T overexpressing cell lines. FIG. 7b shows competitive binding assays of antibodies IMAB362 and NA8-S with NA7-S on human CLDN18.2-HEK293T overexpressing cell lines. FIG. 7c shows expression levels of mutant CLDN18.2 and wild-type CLDN18.2 on human CLDN18.2 wild-type and mutant cell lines determined with an anti-CLDN18 antibody. FIG. 7d shows binding levels of NA7-S and IMAB362 to a CLDN18.2 mutant on human CLDN18.2 mutant cell lines. FIG. 7e shows relative binding percentages of NA7-S and IMAB362 on human CLDN18.2 mutant cell lines relative to wild-type cell lines. FIG. 7f shows binding levels of NA8-S and IMAB362 to a CLDN18.2 mutant on human CLDN18.2 mutant cell lines. FIG. 7g shows relative binding percentages of NA8-S and IMAB362 on human CLDN18.2 mutant cell lines relative to wild-type cell lines.

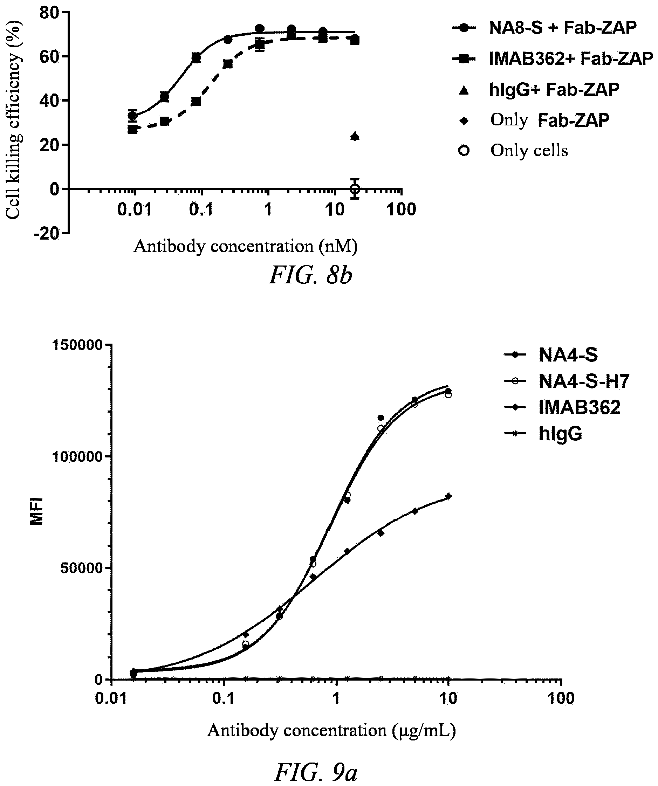

FIGs. 8a-8b show cell killing efficiencies of NA7-S, NA8-S and IMAB362 against human CLDN18.2-HEK293T in the presence of Fab-ZAP. FIG. 8a shows cell killing efficiencies of NA7-S and IMAB362 against human CLDN18.2-HEK293T; and FIG. 8b shows cell killing efficiencies of NA8-S and IMAB362 against human CLDN18.2-HEK293T, wherein hIgG and Fab-ZAP are used as the control in the experiment, and incubation of Fab-ZAP with cells for a long time also causes certain cytotoxic effects on cells.

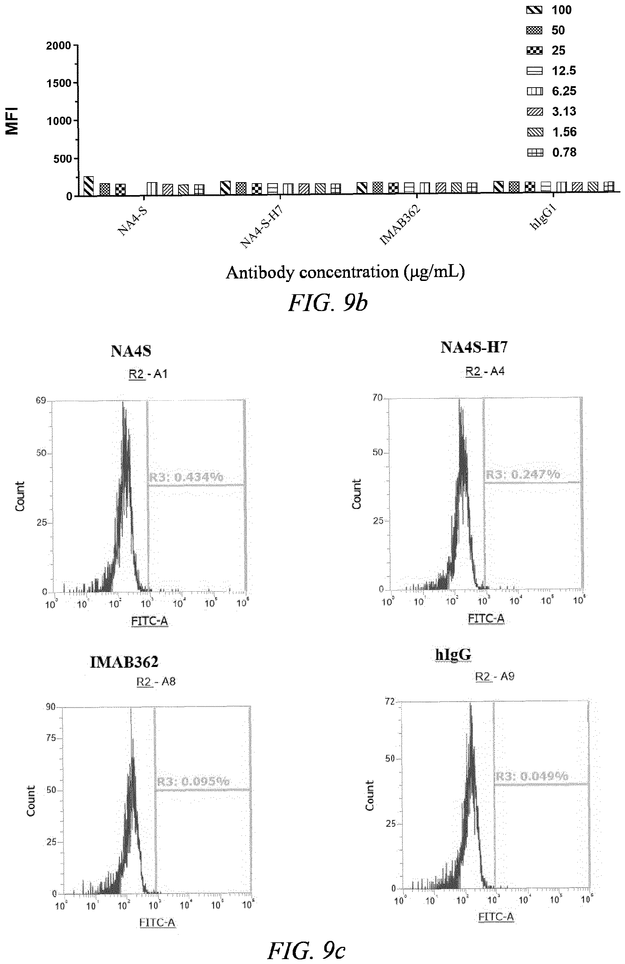

FIGs. 9a-9c show binding activities of NA4-S on human CLDN18.2-HEK293T and human CLDN18.1-HEK293 cells before and after humanized modification of the sequence, wherein NA4-S-H7 is a humanized molecule. FIG. 9a shows comparative binding activities of the NA4-S molecule on human CLDN18.2-HEK293T cells before and after humanization. FIG. 9b shows binding intensities of NA4-S, NA4-S-H7 and IMAB362 at different concentrations on human CLDN18.1-HEK293 cells. FIG. 9c shows binding positive rates of NA4-S, NA4-S-H7 and IMAB362 at a high concentration (100 μg/mL) on human CLDN18.1-HEK293 cells, wherein irrelevant antibodies are used as the control in the experiment.

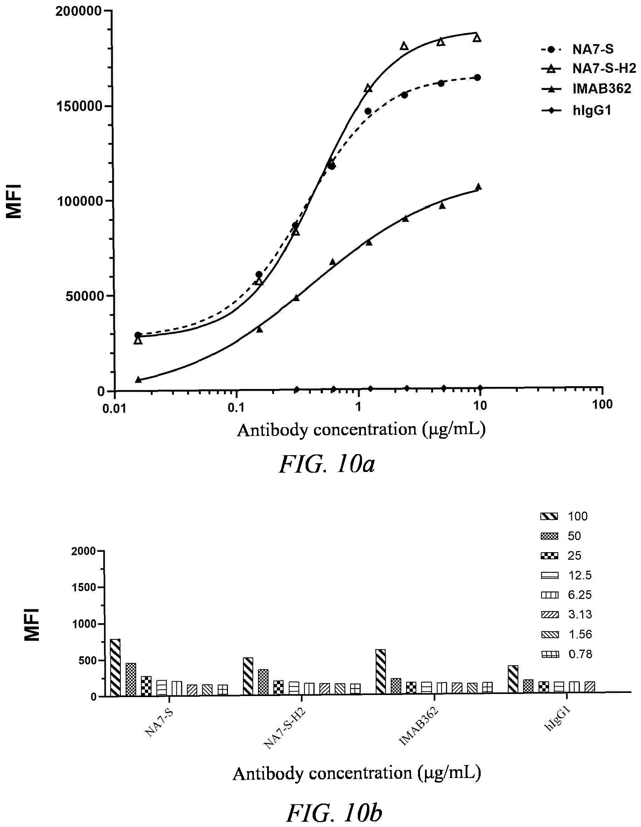

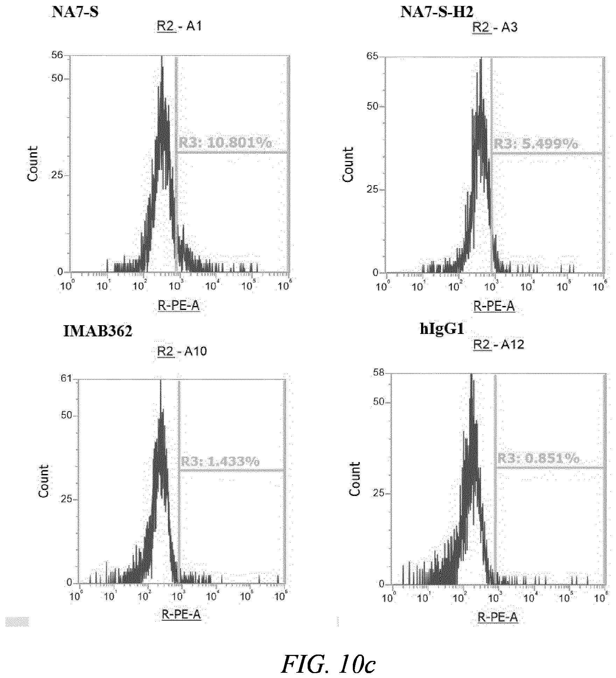

FIGs. 10a-10c show binding activities of NA7-S on human CLDN18.2-HEK293T and human CLDN18.1-HEK293 cells before and after humanized modification of the sequence, wherein NA7-S-H2 is a humanized molecule. FIG. 10a shows comparative binding activities of the NA7-S molecule on human CLDN18.2-HEK293T cells before and after humanization. FIG. 10b shows binding intensities of NA7-S, NA7-S-H2 and IMAB362 at different concentrations on human CLDN18.1-HEK293 cells. FIG. 10c shows binding positive rates of NA7-S, NA7-S-H2 and IMAB362 at a high concentration (100 μg/mL) on human CLDN18.1-HEK293 cells, wherein irrelevant antibodies are used as the control in the experiment.

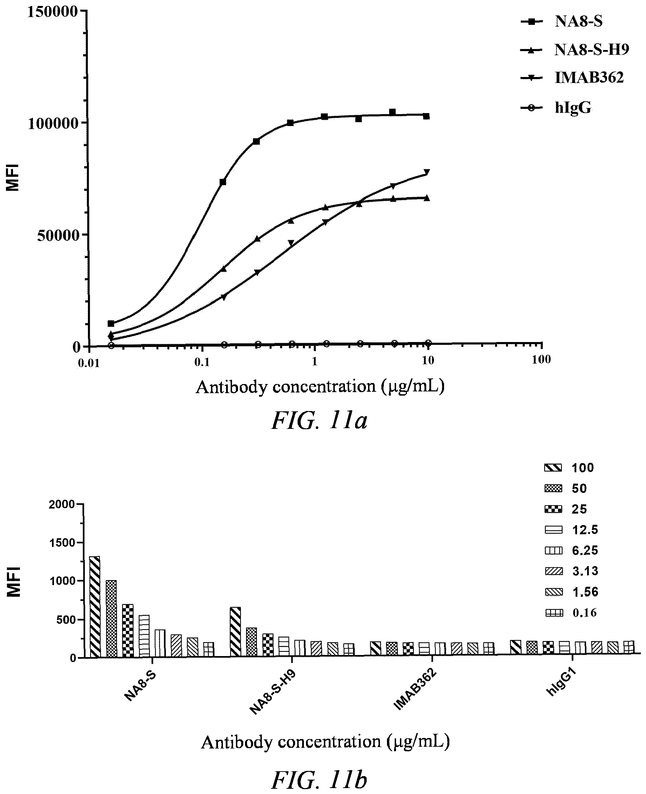

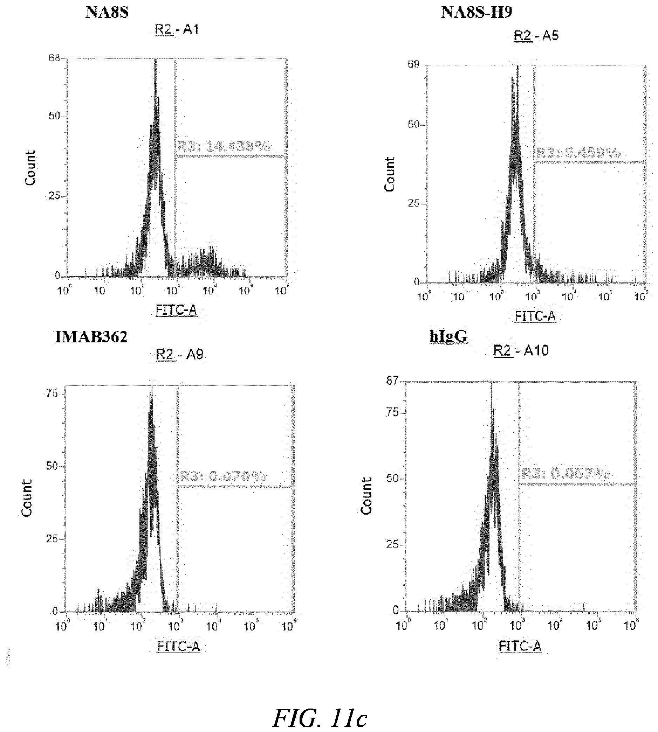

FIGs. 11a-11c show binding activities of NA8-S on human CLDN18.2-HEK293T and human CLDN18.1-HEK293 cells before and after humanized modification of the sequence, wherein NA8-S-H9 is a humanized molecule. FIG. 11a shows comparative binding activities of the NA8-S molecule on human CLDN18.2-HEK293T cells before and after humanization. FIG. 11b shows binding intensities of NA8-S, NA8-S-H9 and IMAB362 at different concentrations on human CLDN18.1-HEK293 cells. FIG. 11c shows binding positive rates of NA8-S, NA8-S-H9 and IMAB362 at a high concentration (100 μg/mL) on human CLDN18.1-HEK293 cells, wherein irrelevant antibodies are used as the control in the experiment.

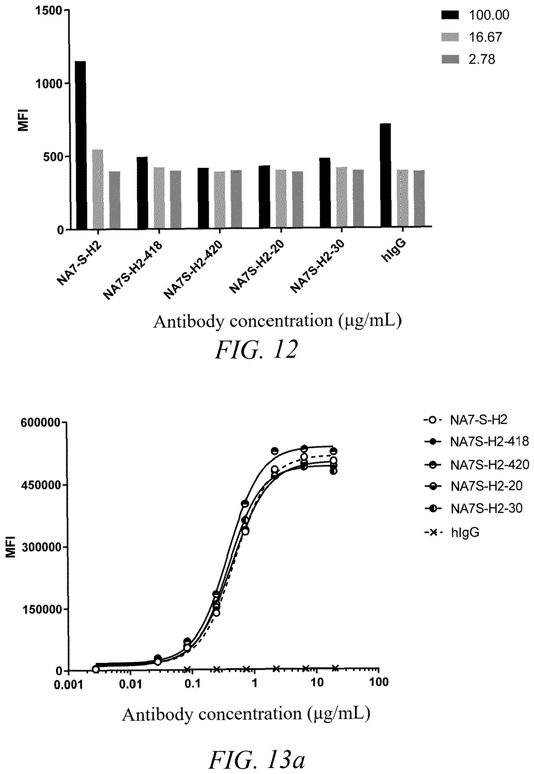

FIG. 12 shows binding activities of NA7-S-H2 on human CLDN18.1-HEK293 cells before and after affinity maturation of the sequence.

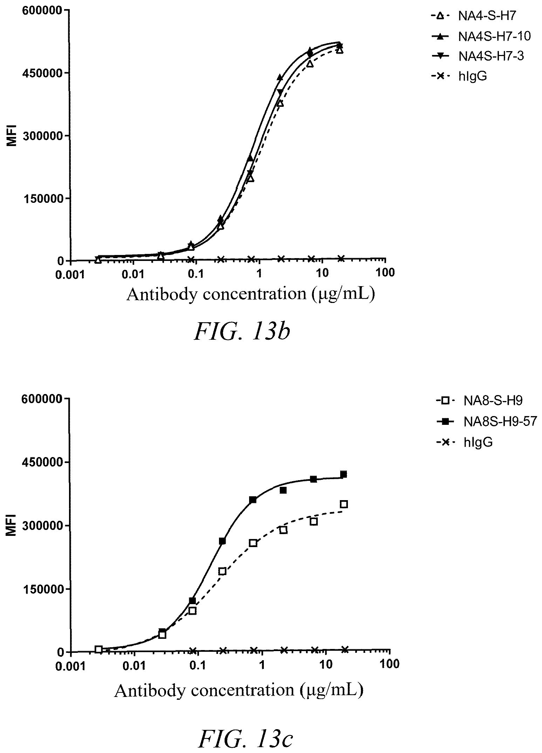

FIGs. 13a-13c show binding activities of affinity-matured molecules on human CLDN18.2-HEK293T cells. FIG. 13a shows binding activities of NA7-S-H2 and its affinity-matured molecules on human CLDN18.2-HEK293T cells. FIG. 13b shows binding activities of NA4-S-H7 and its affinity-matured molecules on human CLDN18.2-HEK293T cells. FIG. 13c shows binding activities of NA8-S-H9 and its affinity-matured molecule on human CLDN18.2-HEK293T cells.

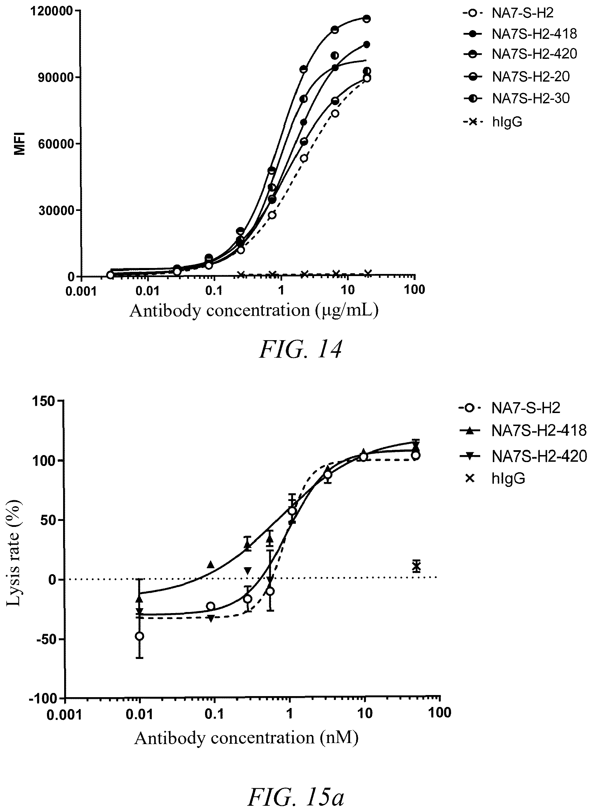

FIG. 14 shows binding activities of NA7-S-H2 and its affinity-matured molecules on human CLDN18.2-KATOIII tumour cell lines.

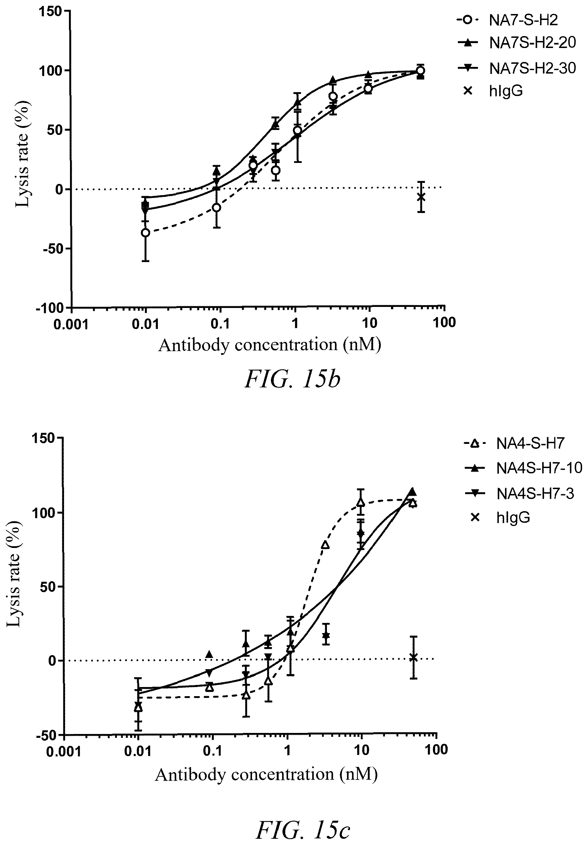

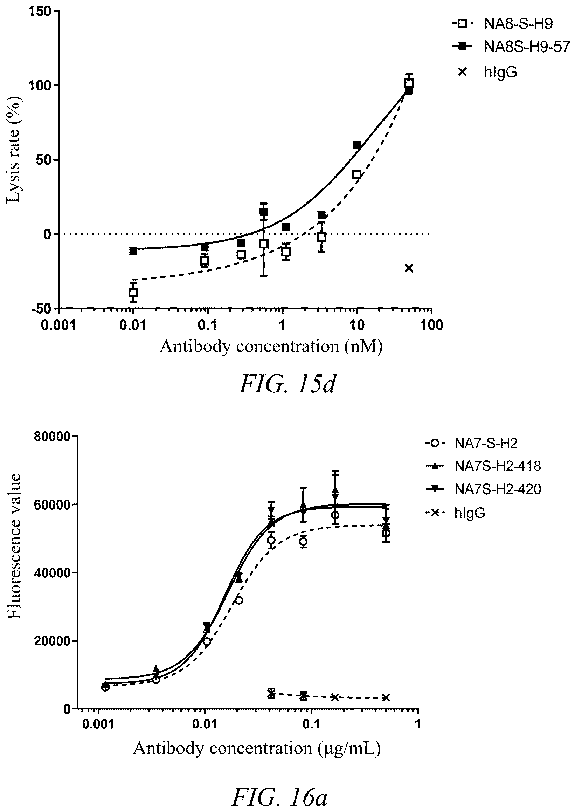

FIGs. 15a-15d show complement-dependent cytotoxicity (CDC) mediated by affinity-matured molecules on human CLDN18.2-HEK293T cells. FIG. 15a shows CDC mediated by affinity-matured molecules NA7S-H2-418 and NA7S-H2-420 on human CLDN18.2-HEK293T. FIG. 15b shows CDC mediated by affinity-matured molecules NA7S-H2-20 and NA7S-H2-30 on human CLDN18.2-HEK293T. FIG. 15c shows CDC mediated by affinity-matured molecules NA4S-H7-10 and NA4S-H7-3 on human CLDN18.2-HEK293T. FIG. 15d shows CDC mediated by the affinity-matured molecule NA8S-H9-57 on human CLDN18.2-HEK293T.

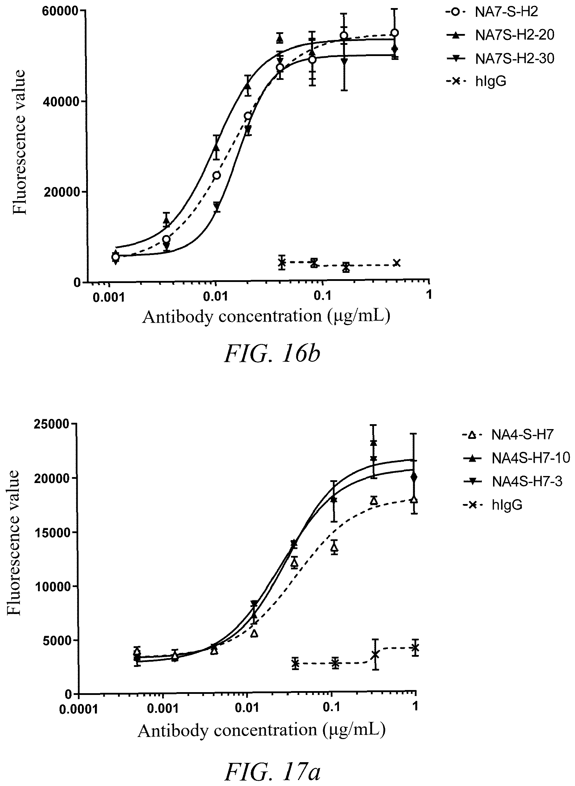

FIGs. 16a and 16b show antibody-dependent cell-mediated cytotoxicity (ADCC) mediated by NA7-S-H2 and its affinity-matured molecules on human CLDN18.2-HEK293T cells. FIG.16a shows ADCC mediated by affinity-matured molecules NA7S-H2-418 and NA7S-H2-420 on human CLDN18.2-HEK293T. FIG. 16b shows ADCC mediated by affinity-maturated molecules NA7S-H2-20 and NA7S-H2-30 on human CLDN18.2-HEK293T.

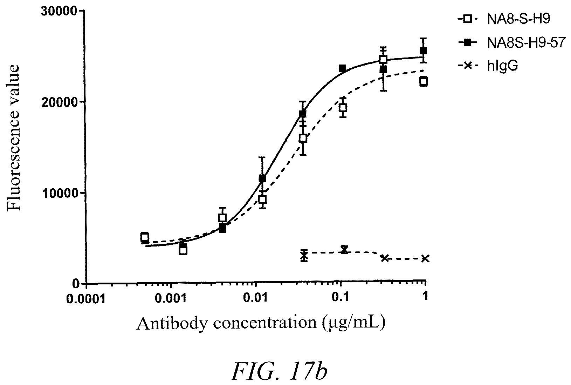

FIGs. 17a and 17b show antibody-dependent cell-mediated cytotoxicity (ADCC) mediated by affinity-matured molecules on human CLDN18.2-KATOIII tumour cell lines. FIG. 17a shows ADCC mediated by affinity-matured molecules NA4S-H7-10 and NA4S-H7-3 on human CLDN18.2-KATOIII. FIG. 17b shows ADCC mediated by the affinity-maturated molecule NA8S-H9-57 on human CLDN18.2-KATOIII.

## Detailed Description of Embodiments

Although the present invention can be practiced in many different forms, disclosed herein are specific illustrative embodiments that verify the principles of the present invention. It should be emphasized that the present invention is not limited to the specific embodiments illustrated. In addition, any section headings used herein is only for organizational purposes and is not construed as limiting the subject matter described.

Unless otherwise defined herein, the scientific and technical terms used in conjunction with the present invention have the meanings commonly understood by one of ordinary skill in the art. In addition, unless the context requires otherwise, terms in the singular form shall include the plural form, and terms in the plural form shall include the singular form. More specifically, as used in the present description and in the appended claims, the singular forms "a", "an" and "the" include plural referents unless the context clearly dictates otherwise. Therefore, for example, reference to "a protein" includes multiple proteins; reference to "a cell" includes a mixture of cells, etc. In the present application, unless otherwise stated, the term "or" used means "and/or". In addition, the term "comprising" and other forms (e.g., "including" and "containing") are not used for limitation. In addition, the range provided in the description and the appended claims includes both endpoints and all values between the endpoints.

Generally, the terms related to the cell and tissue culture, molecular biology, immunology, microbiology, genetics and protein, and nucleic acid chemistry and hybridization described herein and their techniques are well-known and commonly-used terms in the art. Unless otherwise stated, the methods and techniques of the present invention are generally carried out according to conventional methods well-known in the art, and are carried out as described in various general and more specific references cited and discussed throughout the present description. See, for example, Abbas et al., Cellular and Molecular Immunology, 6th ed., W.B. Saunders Company (2010); Sambrook J. & Russell D. Molecular Cloning: A Laboratory Manual, 3rd ed., Cold Spring Harbour Laboratory Press, Cold Spring Harbour, N.Y. (2000); Ausubel et al., Short Protocols in Molecular Biology: A Compendium of Methods from Current Protocols in Molecular Biology, Wiley, John & Sons, Inc. (2002); Harlow and Lane, Using Antibodies: A Laboratory Manual, Cold Spring Harbour Laboratory Press, Cold Spring Harbour, N.Y. (1998); and Coligan et al., Short Protocols in Protein Science, Wiley, John & Sons, Inc. (2003). The terms related to analytical chemistry, synthetic organic chemistry and medicinal chemistry described herein as well as laboratory procedures and techniques are well-known and commonly-used terms in the art. In addition, any section heading used herein is only for organizational purposes and is not construed as limiting the subject matter described.

## Definitions

To better understand the present invention, definitions and explanations of related terms are provided below.

As used herein, the term "antibody" or "Ab" generally refers to any form of an antibody that exhibits a desired biological or binding activity. The term includes, but is not limited to, a humanized antibody, a fully human antibody, a chimeric antibody and a single domain antibody. An antibody can comprise a heavy chain and a light chain. Heavy chains can be divided into $\mu$, $\delta$, $\gamma$, $\alpha$, and $\varepsilon$, which define the isotypes of an antibody as IgM, IgD, IgG, IgA, and IgE, respectively. Each heavy chain consists of a heavy chain variable region (VH) and a heavy chain constant region (CH). The heavy chain constant region generally consists of three domains (for $\gamma$, $\alpha$ and $\delta$: CH1, CH2 and CH3) or four domains (for $\mu$ and $\varepsilon$: CH1, CH2, CH3 and CH4). Each light chain consists of a light chain variable region (VL) and a light chain constant region (CL). The VH and VL regions can be further divided into relatively conserved regions (referred to as framework regions (FRs)) and hypervariable regions (referred to as complementarity determining regions (CDRs)) spaced apart by FRs. Each of the VH and the VL consists of 3 CDRs and 4 FRs in the following order: FR1, CDR1, FR2, CDR2, FR3, CDR3 and FR4 from N terminus to C terminus. The distribution of amino acids in various regions or domains follows the definitions in Kabat Sequences of Proteins of Immunological Interest (National Institutes of Health, Bethesda, Md. (1987 and 1991)) or Chothia & Lesk (1987) J. Mol. Biol. 196:901-917; Chothia et al. (1989), Nature 342:878-883. An antibody may have different antibody isotypes, such as IgG (including, e.g., IgG1, IgG2, IgG3 or IgG4 subtypes), IgA1, IgA2, IgD, IgE or IgM antibodies.

The term "humanized antibody" as used herein refers to an antibody in which a CDR sequence derived from the germline of another non-human mammalian species has been grafted onto a human framework sequence. Additional framework region modifications can be made within the human framework sequence.

The term "chimeric antibody" as used herein refers broadly to an engineered antibody that contains one or more regions from an antibody and one or more regions from one or more other antibodies. Specifically, the chimeric antibody comprises a variable region derived from a non-human animal antibody and a constant region of another antibody, for example, a camelid-derived variable region and a human-derived constant region. The chimeric antibody may also refer to a multispecific antibody that has specificity for at least two different antigens.

The term "CLDN18.2 binding molecule" as used herein means a molecule specifically binding to CLDN18.2.

The terms "CLDN18.2 antibody", "antibody against CLDN18.2", "antibody that specifically binds to CLDN18.2", "antibody that specifically targets CLDN18.2" and "antibody that specifically recognizes CLDN18.2" as used herein can be used interchangeably, and mean antibodies that can specifically bind to the Claudin protein CLDN18.2. Particularly, in a specific embodiment, the terms mean antibodies specifically bind to human CLDN18.2, particularly antibodies that specifically bind to human CLDN18.2 but do not specifically bind to human CLDN18.1. The amino acid sequences of human CLDN18.2 and human CLDN18.1 are as shown in SEQ ID NO: 16 and SEQ ID NO: 17, respectively; the amino acid sequences of murine CLDN18.2 and murine CLDN18.1 are as shown in SEQ ID NO: 18 and SEQ ID NO: 19, respectively.

The term "immunoglobulin single variable domain" or "ISV" as used herein is generally defined herein as such an amino acid sequence that comprises an immunoglobulin fold or can form an immunoglobulin fold (i.e., by means of folding) under suitable conditions (e.g., physiological conditions), i.e., thereby forming the immunoglobulin variable domain (e.g., VH, VL or VHH domain); and forms (or can form under suitable conditions) the immunoglobulin variable domain comprising functional antigen-binding sites (in the sense that no interaction (e.g., VH-VL interaction) with another immunoglobulin variable domain is required to form the functional antigen-binding sites).

The binding molecules, antibodies or antibody numbers (e.g., NA4-S, NA7-S, NA8-S, NA4-S-H7, NA7-S-H2, NA8-S-H9, NA7S-H2-418, NA8S-H9-57, NA7S-H2-420, NA7S-H2-20, NA7S-H2-30, NA4S-H7-10, NA4S-H7-3 and NA8S-H9-57) as used herein are only used to distinguish or identify molecules or products, and are not intended to indicate that such identifiers are features of the molecules or products of the present invention. Those skilled in the art shall understand that, for example, for the purpose of distinguishing or identification, other molecules, antibodies or products may also use such identifiers, but do not refer to the same or equivalent molecules, antibodies or products. Similarly, similar numbers or identifiers used in the Examples are only for convenience of illustration, and the binding molecules or products of the present invention are defined by the features described in the appended claims.

The term "specifically bind(s)" or "specifically bind(s) to" as used herein refers to the non-random binding reaction between two molecules such as between an antibody and an antigen.

As used herein, the ability to "inhibit the binding", "block the binding" or "compete for the same epitope" refers to the ability of an antibody to inhibit the binding of two molecules to any detectable extent. In some embodiments, an antibody that blocks the binding of two molecules inhibits the binding interaction between two molecules by at least 50%. In some embodiments, the inhibition can be greater than 60%, greater than 70%, greater than 80% or greater than 90%.

As used herein, the term "$EC_{50}$", also referred to as "half maximal effective concentration", refers to the concentration of a medicament, antibody or toxic agent which induces a response at 50% between the baseline and the maximum after a specific exposure time. In the context of the present application, the unit of $EC_{50}$ is "nM" or "$\mu$g/mL".

As used herein, the term "epitope" refers to the portion of an antigen to which an immunoglobulin or antibody specifically binds. The "epitope" is also referred to as an "antigenic determinant". The epitope or antigenic determinant generally consists of chemically active surface groups of molecules such as amino acids, carbohydrates or sugar side chains, and generally has specific three-dimensional structural characteristics and specific charge characteristics. For example, the epitope generally comprises at least 3, 4, 5, 6, 7, 8, 9, 10, 11, 12, 13, 14 or 15 continuous or discontinuous amino acids in the unique three-dimensional configuration and can be a "linear epitope" or "conformational epitope". See, for example, Epitope Mapping Protocols in Methods in Molecular Biology, Vol. 66, G. E. Morris, Ed. (1996). In the linear epitope, all interaction sites between a protein and an interaction molecule (e.g., an antibody) are present linearly along the primary amino acid sequence of the protein. In the conformational epitope, the interaction sites span over amino acid residues that are spaced apart from each other in a protein. Antibodies can be screened depending on the competition for binding to the same epitope detected by conventional techniques known to those skilled in the art. For example, competition or cross-competition studies can be carried out to obtain antibodies that compete with each other or cross-compete for binding to an antigen (e.g., CLDN18.2). The international patent application WO 03/048731 describes a high-throughput method for obtaining antibodies that bind to the same epitope, which is based on their cross-competition.

As used herein, the term "isolated" refers to a state of a substance or component obtained from the native state by artificial means. If an "isolated" substance or component occurs naturally, it may be that its natural environment has changed, or the substance or component has been isolated from the natural environment, or both. For example, an unisolated polynucleotide or polypeptide naturally occurs in a living animal, and identical high-purity polynucleotides or polypeptides isolated from the native state are referred to as isolated polynucleotides or polypeptides. The term "isolated" neither excludes mixed artificial or synthetic substances, nor does it exclude other impure substances that do not affect the activity of the substance isolated.

As used herein, the term "isolated antibody" refers to an antibody that is substantially free of other antibodies having different antigen specificities. In addition, the isolated antibody may be substantially free of other cellular materials and/or chemicals.

As used herein, the term "vector" refers to a nucleic acid vehicle into which a polynucleotide can be inserted. When the vector allows the expression of a protein encoded by the polynucleotide inserted therein, the vector is referred to as an expression vector. The vector can be introduced into a host cell by means of transformation, transduction or transfection so that the genetic material elements carried are expressed in the host cell. The vector is well known to those skilled in the art, and includes, but is not limited to, plasmids, phages, cosmids, artificial chromosomes such as yeast artificial chromosomes (YAC), bacterial artificial chromosomes (BAC) or P1-derived artificial chromosomes (PAC), phages such as λ phages or M13 phages and animal viruses. The animal viruses that can be used as vectors include, but are not limited to, retroviruses (including lentiviruses), adenoviruses, adeno-associated viruses, herpes viruses (e.g., herpes simplex viruses), poxviruses, baculoviruses, papilloma viruses and papovaviruses (e.g., SV40). The vector may comprise a plurality of elements for controlling expression, and includes, but is not limited to, promoter sequences, transcription initiation sequences, enhancer sequences, selection elements and reporter genes. Moreover, the vector may comprise an origin of replication.

As used herein, the term "host cell" refers to any kind of cell system into which a vector can be introduced, including but not limited to prokaryotic cells such as *E. coli* or *Bacillus subtilis,* fungal cells such as yeast cells or *Aspergillus,* insect cells such as Drosophila S2 cells or Sf9, and animal cells such as fibroblasts, CHO cells, COS cells, NSO cells, HeLa cells, BHK cells, HEK293 cells or human cells.

The method for producing the antibody of the present invention by using the host cell is conventional in the art, and comprises expressing the antibody in prokaryotic or eukaryotic cells, then isolating the antibody, and generally purifying the antibody to a pharmaceutically acceptable purity. For example, in some embodiments, a nucleic acid encoding the antibody is inserted into an expression vector by means of standard techniques known in the art, and the expression vector is introduced into suitable prokaryotic or eukaryotic host cells; the host cells such as CHO cells, NSO cells, SP2/0 cells, HEK293 cells, COS cells, PER.C6 (R) cells, yeast or *E. coli* cells are cultured under conditions and for a period of time sufficient for the production of the antibody of the present invention or the functional fragment thereof, and the antibody is recovered from the cells (the supernatant or cells obtained after lysis) and is purified. Conventional methods for producing antibodies are known in the art, and are described, for example, in review articles of Makrides, S.C., Protein Expr. Purif.17 (1999) 183-202; Geisse, S. et al., Protein Expr. Purif. 8 (1996) 271-282; Kaufman, R.J., Mol. Biotechnol. 16 (2000) 151-160; fferner, R. G., Drug Res. 48 (1998) 870-880.

As used herein, the term "identity" refers to a relationship between the sequences of two or more polypeptide molecules, or two or more nucleic acid molecules determined by sequence alignment and comparison. "Percent identity" refers to the percentage of identical residues between amino acids or nucleotides in the molecules being compared, and is calculated based on the size of the smallest molecule being compared. For these calculations, gaps (if any) in alignments are preferably addressed by a specific mathematical model or computer program (i.e., an "algorithm"). Methods that can be used to calculate the identity of aligned nucleic acids or polypeptides include those described in Computational Molecular Biology, (Lesk, A. M., ed.), 1988, New York: Oxford University Press; Biocomputing Informatics and Genome Projects, (Smith, D. W., ed.), 1993, New York: Academic Press; Computer Analysis of Sequence Data, Part I, (Griffin, A. M., and Griffin, H. G., eds.), 1994, New Jersey: Humana Press; von Heinje G., 1987, Sequence Analysis in Molecular Biology, New York: Academic Press; Sequence Analysis Primer, (Gribskov M. and Devereux J., eds.), 1991, New York: M. Stockton Press; and Carillo et al., 1988, SIAMJ. Applied Math. 48:1073.

As used herein, the term "immunogenicity" refers to the ability to stimulate the formation of specific antibodies or sensitized lymphocytes in an organism. It not only refers to the property of an antigen stimulating specific immune cells to activate, proliferate and differentiate so as to ultimately produce immune effectors such as antibodies and sensitized lymphocytes, but also refers to that a specific immune response of the antibodies or sensitized T lymphocytes can develop in the immune system of an organism after the organism is stimulated with the antigen. Immunogenicity is the most important property of an antigen. Successful induction of immune response generation in a host by an antigen depends on three factors: the property of the antigen, the reactivity of the host and the immunization means.

As used herein, the term "transfection" refers to the process of introducing a nucleic acid into a eukaryotic cell, particularly a mammalian cell. Solutions and techniques for transfection include, but are not limited to, lipofection, transfection using chemical and physical methods such as electroporation. Many transfection techniques are well-known in the art. See e.g., Graham et al., 1973, Virology 52:456; Sambrook et al., 2001, Molecular Cloning: A Laboratory Manual; Davis et al., 1986, Basic Methods in Molecular Biology, Elsevier; Chu et al, 1981, Gene 13:197.

As used herein, the term "fluorescence-activated cell sorting" or "FACS" refers to a specialized type of flow cytometry. It provides a method for sorting a heterogeneous mixture of biological cells into two or more containers, one cell at a time, based upon the specific light scattering and fluorescent characteristics of each cell (FlowMetric. "Sorting Out Fluorescence Activated Cell Sorting". 2017-11-09). Instruments used to carry out FACS are known to those skilled in the art and are commercially available to the public. Examples of such instruments include FACS Star Plus, FACScan, and FACSort instruments from Becton Dickinson (Foster City, CA), Epics C from Coulter Epics Division (Hialeah, FL) and MoFlo from Cytomation (Colorado Springs, Colorado).

The term "subject" includes any human or non-human animal, preferably human.

As used herein, the term "condition associated with CLDN18.2" refers to any condition induced by or exacerbated by or otherwise associated with increased or reduced expression or activity of CLDN18.2 (e.g., human CLDN18.2).

As used herein, the term "cancer" refers to solid tumours or nonsolid tumours (e.g., leukaemia) mediated by any tumour or malignant cell growth, proliferation or metastasis that induces medical conditions.

The term "treatment" used herein in the context of treating a pathogenetic condition generally refers to the treatment and therapy of humans or animals, wherein some desired treatment effects are achieved, for example, the inhibition in the progression of the pathogenetic condition, including the decline in the rate of progression, the arrest in the rate of progression, the regression of the pathogenetic condition, the improvement of the pathogenetic condition and the elimination of the pathogenetic condition. The term also includes the treatment as preventative measures (i.e., prevention). With regard to cancer, the "treatment" may refer to inhibiting or slowing down the growth, proliferation or metastasis of tumours or malignant cells or a combination thereof. With regard to a tumour, the "treatment" includes removing all or a part of the tumour, inhibiting or slowing down the growth and metastasis of the tumour, preventing or delaying the development of the tumour, or some combination thereof.

As used herein, the term "therapeutically effective amount" relates to the amount of an active compound or a material, composition or dosage form comprising the active compound, which is effective for producing some desired treatment effects that are commensurate with a reasonable benefit/risk ratio, when administered according to a desired treatment regimen. Specifically, the "therapeutically effective amount" means the amount or concentration of an antibody or an antigen-binding portion thereof effective for treating a condition associated with CLDN18.2.

As used herein, the term "pharmaceutically acceptable" refers to that carriers, diluents, excipients and/or salts thereof are chemically and/or physically compatible with other ingredients in a formulation, and are physiologically compatible with a recipient.

As used herein, the term "pharmaceutically acceptable carrier and/or excipient" refers to a carrier and/or excipient which are/is pharmacologically and/or physiologically compatible with a subject and an active agent and are/is well-known in the art (see, e.g., Remington's Pharmaceutical Sciences. Edited by Gennaro AR, 19th ed. Pennsylvania: Mack Publishing Company, 1995), and includes, but is not limited to, a pH regulator, a surfactant, an adjuvant and an ion strength enhancer. For example, the pH regulator includes, but is not limited to, a phosphate buffer; the surfactant includes, but is not limited to, a cationic, anionic or nonionic surfactant, such as Tween-80; and the ion strength enhancer includes, but is not limited to, sodium chloride.

**CLDN18.2 binding molecule**

In some aspects, the present disclosure provides a CLDN18.2 binding molecule.

In general, the CLDN18.2 binding molecule can comprise any molecule that specifically binds to CLDN18.2. The CLDN18.2 binding molecule can be a polypeptide or protein, such as an antibody, more specifically an antibody that specifically binds to CLDN18.2 (e.g., human CLDN18.2).

The antibody includes, but is not limited to, a chimeric antibody, a humanized antibody or a single domain antibody, etc. In specific embodiments, the CLDN18.2 binding molecule is a single domain antibody, which generally refers to an antibody consisting of a single monomeric variable antibody domain. Like an intact antibody, the single domain antibody can selectively bind to a specific antigen.

More particularly, the CLDN18.2 binding molecule is a heavy chain single domain antibody, which can be interchangeably used with the term "VHH", "VHH antibody", "VHH domain", "VHH antibody fragment", "$V_{HH}$" or "nanobody". The VHH molecule derived from a camelid antibody is one of the smallest known intact antigen-binding domains (about 15 KDa, or 1/10 of conventional IgG), therefore, the VHH is very suitable for being delivered to dense tissues and entering limited spaces between macromolecules.

The single domain antibody disclosed herein can be prepared by those skilled in the art according to methods known in the art or any future method. For example, the VHH can be obtained by methods known in the art, such as by immunizing a camel and obtaining the VHH that binds to and neutralizes a target antigen from the camel, or by cloning the VHH library of the present invention by using molecular biology techniques known in the art and then making a selection by using phage display. In some embodiments, the single domain antibody of the present invention is naturally produced in a camelid animal, i.e., is produced by immunizing a camel with CLDN18.2 or a fragment thereof by using techniques described herein for other antibodies.

In some embodiments, the single domain antibody is obtained by immunizing a llama or an alpaca with a desired antigen and subsequently isolating the mRNA encoding a heavy chain antibody. A gene library containing millions of single domain antibodies clones is produced by reverse transcription-polymerase chain reaction. Screening techniques such as phage display and ribosome display facilitate the identification of clones that bind to antigens. Phage display refers to synthesizing an antibody library on phages, screening the library with an antigen of interest or an antibody binding portion thereof, and isolating the antigen-binding phages, from which immunoreactive fragments can be obtained. Methods for preparing and screening such library is well-known in the art, and kits for producing the phage display library are commercially available (e.g., Pharmacia recombinant phage antibody system, Cat. No. 27-9400-01; and Stratagene SurfZAP™ phage display kit, Cat. No. 240612). Other methods and reagents may also be used to produce and screen antibody display libraries (see, e.g., Barbas et al., Proc. Natl. Acad. Sci. USA 88:7978-7982 (1991)).

When the most effective clone is identified, its DNA sequence can be optimized by, for example, affinity maturation or humanization to prevent the immune response against the antibody in the human body. As used herein, one or more CDRs of an "affinity-matured" antibody comprise the addition, deletion and/or substitution of one or more amino acids in comparison with one or more CDRs of a parent antibody, so that the affinity of the affinity-matured antibody for the antigen is improved in comparison with the parent antibody. A method for performing affinity maturation on an antibody is well-known in the art. See, for example, Marks et al., Bio/Technology 10: 779-783 (1992); Barbas et al., Proc. Nat. Acad. Sci. USA 91: 3809 -3813 (1994); Scier et al., Gene 169: 147-155 (1995); and Hawkins et al., J. Mol. Biol. 226: 889-896 (1992).

Therefore, the single domain antibody of the present invention can be obtained by: (1) isolating a naturally occurring VHH domain of a heavy chain antibody; (2) expressing a nucleotide sequence that encodes a naturally occurring VHH domain; (3) "humanizing" a naturally occurring VHH domain or expressing a nucleic acid that encodes such a humanized VHH domain; (4) "camelising" a naturally occurring VH domain from any animal species, particularly a mammalian species, such as, from human, or expressing a nucleic acid that encodes such a camelised VH domain; (5) "camelising" a "domain antibody" or "dAb" (see, e.g., Ward et al., 1989, Nature 341: 544-546), or expressing a nucleic acid that encodes such a camelised VH domain; (6) preparing a protein, a polypeptide or other amino acid sequences by using synthetic or semi-synthetic techniques; (7) preparing a nucleic acid encoding VHH by using a technique for nucleic acid synthesis, and then expressing the nucleic acid obtained therefrom; and/or (8) a combination of any of the foregoing. Based on the disclosure herein, suitable methods and techniques for performing the foregoing content would be clear to those skilled in the art, and include, for example, the methods and techniques described in more detail below.

Single domain antibodies are generally produced by PCR cloning a variable domain library from cDNA of blood, lymph nodes or splenic lymphocytes obtained from immunized animals into a phage display vector. Antigen-specific single domain antibodies are generally selected by panning the corresponding library on the immobilized antigen (e.g., an antigen coated on the plastic surface of a test tube, a biotinylated antigen immobilized on streptavidin beads or a membrane protein expressed on the cell surface). Affinity of sdAbs can be improved by simulating this strategy *in vitro,* for example, by site-directed mutagenesis in CDR regions and by further panning immobilized antigens under increased stringency conditions (higher temperature, high or low salt concentration, high or low pH and low antigen concentration) (Wesolowski et al., Single Domain Antibodies: Promising Experimental and Therapeutic Tools in Infection and Immunity. Med Microbiol Immunol (2009) 198: 157-174).

Methods for preparing VHH specifically binds to an antigen or epitope are described in references. See, for example, R. van der Linden et al., Journal of Immunological Methods, 240 (2000) 185-195; Li et al., J Biol Chem., 287(2012) 13713-13721; Deffar et al., African Journal of Biotechnology, Vol. 8(12), pp. 2645, 17 June, 2009 and WO 94/04678.

In some embodiments, the VHH in the CLDN18.2 binding molecule is fused to the Fc domain of an antibody (e.g., the Fc domain of IgG (e.g., IgG1 or IgG4)). Effector functions such as ADCC and CDC can be more effectively recruited by fusing VHH to the Fc domain. Moreover, the fusion of VHH and the Fc domain can help the CLDN18.2 binding molecule form a dimer, and can also help prolong the *in vivo* half-life of the CLDN18.2 binding molecule.

As used herein, the term "antibody-dependent cell-mediated cytotoxicity" or "ADCC" refers to a cytotoxic form through which secreted antibodies that bind to Fc receptors (FcRs) present on some cytotoxic effector cells (e.g., natural killer (NK) cells, neutrophils and macrophages) enable these cytotoxic effector cells to specifically bind to antigen-carrying target cells and subsequently kill the target cells with cytotoxins. "Arming" the cytotoxic cells with the antibodies is absolutely necessary for this killing. NK cells, as primary cells for mediating ADCC, express FcγRIII only, whereas

monocytes express FcγRI, FcγRII and FcγRIII. FcR expression on hematopoietic cells is summarized in Table 3 on page 464 of Ravetch and Kinet, Annu. Rev. Immunol 9:457-92 (1991). To evaluate the ADCC activity of a molecule of interest, an *in vitro* ADCC assay, such as an assay described in U.S. Patent US 5500362 or US 5821337, may be performed. Effector cells for such an assay include peripheral blood mononuclear cells (PBMCs) and natural killer (NK) cells. Alternatively or additionally, the ADCC activity of the molecule of interest may be evaluated *in vivo,* e.g., in an animal model as disclosed in Clynes et al., PNAS (USA) 95:652-656 (1998).

The term "complement-dependent cytotoxicity" or "CDC" refers to lysis of a target cell in the presence of a complement. Activation of the classical complement pathway is initiated by the binding of a first component (C1q) of the complement system to an antibody (an appropriate subclass) binding to its cognate antigen. To evaluate the complement activation, a CDC assay may be performed by using, for example, a method as described in Gazzano-Santoro et al., J. Immunol. Methods 202: 163 (1996).

For the convenience of description, the CLDN18.2 binding molecule is described as the CLDN18.2 antibody below.

*CLDN18.2 antibody capable of specifically binding to specific epitope of CLDN18.2*

In one aspect, the present disclosure relates to a single domain antibody that specifically binds to CLDN18.2, but does not or substantially does not bind to CLDN18.1.

The present inventors have discovered a CLDN18.2 binding molecule that can specifically bind to extracellular domain 1 (ECD1) of human CLDN18.2 (e.g., a single domain antibody targeting CLDN18.2).

As described hereinbefore, CLDN18.2 has two extracellular domains (ECDs), wherein the full-length sequence of human CLDN18.2 is as shown in SEQ ID NO: 16, and the sequence of ECD1 is as shown in SEQ ID NO: 20. Murine CLDN18.2 is as shown in SEQ ID NO: 18.

The sequences of human and murine CLDN18.1 are as shown in SEQ ID NO: 17 and SEQ ID NO: 19, respectively.

*CLDN18.2 antibody comprising CDRs having a certain sequence identity to a specific sequence*

In some embodiments, the CLDN18.2 antibody of the present disclosure comprises at least one immunoglobulin single variable domain (e.g., VHH), wherein the VHH comprises CDR1, CDR2 and CDR3, and wherein CDR1 comprises an amino acid sequence that is at least 80% identical to SEQ ID NO: 1, 4 or 7, CDR2 comprises an amino acid sequence that is at least 80% identical to SEQ ID NO: 2, 5 or 8 and CDR3 comprises an amino acid sequence that is at least 80% identical to SEQ ID NO: 3, 6 or 9.

In a given heavy chain variable region amino acid sequence, the precise amino acid sequence boundary of each CDR may be determined by using any one of many well-known antibody CDR assignment systems or a combination thereof, wherein the assignment systems include, for example, Chothia based on the three-dimensional structures of antibodies and the topology of CDR loops (Chothia et al., (1989) Nature 342: 877-883, Al-Lazikani et al., "Standard Conformations for the Canonical Structures of Immunoglobulins", Journal of Molecular Biology, 273, 927-948 (1997)), Kabat based on the antibody sequence variability (Kabat et al., Sequences of Proteins of Immunological Interest, version 4, U.S. Department of Health and Human Services, National Institutes of Health (1987)), AbM (University of Bath), Contact (University College London), the International ImMunoGeneTics database (IMGT) (World-Wide-Web imgt.cines.fr/), and the North CDR definition based on affinity propagation clustering using a large number of crystal structures. In the CLDN18.2 antibody of the present disclosure, the amino acid sequences of the CDR regions are defined by using an IMGT or AbM method, but the method for defining CDRs is not unique and restrictive, and it is not meant that the present disclosure only protects an antibody molecule represented by the CDR regions defined by the IMGT or AbM method. Based on the aforementioned various conventional antibody CDR assignment systems in the art, the CLDN18.2 antibody molecules of the present disclosure can also be characterized by using various other different CDR region definition methods. It shall be noted that the boundaries of CDRs of a variable region in the same antibody obtained based on different assignment systems may be different, that is, the CDR sequences of the variable region in the same antibody defined under different assignment systems may be different. The CDR sequences defined under these different assignment systems and the antibody molecules characterized thereby are also intended to be protected by the present disclosure.

Variable regions and CDRs in the antibody sequence may be identified according to general rules that have been developed in the art (as described above, e.g., the Kabat numbering system) or by aligning the sequence with a database of known variable regions. Methods for identifying these regions are described in Kontermann and Dubel, eds., Antibody Engineering, Springer, New York, NY, 2001 and Dinarello et al., Current Protocols in Immunology, John Wiley and Sons Inc., Hoboken, NJ, 2000. An exemplary database of the antibody sequence is described in and available at the website "Abysis": www.bioinf.org.uk/abs (maintained by A.C. Martin, Department of Biochemistry & Molecular Biology University College London, London, England) and the website VBASE2: www.vbase2.org, as described in Retter et al., Nucl. Acids Res., 33 (Database issue): D671-D674 (2005). Preferably, the sequence is analysed by using the Abysis database which integrates sequence data from Kabat, IMGT and Protein Data Bank (PDB) and structure data from PDB, see Protein Sequence and Structure Analysis of Antibody Variable Domains. in the book: Antibody Engineering Lab Manual wrote by Dr. Andrew C. R. Martin (Ed.: Duebel, S. and Kontermann, R., Springer-Verlag, Heidelberg, ISBN-13: 978-3540413547, which is also available at the website: bioinforg.uk/abs). The Abysis database website also includes general rules that have been developed to identify the CDRs that can be used in accordance with the teaching herein.

The percent identity between two amino acid sequences can be determined by using the algorithm of E. Meyers and W. Miller (Comput. Appl. Biosci., 4:11-17 (1988)) which has been incorporated into the ALIGN program (version 2.0), and a PAM120 weight residue table, a gap length penalty of 12 and a gap penalty of 4 are used. In addition, the percent identity between two amino acid sequences can be determined by using the Needleman-Wunsch algorithm (J. Mol. Biol. 48:444-453 (1970)) which has been incorporated into a GAP program in a GCG software package (available at http://www.gcg.com), and a Blossom 62 matrix or a PAM250 matrix, a gap weight of 16, 14, 12, 10, 8, 6, or 4 and a length weight of 1, 2, 3, 4, 5, or 6 are used.

Additionally or alternatively, the protein sequences of the present disclosure can further be used as a "query sequence" to perform a search directed at public databases to, for example, identify related sequences. Such search can be performed by using the XBLAST program (version 2.0) of Altschul, et al. (1990) J. Mol. Biol. 215:403-10. A BLAST protein search can be performed by using an XBLAST program, with the score = 50 and the word length = 3, to obtain an amino acid sequence homologous to the antibody molecule of the present disclosure. To obtain gapped alignments for comparison purposes, Gapped BLAST can be used, as described in Altschul et al., (1997) Nucleic Acids Res. 25(17):3389-3402. When BLAST and Gapped BLAST programs are used, the default parameters of the respective program (e.g., XBLAST and NBLAST) can be used. See www.ncbi.nlm.nih.gov.

In some other embodiments, the amino acid sequences of CDRs can be at least 85%, 86%, 87%, 88%, 89%, 90%, 91%, 92%, 93%, 94%, 95%, 96%, 97%, 98% or 99% identical to each sequence given above.

*CLDN18.2 antibody comprising CDRs having addition, deletion or substitution of amino acids*

In some embodiments, the CLDN18.2 antibody of the present disclosure comprises at least one immunoglobulin single variable domain (e.g., VHH), wherein the VHH comprises CDR1, CDR2 and CDR3. In some embodiments, an amino acid sequence of CDR1 differs from a sequence as shown in SEQ ID NO: 1, 4 or 7 by amino acid addition, deletion or substitution of no more than 2 (e.g., 0, 1 or 2) amino acids; an amino acid sequence of CDR2 differs from a sequence as shown in SEQ ID NO: 2, 5 or 8 by amino acid addition, deletion or substitution of no more than 2 (e.g., 0, 1 or 2) amino acids; and/or an amino acid sequence of CDR3 differs from a sequence as shown in SEQ ID NO: 3, 6 or 9 by amino acid addition, deletion or substitution of no more than 2 (e.g., 0, 1 or 2) amino acids. For example, CDR1, CDR2 and CDR3 differ from the amino acid sequences as shown in SEQ ID NO: 1, 4 or 7, SEQ ID NO: 2, 5 or 8 and SEQ ID NO: 3, 6 or 9 by amino acid addition, deletion or substitution of only one amino acid, respectively. The above-mentioned CDR coding system can use IMGT. In some other embodiments, an amino acid sequence of CDR1 differs from a sequence as shown in SEQ ID NO: 30, 37 or 45 by amino acid addition, deletion or substitution of no more than 2 (e.g., 0, 1 or 2) amino acids; an amino acid sequence of CDR2 differs from a sequence as shown in SEQ ID NO: 31, 38 or 46 by amino acid addition, deletion or substitution of no more than 2 (e.g., 0, 1 or 2) amino acids; and/or an amino acid sequence of CDR3 differs from a sequence as shown in SEQ ID NO: 32, 39 or 47 by amino acid addition, deletion or substitution of no more than 2 (e.g., 0, 1 or 2) amino acids. The above-mentioned CDR coding system can use AbM.

Preferably, the CDR of the isolated antibody or the antigen-binding portion thereof contains conservative substitution of no more than 2 amino acids or no more than 1 amino acid. As used herein, the term "conservative substitution" refers to amino acid substitution that does not negatively affect or change the basic property of the protein/polypeptide comprising amino acid sequences. For example, conservative substitution can be introduced by standard techniques (e.g., site-directed mutagenesis and PCR-mediated mutagenesis) known in the art. Conservative amino acid substitution includes substitution in which an amino acid residue is substituted with another amino acid residue having a similar side chain, for example, physically or functionally similar residues (e.g., having similar sizes, shapes, charges, chemical properties including the ability to form a covalent bond or a hydrogen bond, etc.) are substituted with corresponding amino acid residues. Families of amino acid residues having similar side chains have been defined in the art. These families include amino acids having basic side chains (e.g., lysine, arginine and histidine), amino acids having acidic side chains (e.g., aspartic acid and glutamic acid), amino acids having uncharged polar side chains (e.g., glycine, asparagine, glutamine, serine, threonine, tyrosine, cysteine and tryptophan), amino acids having non-polar side chains (e.g., alanine, valine, leucine, isoleucine, proline, phenylalanine and methionine), amino acids having β-branched side chains (e.g., threonine, valine and isoleucine) and amino acids having aromatic side chains (e.g., tyrosine, phenylalanine, tryptophan and histidine). Therefore, a corresponding amino acid residue is preferably substituted with another amino acid residue from the family having the same side chain. A method for identifying conservative substitution of amino acids is well-known in the art (see, e.g., Brummell et al., Biochem. 32: 1180-1187 (1993); Kobayashi et al., Protein Eng. 12(10): 879-884 (1999); and Burks et al., Proc. Natl. Acad. Sci. USA 94: 412-417 (1997), which are incorporated herein by reference).

In certain embodiments, the immunoglobulin single variable domain of the CLDN18.2 antibody comprises:

i) CDR1 having an amino acid sequence as shown in SEQ ID NO: 1, 4 or 7;
ii) CDR2 having an amino acid sequence as shown in SEQ ID NO: 2, 5 or 8; and
iii) CDR3 having an amino acid sequence as shown in SEQ ID NO: 3, 6 or 9.

In certain embodiments, the immunoglobulin single variable domain of the CLDN18.2 antibody comprises:

i) CDR1 having an amino acid sequence as shown in SEQ ID NO: 30, 37 or 45;
ii) CDR2 having an amino acid sequence as shown in SEQ ID NO: 31, 38 or 46; and
iii) CDR3 having an amino acid sequence as shown in SEQ ID NO: 32, 39 or 47.

In some other embodiments, the immunoglobulin single variable domain of the CLDN18.2 antibody comprises:

i) CDR1 having an amino acid sequence as shown in SEQ ID NO: 1, 4, 7, 30, 34, 37, 40, 41, 45 or 48;
ii) CDR2 having an amino acid sequence as shown in SEQ ID NO: 2, 5, 8, 31, 35, 38, 43 or 46; and
iii) CDR3 having an amino acid sequence as shown in SEQ ID NO: 3, 6, 9, 32, 33, 36, 39, 42, 44 or 47.

*CLDN18.2 antibody comprising CDRs*

In some embodiments, the CLDN18.2 antibody of the present disclosure comprises at least one immunoglobulin single variable domain (e.g., VHH), wherein the VHH comprises CDR1, CDR2 and CDR3, and wherein the CDR1, CDR2 and CDR3 are selected from:

(a) CDR1 having an amino acid sequence as shown in SEQ ID NO: 1, CDR2 having an amino acid sequence as shown in SEQ ID NO: 2 and CDR3 having an amino acid sequence as shown in SEQ ID NO: 3;
(b) CDR1 having an amino acid sequence as shown in SEQ ID NO: 4, CDR2 having an amino acid sequence as shown in SEQ ID NO: 5 and CDR3 having an amino acid sequence as shown in SEQ ID NO: 6;
(c) CDR1 having an amino acid sequence as shown in SEQ ID NO: 7, CDR2 having an amino acid sequence as shown in SEQ ID NO: 8 and CDR3 having an amino acid sequence as shown in SEQ ID NO: 9;
(d) CDR1 having an amino acid sequence as shown in SEQ ID NO: 30, CDR2 having an amino acid sequence as shown in SEQ ID NO: 31 and CDR3 having an amino acid sequence as shown in SEQ ID NO: 32;
(e) CDR1 having an amino acid sequence as shown in SEQ ID NO: 30, CDR2 having an amino acid sequence as shown in SEQ ID NO: 31 and CDR3 having an amino acid sequence as shown in SEQ ID NO: 33;
(f) CDR1 having an amino acid sequence as shown in SEQ ID NO: 34, CDR2 having an amino acid sequence as shown in SEQ ID NO: 35 and CDR3 having an amino acid sequence as shown in SEQ ID NO: 36;
(g) CDR1 having an amino acid sequence as shown in SEQ ID NO: 37, CDR2 having an amino acid sequence as shown in SEQ ID NO: 38 and CDR3 having an amino acid sequence as shown in SEQ ID NO: 39;
(h) CDR1 having an amino acid sequence as shown in SEQ ID NO: 40, CDR2 having an amino acid sequence as shown in SEQ ID NO: 38 and CDR3 having an amino acid sequence as shown in SEQ ID NO: 39;
(i) CDR1 having an amino acid sequence as shown in SEQ ID NO: 41, CDR2 having an amino acid sequence as shown in SEQ ID NO: 38 and CDR3 having an amino acid sequence as shown in SEQ ID NO: 39;
(j) CDR1 having an amino acid sequence as shown in SEQ ID NO: 37, CDR2 having an amino acid sequence as shown in SEQ ID NO: 38 and CDR3 having an amino acid sequence as shown in SEQ ID NO: 42;
(k) CDR1 having an amino acid sequence as shown in SEQ ID NO: 37, CDR2 having an amino acid sequence as shown in SEQ ID NO: 43 and CDR3 having an amino acid sequence as shown in SEQ ID NO: 44;
(l) CDR1 having an amino acid sequence as shown in SEQ ID NO: 45, CDR2 having an amino acid sequence as shown in SEQ ID NO: 46 and CDR3 having an amino acid sequence as shown in SEQ ID NO: 47;
(m) CDR1 having an amino acid sequence as shown in SEQ ID NO: 48, CDR2 having an amino acid sequence as shown in SEQ ID NO: 46 and CDR3 having an amino acid sequence as shown in SEQ ID NO: 47.

*CLDN18.2 antibody defined with VHH sequence*

In some embodiments, the CLDN18.2 antibody of the present disclosure comprises at least one (e.g., one) immunoglobulin single variable domain (e.g., VHH), wherein the VHH comprises:

(A) an amino acid sequence as shown in SEQ ID NO: 10, 11, 12, 13, 14 or 15;

(B) an amino acid sequence that is at least 80%, 85%, 90%, 95%, 98% or 99% identical to SEQ ID NO: 10, 11, 12, 13, 14 or 15; or

(C) an amino acid sequence having addition, deletion and/or substitution of one or more (e.g., 1, 2, 3, 4, 5, 6, 7, 8, 9 or 10) amino acids in comparison with the amino acid sequence as shown in SEQ ID NO: 10, 11, 12, 13, 14 or 15, wherein preferably, the addition, deletion and/or substitution do/does not occur in a CDR region.

In some other embodiments, the CLDN18.2 antibody of the present disclosure comprises at least one (e.g., one) immunoglobulin single variable domain (e.g., VHH), wherein the VHH comprises:

(A) an amino acid sequence as shown in SEQ ID NO: 10, 11, 12, 13, 14, 15, 23, 24, 25, 26, 27, 28 or 29;

(B) an amino acid sequence that is at least 80%, 85%, 90%, 95%, 98% or 99% identical to the amino acid sequence as shown in SEQ ID NO: 10, 11, 12, 13, 14, 15, 23, 24, 25, 26, 27, 28 or 29; or

(C) an amino acid sequence having addition, deletion and/or substitution of one or more (e.g., 1, 2, 3, 4, 5, 6, 7, 8, 9 or 10) amino acids in comparison with the amino acid sequence as shown in SEQ ID NO: 10, 11, 12, 13, 14, 15, 23, 24, 25, 26, 27, 28 or 29, wherein preferably, the addition, deletion and/or substitution do/does not occur in a CDR region.

In other embodiments, the amino acid sequence of the VHH may be at least 85%, 86%, 87%, 88%, 89%, 90%, 91%, 92%, 93%, 94%, 95%, 96%, 97%, 98% or 99% identical to each sequence mentioned above. As an illustrative example, the antibody may comprise VHH having at least 85%, 86%, 87%, 88%, 89%, 90%, 91%, 92%, 93%, 94%, 95%, 96%, 97%, 98% or 99% sequence identity to SEQ ID NO: 10, 11, 12, 13, 14, 15, 23, 24, 25, 26, 27, 28 or 29.

In some further embodiments, the CLDN18.2 antibody of the present disclosure may comprise conservative substitution or modification of amino acids in the heavy chain variable region. It is understood in the art that certain conservative sequence modifications that do not eliminate the antigen-binding properties may be made. See, for example, Brummell et al., (1993) Biochem 32:1180-8; de Wildt et al., (1997) Prot. Eng. 10:835-41; Komissarov et al., (1997) J. Biol. Chem. 272:26864-26870; Hall et al., (1992) J. Immunol. 149:1605-12; Kelley and O' Connell (1993) Biochem. 32:6862-35; Adib-Conquy et al., (1998) Int. Immunol. 10:341-6 and Beers et al., (2000) Clin. Can. Res. 6:2835-43.

**Nucleic acid molecule encoding antibody of the present disclosure and method for producing CLDN18.2 antibody**

In some aspects, the present disclosure relates to an isolated nucleic acid molecule comprising a nucleic acid sequence encoding the CLDN18.2 antibody of the present disclosure.

The nucleic acid of the present disclosure can be obtained by using standard molecular biological techniques. With regard to an antibody obtained from an immunoglobulin gene library (e.g., using the phage display technology), a nucleic acid encoding such an antibody can be recovered from the gene library.

The nucleic acid molecule encoding the CLDN18.2 antibody of the present disclosure can be inserted into a vector by using the recombinant techniques known in the art for further cloning (amplification of DNA) or expression. Many vectors are available. A vector or vector component generally includes, but is not limited to, one or more of the following: a signal sequence, an origin of replication, one or more marker genes, an enhancer element, a promoter (e.g., SV40, CMV and EF-1α) and a transcriptional termination sequence. A selective marker gene facilitates the selection of a host cell into which a vector is introduced (see, e.g., U.S. Pat. Nos. 4,399,216; 4,634,665 and 5,179,017). For example, the selective marker gene generally confers drug (e.g., G418, hygromycin, or methotrexate) resistance to the host cell into which the vector has been introduced. In one embodiment, the selective marker gene can include a dihydrofolate reductase (DHFR) gene (for dhfr-host cells having methotrexate selection/amplification) and a neo gene (for G418 selection). In another embodiment, the antibody can be produced by homologous recombination known in the art. DNA encoding monoclonal antibodies is readily isolated and sequenced by using a conventional method (e.g., by using an oligonucleotide probe that can specifically bind to a gene encoding an antibody heavy chain).

In some embodiments, the vector system includes a mammalian system, a bacterial system, a yeast system, etc., and includes a plasmid, for example, but not limited to, pALTER, pBAD, pcDNA, pCal, pL, pET, pGEMEX, pGEX, pCI, pCMV, pEGFP, pEGFT, pSV2, pFUSE, pVITRO, pVIVO, pMAL, pMONO, pSELECT, pUNO, pDUO, Psg5L, pBABE, pWPXL, pBI, p15TV-L, pPro18, pTD, pRS420, pLexA and pACT2.2, and other laboratory vectors and commercially available vectors. Suitable vectors can include plasmids or viral vectors (e.g., replication-defective retroviruses, adenoviruses and adeno-associated viruses). In one embodiment of the present disclosure, the vector may be pET, such as pETbac containing hexahistidine-tagged and c-Myc-tagged genes.

The vector comprising the nucleic acid sequence that encodes the CLDN18.2 antibody of the present disclosure can be introduced into a host cell for cloning or gene expression. Suitable host cells for cloning or expressing DNA in the vector herein are prokaryotes, yeasts or higher eukaryote cells. Suitable prokaryotes for this purpose include eubacteria, such as Gram-negative or Gram-positive organisms, for example, Enterobacteriaceae, such as *Escherichia,* e.g., *E. coli; Enterobacter; Erwinia; Klebsiella; Proteus; Salmonella* such as *Salmonella typhimurium; Serratia* such as *Serratia marcescans;* and *Shigella; Bacillus* such as *B. subtilis* and *B. licheniformis; Pseudomonas* such as *Pseudomonas aeruginosa;* and *Streptomyces.*

In addition to prokaryotes, eukaryotes such as filamentous fungi or yeasts are suitable host cells for expressing the CLDN18.2 antibody of the present disclosure. *Saccharomyces cerevisiae* or common baker's yeast, is the most commonly used among lower eukaryotic host microorganisms. However, many other genera, species and strains are generally available and can be used in the present disclosure, for example, *Schizosaccharomyces pombe; Kluyveromyces* hosts such as *K. lactis, K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906), *K. thermotolerans,* and *K. marxianus; Yarrowia* (EP 402,226); *Pichia pastoris* (EP 183,070); *Candida; Trichoderma reesia* (EP 244,234); *Neurospora crassa; Schwanniomyces* such as *Schwanniomyces occidentalis;* and filamentous fungi such as *Neurospora, Penicillium, Tolypocladium,* and *Aspergillus* hosts such as *A. nidulans* and *A. niger.*

Other suitable host cells for expressing the CLDN18.2 antibody of the present disclosure are derived from multicellular organisms. Examples of invertebrate cells include plant and insect cells. At present, a large number of baculovirus strains and variants and corresponding permissive insect host cells have been identified from the following hosts: *Spodoptera frugiperda* (caterpillar), *Aedes aegypti* (mosquito), *Aedes albopictus* (mosquito), *Drosophila melanogaster* (fruitfly), and *Bombyx mori.* Various virus strains for transfection are publicly available, such as the L-1 variant of *Autographa californica* NPV and the Bm-5 strain of *Bombyx mori* NPV, and according to the present disclosure, these viruses may be used in the transfection process through which the CLDN18.2 antibody is expressed in a suitable host cell, in particular for the transfection of *Spodoptera frugiperda* cells. Plant cell cultures of cotton, corn, potato, soybean, petunia, tomato, and tobacco can also be used as hosts.

Host cells are transformed with the vector described above for the production of the CLDN18.2 antibody of the present disclosure, and are cultured in conventional nutrient culture media modified as appropriate for inducing promoters, selecting transformants or amplifying genes encoding the desired sequences.

The host cells used for producing the CLDN18.2 antibody of the present disclosure may be cultured in various culture media. Commercially available culture media such as Ham's F10 (Sigma), Minimal Essential Medium (MEM) (Sigma), RPMI-1640 (Sigma) and Dulbecco's Modified Eagle's Medium (DMEM, Sigma) are suitable for culturing host cells. In addition, any culture medium described in Ham et al., Meth. Enz. 58: 44 (1979); Barnes et al., Anal. Biochem. 102: 255 (1980); U.S. Pat. No. 4,767,704, 4,657,866, 4,927,762, 4,560,655 or 5,122,469; WO 90/03430; WO 87/00195; or U.S. Pat. Re. 30,985 can be used as a culture medium for the host cells. If necessary, hormones and/or other growth factors (e.g., insulin, transferrin or epidermal growth factors), salts (e.g., sodium chloride, calcium, magnesium and phosphate), buffers (e.g., HEPES), nucleotides (e.g., adenosine and thymine), antibiotics (e.g., GENTAMYCIN drug), trace elements (defined as inorganic compounds generally present at final concentrations in a micromolar range) and glucose or an equivalent energy source can be added to any of these culture media. Any other necessary supplements may also be included at appropriate concentrations that would be known to those skilled in the art. Culture conditions, such as temperature and pH, are those preciously used with the host cells selected for expression and would be apparent to one of ordinary skill in the art.

When recombinant techniques are used, an antibody can be produced in the periplasmic space in a cell or directly secreted into a culture medium. If the antibody is produced in the cell, as a first step, particulates and debris (host cells or lysis fragments) are removed, for example, by means of centrifugation or ultrafiltration. A method for isolating an antibody secreted into the periplasmic space *of E. coli* is described in Carter et al., Bio/Technology 10:163-167 (1992). Briefly, cells are thawed in the presence of sodium acetate (pH 3.5), EDTA and phenylmethylsulphonyl fluoride (PMSF) within about 30 min. Cell debris can be removed by means of centrifugation. In the case that the antibody is secreted into the culture medium, the supernatant from such an expression system is first concentrated by using a commercially available protein concentration filter, such as an Amicon or Millipore Pellicon ultrafiltration unit. A protease inhibitor such as PMSF may be included in any of the foregoing steps to inhibit proteolysis, and antibiotics may be contained to prevent the growth of external contaminants.

An antibody prepared from a cell can be purified by using, for example, hydroxylapatite chromatography, gel electrophoresis, dialysis, DEAE-cellulose ion exchange chromatography, ammonium sulphate precipitation, salt precipitation and affinity chromatography, wherein the affinity chromatography is a preferred purification technique.

Following any preliminary purification step, the mixture comprising the target antibody and contaminants can be subjected to low-pH hydrophobic interaction chromatography by using an elution buffer having a pH of about 2.5-4.5, preferably at a low salt concentration (e.g., about 0-0.25M salt).

**Pharmaceutical composition**

In some aspects, the present disclosure relates to a pharmaceutical composition comprising at least one CLDN18.2 binding molecule (e.g., the CLDN18.2 antibody of the present disclosure) as disclosed herein and a pharmaceutically acceptable carrier.

*Components of pharmaceutical composition*

The pharmaceutical composition may optionally contain one or more additional active pharmaceutical ingredients, such as another antibody or drug. The pharmaceutical composition of the present disclosure may also be administered in combination with, for example, another immunostimulant, anticancer agent, antiviral agent or vaccine, so that the anti-CLDN18.2 antibody enhances an immune response to a vaccine. A pharmaceutically acceptable carrier can include, for example, a pharmaceutically acceptable liquid, gel or solid carrier, an aqueous medium, a non-aqueous medium, an antimicrobial agent, an isotonic agent, a buffer, an antioxidant, an anaesthetic, a suspending agent/dispersant, a chelating agent, a diluent, an adjuvant, an excipient or a non-toxic auxiliary substance, a combination of various components known in the art or more.

Suitable components can include, for example, antioxidants, fillers, binders, disintegrants, buffers, preservatives, lubricants, flavouring agents, thickening agents, colouring agents, emulsifying agents or stabilizers such as sugar and cyclodextrin. Suitable antioxidants can include, for example, methionine, ascorbic acid, EDTA, sodium thiosulphate, platinum, hydrogen peroxidase, citric acid, cysteine, mercaptoglycerol, mercaptoacetic acid, mercaptosorbitol, butylmethyl anisole, butylated hydroxytoluene and/or propyl arsenate. In the present disclosure, the one or more antioxidants can be oxidized in a solvent of an antibody or an antigen-binding fragment that comprises one or more antioxidants such as methionine reducing the antibody or the antigen-binding fragment thereof and comprises the composition of the present disclosure. Redox can prevent or reduce the decrease in binding affinity, thereby enhancing the antibody stability and prolonging the shelf life. Therefore, in some embodiments, the present disclosure provides a composition comprising one or more antibodies or antigen-binding fragments thereof and one or more antioxidants such as methionine. The present disclosure further provides a plurality of methods, wherein the antibodies or the antigen-binding fragments thereof are mixed with one or more antioxidants such as methionine. Accordingly, the antibodies or the antigen-binding fragments thereof can be prevented from oxidation, thereby prolonging its shelf life and/or increasing the activity.

For further illustration, the pharmaceutically acceptable carrier can include, for example, an aqueous carrier such as sodium chloride injection, Ringer's injection, isotonic dextrose injection, sterile water injection or dextrose and lactated Ringer's injection; a non-aqueous carrier such as plant-derived fixed oil, cottonseed oil, corn oil, sesame oil or peanut oil; a bacteriostat or an antimicrobial agent at a fungistatic concentration; an isotonic agent such as sodium chloride or glucose; a buffer such as a phosphate or citrate buffer; an antioxidant such as sodium bisulphate; a local anaesthetic such as procaine hydrochloride; a suspending agent and a dispersant such as sodium carboxymethylcellulose, hydroxypropylmethylcellulose or polyvinylpyrrolidone; an emulsifier such as polysorbate 80 (TWEEN-80); and a chelating agent such as EDTA (ethylenediaminetetraacetic acid) or EGTA (ethylene glycol tetraacetic acid), ethanol, polyethylene glycol, propylene glycol, sodium hydroxide, hydrochloric acid, citric acid or lactic acid. The antimicrobial agent used as a carrier can be added to the pharmaceutical composition in a multi-dose container comprising phenol or methylphenol, a mercurial formulation, benzyl alcohol, chlorobutanol, methylparaben and propylparaben, thimerosal, benzalkonium chloride and benzethonium chloride. Suitable excipients can include, for example, water, saline, dextrose, glycerol or ethanol. Suitable non-toxic auxiliary substances can include, for example, a humectant or an emulsifying agent, a pH buffer, a stabilizer, a solubility-enhancing agent or a reagent such as sodium acetate, sorbitan monolaurate, triethanolamine oleate or cyclodextrin.

*Administration, formulation and dose*

The pharmaceutical composition of the present disclosure can be administered *in vivo* to a subject in need thereof by various routes including, but not limited to, routes of oral, intravenous, intraarterial, subcutaneous, parenteral, intranasal, intramuscular, intracranial, intracardiac, intraventricular, intratracheal, buccal, rectal, intraperitoneal, intracutaneous, topical, transdermal and intrathecal administration, or implantation or inhalation. The pharmaceutical composition of the present disclosure can be formulated into a formulation in a solid, semisolid, liquid, or gaseous form, including, but not limited to, tablets, capsules, powders, granules, ointments, solutions, suppositories, enemas, injections, inhalants and aerosols. Suitable formulations and routes of administration can be selected according to intended uses and treatment regimens.

Suitable formulations for enteral administration include hard or soft gelatin capsules, pills, tablets including coated tablets, elixirs, suspensions, syrups or inhalants and controlled release dosage forms thereof.

Formulations suitable for parenteral administration (e.g., by injection) include aqueous or non-aqueous, isotonic, pyrogen-free and sterile liquids (e.g., solutions or suspensions) in which active ingredients are dissolved, suspended or provided otherwise (e.g., in liposomes or other particulates). These liquids can additionally contain other pharmaceutically acceptable ingredients, such as antioxidants, buffers, preservatives, stabilizers, bacteriostats, suspending agents, thickening agents and solutes which render the formulation isotonic with the blood (or other related body fluids) of an intended recipient. Examples of excipients include, for example, water, alcohol, polyols, glycerol, vegetable oil, etc. Examples of isotonic carriers suitable for such formulations include sodium chloride injection, Ringer's solution or lactated Ringer's injection. Similarly, a particular dose regimen (i.e., dose, time, and repetition) depends on a specific individual and the medical history of the individual, and empirical considerations such as pharmacokinetics (e.g., half-life, clearance, etc.).

The frequency of administration can be determined and adjusted during the course of treatment, and maintains the decrease of such tumour cells, decreases the proliferation of tumour cells or delays the development of metastasis on the basis that the proliferation or number of tumourigenic cells is decreased. In some embodiments, the dose administered can be adjusted or reduced so as to control the potential side effects and/or toxicity. Alternatively, a sustained and continuous release formulation of the pharmaceutical composition of the present disclosure for treatment may be suitable.

Those skilled in the art would appreciate that suitable doses may vary from patients. Determining the optimal dose generally involves balancing the level of therapeutic benefits with any risk or unwanted side effect. The dose level selected depends upon various factors including, but not limited to, the activity of a particular compound, the administration, the time of administration, the clearance rate of the compound, the duration of treatment, other drugs, compounds and/or materials used in combination, the severity of a condition, the species, and the sex, age, weight, pathogenetic condition, general health condition and prior medical history of a patient. The dose and route of administration of a compound are ultimately determined by a doctor, veterinarian, or clinician, but the dose is generally selected to reach a local concentration at a site of action that achieves the desired effects without causing substantial unwanted or unfavourable side effects.

Generally, the CLDN18.2 binding molecule can be administered in various dose ranges. In some embodiments, the CLDN18.2 binding molecule provided herein may be administered at a therapeutically effective dose of about 0.01 mg/kg to about 100 mg/kg (e.g., about 0.01 mg/kg, about 0.5 mg/kg, about 1 mg/kg, about 2 mg /kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 55 mg/kg, about 60 mg/kg, about 65 mg/kg, about 70 mg/kg, about 75 mg/kg, about 80 mg/kg, about 85 mg/kg, about 90 mg/kg, about 95 mg/kg or about 100 mg/kg). In certain embodiments of these embodiments, the antibody is administered at a dose of about 50 mg/kg or less, and in certain embodiments of these embodiments, the dose is 10 mg/kg or less, 5 mg/kg or less, 1 mg/kg or less, 0.5 mg/kg or less, or 0.1 mg/kg or less. In certain embodiments, the dose administered may vary during the course of treatment. For example, in certain embodiments, the initial dose administered can be higher than the dose administered subsequently. In certain embodiments, the dose administered may vary during the course of treatment depending on the response of a subject.

In any case, the antibody or the antigen-binding portion thereof of the present disclosure is preferably administered to a subject in need thereof as needed. The frequency of administration can be determined by those skilled in the art, such as an attending physician, based on considerations such as the condition being treated, the age of the subject being treated, the severity of the condition being treated and the general health condition of the subject being treated.

In certain preferred embodiments, the course of treatment that involves the antibody or the antigen-binding portion thereof of the present disclosure comprises multiple doses of the selected drug products administered over a period of weeks or months. More specifically, the antibody or the antigen-binding portion thereof of the present disclosure may be administered every day, every two days, every four days, every week, every ten days, every two weeks, every three weeks, every month, every six weeks, every two months, every ten weeks or every three months. In this regard, it is understandable that the dose can be changed or the interval can be adjusted based on patient responses and clinical practices.

The dose and regimen of the pharmaceutical composition disclosed for treatment can also be determined empirically in an individual who is given one or more administrations. For example, an individual may be given incremental doses of the pharmaceutical composition as described herein. In a selected embodiment, doses can be determined empirically, or increased or reduced gradually according to the side effects or toxicity observed, respectively. To evaluate the efficacy of the selected composition, markers of a particular disease, condition or pathogenetic condition can be tracked. For cancer, these include direct measurement of tumour size via palpation or visual observation; indirect measurement of tumour size by x-ray or other imaging techniques; improvement as evaluated by direct tumour biopsy and microscopic examination of tumour samples; measurement of an indirect tumour marker (e.g., PSA for prostate cancer) or a tumourigenic antigen identified according to the method described herein; decrease in pain or paralysis; improvement of speech, vision, breathing or other disabilities associated with tumours; increased appetite; or improvement of quality of life or prolongation of lifetime as measured by accepted experiments. It would be apparent to those skilled in the art that doses would vary according to the individuals, types of tumour conditions, stages of tumour conditions, the fact whether tumour conditions have begun to metastasize to other locations in individuals, and past and concurrent treatments being used.

A compatible formulation for parenteral administration (e.g., intravenous injection) may comprise the CLDN18.2 binding molecule as provided herein at a concentration of about 10 μg/ml to about 100 mg/ml. In some embodiments, the concentration of the CLDN18.2 binding molecule may comprise 20 μg/ml, 40 μg/ml, 60 μg/ml, 80 μg/ml, 100 μg/ml, 200 μg/ml, 300 μg/ml, 400 μg/ml, 500 μg/ml, 600 μg/ml, 700 μg/ml, 800 μg/ml, 900 μg/ml or 1 mg/ml. In other preferred embodiments, the concentration of the CLDN18.2 binding molecule comprises 2 mg/ml, 3 mg/ml, 4 mg/ml, 5 mg/ml, 6 mg/ml, 8 mg/ml, 10 mg/ml, 12 mg/ml, 14 mg/ml, 16 mg/ml, 18 mg/ml, 20 mg/ml, 25 mg/ml, 30 mg/ml, 35 mg/ml, 40 mg/ml, 45 mg/ml, 50 mg/ml, 60 mg/ml, 70 mg/ml, 80 mg/ml, 90 mg/ml or 100 mg/ml. If calculated by a molar concentration, in some embodiments, the concentration of the CLDN18.2 binding molecule may comprise, for example, 133 nM, 266 nM, 400 nM, 533 nM, 667 nM, 1.3 μM, 2 μM, 2.67 μM, 3.33 μM, 4 μM, 4.67 μM, 5.33 μM, 6 μM or 6.67 μM.

## Use **of the present invention**

The CLDN18.2 binding molecule of the present invention has many *in vitro* and *in vivo* uses.

### *Treatment of diseases*

Conditions and disorders associated with CLDN18.2 may be immune-related diseases or conditions including, but not limited to, "diseases related to cells expressing CLDN18.2" or "diseases associated with cells expressing CLDN18.2" or similar expressions, and means that CLDN18.2 is expressed in cells of diseased tissues or organs. In one embodiment, the expression of CLDN18.2 in cells of diseased tissues or organs is increased in comparison with the state in corresponding healthy tissues or organs. The increase refers to an increase of at least 10%, particularly at least 20%, at least 50%, at least 100%, at least 200%, at least 500%, at least 1000%, at least 10000% or even more. In one embodiment, the expression is only found in diseased tissues, and the expression in corresponding healthy tissues is inhibited. According to the present disclosure, the diseases associated with cells expressing CLDN18.2 include cancer diseases. In addition, according to the present disclosure, the cancer diseases are preferably those in which cancer cells express CLDN18.2.

The term "cancer disease" or "cancer" refers to or describes a physiological condition that is generally characterized by unregulated cell growth in an individual. Examples of the cancer include, but are not limited to, epithelial cancer, lymphoma, blastoma, sarcoma and leukaemia. More particularly, examples of such cancer include bone cancer, blood cancer, lung cancer, liver cancer, pancreatic cancer, skin cancer, head and neck cancer, cutaneous or intraocular melanoma, uterine cancer, ovarian cancer, rectal cancer, cancer of the anal region, gastric cancer, colon cancer, breast cancer, prostate cancer, cancer of sexual organs and reproductive organs, Hodgkin's disease, oesophageal cancer, small intestine cancer, cancer of the endocrine system, thyroid cancer, parathyroid cancer, adrenal cancer, soft tissue sarcomas, bladder cancer, renal cancer, renal cell carcinoma, renal pelvis cancer, central nervous system (CNS) tumour, neuroectodermal cancer, spinal axis tumour, glioma, meningioma and pituitary adenoma. The term "cancer" according to the present disclosure also includes cancer metastasis. Preferably, the "cancer disease" is characterized by cells expressing CLDN18.2 and characterized in that cancer cells express CLDN18.2. Cells expressing CLDN18.2 are preferably cancer cells, preferably cancer cells of the cancer described herein.

According to the present disclosure, the term "tumour" or "tumour disease" refers to abnormal growth of cells (referred to as neoplastic cells, tumourigenic cells or tumour cells), preferably forming swelling or lesions. The "tumour cells" mean abnormal cells that grow by rapid and uncontrolled cellular proliferation and continue to grow after stimulation that triggers the new growth is stopped. Tumours show partial or complete lack of structural organization and functional coordination with the normal tissue, and generally form a distinct mass of tissue, which may be either benign, pre-malignant or malignant. According to the present disclosure, the "cancer disease" is preferably the "tumour disease". However, in general, the terms "cancer" and "tumour" are interchangeably herein.

In one embodiment, the cancer according to the present disclosure relates to cancer cells expressing CLDN18.2. In one embodiment, the cancer is CLDN18.2 positive. In one embodiment, the expression of CLDN18.2 occurs on the surface of cells. In one embodiment, at least 50%, preferably 60%, 70%, 80% or 90% of cancer cells are CLDN18.2 positive, and/or at least 40%, preferably at least 50% of cancer cells are positive for the surface expression of CLDN18.2. In one embodiment, at least 95% or at least 98% of cancer cells are *CLDN18.2* positive. In one embodiment, at least 60%, at least 70%, at least 80% or at least 90% of cancer cells are positive for the surface expression of CLDN18.2.

In one embodiment, cancer expressing CLDN18.2, cancer related to cancer cells expressing CLDN18.2 or CLDN18.2 positive cancer is selected from: gastric cancer, oesophageal cancer, pancreatic cancer, lung cancer (e.g., non-small cell lung cancer (NSCLC)), ovarian cancer, colon cancer, liver cancer, head and neck cancer and gallbladder cancer, and their metastasis, particularly gastric cancer metastasis (e.g., Krukenberg tumour), peritoneal metastasis and lymph node metastasis. In one embodiment, the cancer is adenocarcinoma, particularly advanced adenocarcinoma. Particularly preferred cancer diseases are adenocarcinomas of the stomach, the oesophagus, the pancreatic duct, the bile duct, the lung and the ovary. In one embodiment, the cancer is selected from gastric cancer, oesophageal cancer (particularly cancer of the lower oesophagus), cancer of the oesophagogastric junction and gastrooesophageal cancer.

In a particularly preferred embodiment, the cancer is gastrooesophageal cancer such as metastatic, refractory or recurrent advanced gastrooesophageal cancer.

In addition, the antibody or the antigen-binding portion thereof of the present disclosure can be used in combination with chemotherapy or radiotherapy.

*Combined use with chemotherapy*

The antibody or the antigen-binding portion thereof may be used in combination with an anticancer agent, a cytotoxic agent or a chemotherapeutic agent.

The term "anticancer agent" or "antiproliferative agent" means any agent that can be used to treat a cell proliferative condition such as cancer, and includes, but is not limited to, a cytotoxic agent, a cytostatic agent, an antiangiogenic agent, a radiotherapy and a radiotherapeutic agent, a targeted anticancer agent, BRM, a therapeutic antibody, a cancer vaccine, a cytokine, a hormone therapy, a radiotherapy, an antimetastatic agent and an immunotherapeutic agent. It would be appreciated that, in selected embodiments as described above, such anticancer agents may comprise a conjugate and may bind to the disclosed site-specific antibody prior to administration. More specifically, in some embodiments, the anticancer agent selected is ligated to unpaired cysteines of an engineered antibody to provide an engineered conjugate as described herein. Therefore, such engineered conjugate is explicitly considered as falling within the scope of the present disclosure. In other embodiments, the disclosed anticancer agent is administered in combination with a site-specific conjugate comprising a different therapeutic agent as described above.

As used herein, the term "cytotoxic agent" refers to a substance that is toxic to cells and decreases or inhibits the function of the cells and/or causes destruction of the cells. In some embodiments, the substance is a naturally occurring molecule derived from a living organism. Examples of the cytotoxic agent include, but are not limited to, small-molecule toxins or enzymatically active toxins of bacteria (e.g., *Diptheria* toxin, *Pseudomonas* endotoxin and exotoxin, *Staphylococcal enterotoxin* A), fungi (e.g., $\alpha$-sarcinaxanthin and restrictocin), plants (abrin, ricin, modeccin, viscin, pokeweed antiviral proteins, saporin, gelonin, momoridin, trichosanthin, barley toxin, *Aleurites fordii* proteins, dianthin proteins, *Phytolacca mericana* proteins (PAPI, PAPII and PAP-S), *Momordica charantia* inhibitors, curcin, crotin, *Saponaria officinalis* inhibitors, gelonin, mitegellin, restrictocin, phenomycin, neomycin and trichothecenes) or animals (e.g., cytotoxic RNases such as extracellular pancreatic RNases; and DNase I, including fragments and/or variants thereof).

For the purposes of the present disclosure, the "chemotherapeutic agent" comprises a chemical compound (e.g., a cytotoxic agent or a cytostatic agent) that non-specifically decreases or inhibits the growth, proliferation and/or survival of cancer cells. These chemical agents are generally directed to intracellular processes necessary for cell growth or division, and are thus particularly effective for cancer cells, which generally grow and divide rapidly. For example, vincristine allows for depolymerization of microtubules, and thus inhibits cells from entering mitosis. In general, the chemotherapeutic agent can comprise any chemical agent that inhibits, or is designed to inhibit, cancer cells or cells which are likely to become cancer cells or generate tumourigenic progeny (e.g., TIC). These agents are generally administered in combination, and are generally the most effective, for example, in regimens such as CHOP or FOLFIRI.

Examples of the anticancer agent (either as a component of a site-specific conjugate or in an unconjugated state) that can be used in combination with the site-specific construct of the present disclosure include, but are not limited to, an alkylating agent, alkane sulfonate, aziridine, ethyleneimine and methylmelamine, acetogenins, camptothecin, bryostatin, callystatin, CC-1065, cryptophycins, dolastatin, duocarmycin, eleutherobin, pancratistatine, sarcodictyin, spongistatin, nitrogen mustards, antibiotics, enediyne antibiotics, dynemicin, bisphosphonates, esperamicin, chromophores of enediyne chromoprotein antibiotics, aclacinomysins, actinomycin, antramycin, azaserine, bleomycin, actinomycin C, carabicin, carminomycin, carzinophillin, chromomycinis, dactinomycin, daunorubicin, detorubicin, 6-diazo-5-oxo-L-norleucine, ADRIAMYCIN® doxorubicin, epirubicin, esorubicin, idarubicin, marcellomycin, mitomycin, mycophenolic acid, nogalamycin, olivomycin, peplomycin, potfiromycin, puromycin, triferricdoxorubicin, rodorubicin, streptonigrin, streptozocin, tubercidin, ubenimex, zinostatin, zorubicin; antimetabolites, erlotinib, vemurafenib, crizotinib, sorafenib, ibrutinib, enzalutamide, folic acid analogues, purine analogues, androgens, antiadrenergic drugs, folic acid replenishers such as frolinic acid, aceglatone, aldophosphamide glycoside, aminolevulinic acid, eniluracil, amsacrine, bestrabucil, bisantrene, edatrexate, defofamine, demecolcine, diaziquone, elfomithine, elliptinium acetate, epothilone, etoglucid, gallium nitrate, hydroxyurea, lentinan, lonidainine, maytansinoids, mitoguazone, mitoxantrone, mopidanmol, nitraerine, pentostatin, phenamet, pirarubicin, losoxantrone, podophyllinic acid, 2-ethylhydrazide, procarbazine, PSK® polysaccharide complex (JHS Natural Products, Eugene, OR), razoxane; rhizomycin; sizofiran; spirogermanium; tenuazonic acid; triaziquone; 2,2',2"-trichlorotriethylamine; trichothecenes (particularly T-2 toxin, verracurin A, bacillosporin A and diacetoxysciroenol); urethane; vindesine; dacarbazine; mannomustine; dibromannitol; dibromodulcitol; pipobroman; gacytosine; cytosine arabinoside ("Ara-C"); cyclophosphamide; thiotepa; taxanes; chloranbucil; GEMZAR® gemcitabine; 6-thioguanine; mercaptopurine; methotrexate; platinum analogues; vinblastine; platinum; etoposide (VP-16); ifosfamide; mitoxantrone; vincristine, NAVELBINE® vinorelbine; novantrone; teniposide; edatrexate; daunorubicin; aminopterin; xeloda; ibandronate; irinotecan (Camptosar, CPT-11); topoisomerase inhibitor RFS 2000; difluoromethylomithine; retinoids; capecitabine; combretastatin; leucovorin; oxaliplatin; and inhibitors of PKC-$\alpha$, Raf, H-Ras, EGFR and VEGF-A (which reduce cell proliferation), and a pharmaceutically acceptable salt, acid or derivative of any one of the foregoing. Also included in this definition are an antihormone agent for regulating or inhibiting hormone actions on tumours, such as an antioestrogen, a selective oestrogen receptor modulator, an aromatase inhibitor that inhibits the enzyme aromatase which regulates oestrogen production in the adrenal glands, and an antiandrogen; troxacitabine (a 1,3-dioxolane nucleoside cytosine analogue); an antisense oligonucleotide, and a ribozyme such as a VEGF expression inhibitor and an HER2 expression inhibitor; a vaccine, and PROLEUKIN® rIL-2; a LURTOTECAN® topoisomerase 1 inhibitor; ABARELIX® rmRH; vinorelbine, and esperamicin, and a pharmaceutically acceptable salt, acid or derivative of any one of the foregoing.

*Combined use with radiotherapy*

The present disclosure further provides a combination of an antibody or an antigen-binding portion thereof and a radiotherapy (i.e., any mechanism for locally inducing DNA damage within tumour cells, such as $\gamma$-irradiation. X-rays, UV-irradiation, microwaves and electron emission). Combined therapy using directed delivery of radioisotopes to tumour cells is also considered, and the disclosed conjugate may be used in combination with a targeted anticancer agent or other targeting means. In general, radiotherapy is administered in pulses over a period of time of about 1 to about 2 weeks. Radiotherapy may be administered to subjects with head and neck cancer for about 6 to 7 weeks. Optionally, radiotherapy may be administered as a single dose or as multiple sequential doses.

**Diagnosis**

The present disclosure provides a method for detecting, diagnosing or monitoring proliferative conditions *in vitro* and *in vivo* and a method for screening cells from a patient to identify tumour cells including tumourigenic cells. Such methods include identifying an individual with cancer for treatment or monitoring progression of cancer, comprising bringing a patient or a sample obtained from the patient (either *in vivo* or *in vitro*) into contact with the antibody as described herein and detecting presence or absence, or level of binding, of the antibody binding to bound or free target molecules in the sample. In some embodiments, the antibody comprises a detectable marker or a reporter molecule.
In some embodiments, the binding of the antibody to particular cells in the sample can denote that the sample may contain tumourigenic cells, thereby indicating that the individual with cancer may be effectively treated with the antibody as described herein.
Samples can be analysed by a plurality of assays, for example, radioimmunoassays, enzyme immunoassays (e.g., ELISA), competitive-binding assays, fluorescent immunoassays, immunoblot assays, Western blot analysis and flow cytometry assays. Compatible *in vivo* diagnosis or diagnostic assays can comprise imaging or monitoring techniques that are well known in the art, for example, magnetic resonance imaging, computerized tomography (e.g., CAT scan), positron tomography (e.g., PET scan), radiography and ultrasonic waves, as would be known to those skilled in the art.

**Pharmaceutical package and kit**

The present disclosure further provides a pharmaceutical package and a kit, each of which comprises one or more containers containing one or more doses of the antibody or the antigen-binding portion thereof. In some embodiments, a unit dose is provided, wherein the unit dose contains a predetermined amount of a composition comprising, for example, an antibody or an antigen-binding portion thereof, with or without one or more additional agents. For other embodiments, such unit dose is supplied in single-use prefilled syringe for injection. In other embodiments, the composition contained in the unit dose may comprise saline, sucrose or the like and a buffer such as phosphate, and/or is formulated within a stable and effective pH range. Alternatively, in some embodiments, the composition is a conjugate composition, which may be provided as lyophilized powder and is reconstituted following addition of an appropriate liquid (for example, sterile water or a saline solution). Any label on, or associated with, the container indicates that the encapsulated conjugate composition is used for treating a selected tumour disease condition. In some preferred embodiments, the composition comprises one or more substances that inhibit protein aggregation, including, but not limited to, sucrose and arginine.
The present disclosure further provides a kit for producing single-dose or multi-dose administration units of a site-specific conjugate and optionally, one or more anticancer agents. The kit comprises a container and a label or package insert on or associated with the container. Suitable containers include, for example, bottles, vials and syringes. The container may be formed from various materials such as glass or plastic and contains a pharmaceutically effective amount of the disclosed conjugates in a conjugated or unconjugated form. In other preferred embodiments, the container comprises a sterile access port (for example, the container may be an intravenous solution bag or a vial having a stopper pierceable by a hypodermic injection needle head). Such kit generally contains a pharmaceutically acceptable formulation of an engineered conjugate in a suitable container and optionally, one or more anticancer agents in the same or different container. The kit may also contain other pharmaceutically acceptable formulations, either for diagnosis or combined therapy. For example, in addition to the antibody or the antigen-binding portion thereof of the present disclosure, such kit may contain any one or more anticancer agents such as a chemotherapeutic or a radiotherapeutic agent, an antiangiogenic agent, an antimetastatic agent, a targeted anticancer agent, a cytotoxic agent, and/or other anticancer agents.

More specifically, the kit may have a single container that contains the disclosed antibody or the antigen-binding portion thereof, with or without additional components, or it may have different containers for different desired agents. In the case that a combined therapeutic agent is provided for conjugation, a single solution is combined according to a molar equivalent or pre-mixed in a manner that one component is more than the other. Alternatively, the conjugate and any optional anticancer agent of the kit may be stored separately within different containers prior to administration to a patient. The kit may also comprise a second/third container device for containing a pharmaceutically acceptable sterile buffer or other diluents such as bacteriostatic water for injection (BWFI), phosphate-buffered saline (PBS), Ringer's solution and a glucose solution.

When the components of the kit are provided as one or more liquid solutions, the liquid solution is preferably an aqueous solution, particularly preferably a sterile aqueous solution or a saline solution. However, the components of the kit may be provided as dry powder. When reagents or components are provided as dry powder, the powder can be reconstituted by adding a suitable solvent. It can be assumed that the solvent may also be provided in another container.

As described briefly above, the kit may also contain a tool by which the antibody or the antigen-binding portion thereof and any optional component are administered to a patient, such as one or more needles, an I.V. bag, or a syringe, or even an eye dropper, a pipette or other similar devices by which the formulation may be injected or introduced into the animal body or applied to a diseased area of the body. The kit of the present disclosure generally further comprises a device for containing vials or the like and other tightly closed components for commercial sale, such as an injection or blow-moulding plastic container where the required vials and other devices are placed and maintained.

## Advantages of the present invention

Development of antibodies against CLDN18.2, particularly human CLDN18.2, has at least the following advantages:

firstly, human CLDN18.2 is highly expressed in cancer cells but is only specifically expressed in gastric epithelial cells in normal cells, and therefore the toxic and side effects are low and the druggability is higher;

secondly, CLDN18.2 is expressed in the tight junctions of gastric epithelial cells which are less easily susceptible to antibodies in comparison with relatively loose cancer cells, and even if epithelial cells are killed by the antibodies, stem cells beneath the epithelial cells can regenerate the gastric epithelial cells damaged by means of differentiation as they do not express CLDN18.2; and

thirdly, the antibodies against CLDN18.2 not only can kill cells by means of ADCC and CDC, but also can mediate cell apoptosis by the cross-linking with CLDN18.2, and can inhibit cell proliferation to a certain extent.

Caplacizumab, a nanobody (as a new antibody), has been approved for marketing, which fully demonstrates the druggability of the nanobody. Such antibody, relative to other antibodies, has significant advantages, such as adjustable half-life achieved by chemical modification or protein fusion and engineering, strong penetration, recognition of hidden epitopes unavailable to common antibodies, pepsin tolerance, acid resistance, heat resistance, easy production, and easy assembling into bivalent antibodies and multivalent antibodies with other types of antibodies due to its nature as a single chain.

In some embodiments of the present disclosure, the antibody against CLDN18.2 is obtained by screening an alpaca immune library, and on this basis, the C-terminus of the antibody is fused to the Fc fragment of IgG1. At present, the experimental results of CDC and ADCC at a cellular level of some candidate antibodies show that the candidate antibodies exhibit activities better than or at least comparable to that of the control antibody. In combination with the property of a CLDN18.2 target, such antibody is used for cancer immunotherapy, has lower toxic and side effects and better clinical efficacy, and provides patients with more drug options.

## Examples

The present invention generally described herein will be understood more readily by reference to the following Examples, which are provided by way of illustration and are not intended to limit the present invention. The Examples are not intended to represent that the experiments below are all or only experiments performed.

**Example 1**

**Construction and identification of overexpressing cell lines and tumour cell lines**

In this example, different types of overexpressing cell lines and tumour cell lines were constructed, respectively and identified by flow cytometry.

1. Construction and identification of overexpressing cell lines

The nucleic acid sequences of full-length human CLDN18.1 (SEQ ID NO: 17), murine CLDN18.2 (SEQ ID NO: 18) and murine CLDN18.1 (SEQ ID NO: 19) were constructed into pLVX-puro plasmids (Clontech, Cat#632164). Then, the resulting plasmids were transformed into HEK293 cells (ATCC® CRL-1573™) via electrotransformation. By means of screening, overexpressing cell lines expressing full-length human CLDN18.1 (human CLDN18.1-HEK293), murine CLDN18.2 (murine CLDN18.2-HEK293) and murine CLDN18.1 (murine CLDN18.1-HEK293) were obtained. Subsequently, the flow cytometry identification was performed via the IMAB362 antibody (obtained by expression and purification according to the sequence information disclosed in the patent US20180127489A1) and the anti-CLDN18 antibody [34H14L15] (Abeam, ab203563) that recognizes the C-terminal intracellular segment of CLDN18 (GFKASTGFGSNTKN, SEQ ID NO: 22), and overexpressing cell lines were successfully obtained. The specific method is as follows.

1.1 Electrotransformation

Firstly, HEK293 cells were revived and cultured and serially passaged 2-3 times. One day prior to transfection, the cells were plated at a density of $3 \times 10^5$ cells/mL in a cell culture dish and could be used the next day when the cell confluence reached about 70%. The cells were digested with Trypsin (Gibco, 25200-072) containing 0.25% by volume of EDTA for 2 min, then collected, and centrifuged at 100 g at room temperature for 5 min. The supernatant was discarded, and the cells were resuspended by adding $1 \times$ DPBS (Shanghai BasalMedia Technologies Co., LTD., B210) and counted. A total of $5 \times 10^6$ cells were taken, centrifuged, collected and resuspended with 250 $\mu$L of Buffer R (Invitrogen, Neon™ Kit, PK10096), to which 25 $\mu$g of target plasmids were added, and the resulting solution was mixed gently and uniformly with a pipette. After that, the suspension was placed into an electroporator (Invitrogen, NeonTM Transmission System, MP922947), and electrotransformation was performed under the reaction condition set as 1100 V/20 ms/2 times.

1.2 Cell culture

After electrotransformation, the cells thus obtained were transferred to an antibiotics-free DMEM culture medium (Gibco, 11995065) containing 10% by volume of FBS (Gibco, 15140-141), respectively. Then, the cells were plated into a 10 cm $\times$ 10 cm cell culture dish and cultured for 48 h. Next, the cells were plated into a 96-well cell culture plate at a density of 0.5 cells/well on average. Puromycin (Gibco, A111138-03) at a final concentration of 2 $\mu$g/mL was added for screening, and the growth situation of cell line clones was observed after about 2 weeks, and the cloned cell lines were picked for identification.

1.3 Flow cytometry identification of overexpressing cell lines

1.3.1 Flow cytometry identification of human CLDN18.2-HEK293T and murine CLDN18.2-HEK293 cell lines

Human CLDN18.2-HEK293T (KYinno Biotechnology Co., Ltd., KC-0986) and murine CLDN18.2-HEK293 cell lines were directly identified by using IMAB362 antibodies.
A total of $1 \times 10^5$ cells were taken and centrifuged at low speed (300 g), and the supernatant was removed. The cells at the bottom of the centrifuge tube were rinsed once with the formulated FACS buffer ($1 \times$ PBS buffer containing 2% by volume of FBS). Then, 12.5 $\mu$g/mL of the antibody IMAB362 was added to the rinsed cells, and the mixture was incubated at 4 °C for 1 h. Next, the cultures were rinsed three times with the above-mentioned FACS buffer, and 0.5 $\mu$g of PE-labelled goat anti-human IgG Fc antibody (Abeam, ab98596) was added. The resulting mixture was incubated at 4 °C for 1 h. Subsequently, the cultures were rinsed three times with the FACS buffer, and then 200 $\mu$L of FACS buffer was added to the cells and the cells were resuspended, and finally, the cells were detected by a flow cytometer (Beckman, CytoFLEX AOO-1-1102).

1.3.2 Flow cytometry identification of human CLDN18.1-HEK293 and murine CLDN18.1-HEK293 cell lines

Since the IMAB362 antibody only recognized CLDN18.2 but did not recognize CLDN18.1, human CLDN18.1-HEK293 and murine CLDN18.1-HEK293 cell lines needed to be identified in conjunction with other methods. Considering the sequence identity of human CLDN18.1 and murine CLDN18.1, the cells were firstly fixed and permeabilized according to the method in the instruction of a cell permeabilization kit (eBioscience, 88-8824-00). Then, flow cytometry identification was performed according to the recognition results of the C-terminal intracellular segment of CLDN18 by the anti-CLDN18 antibody [34H14L15] and the specific binding results with the IMAB362 antibody.

The specific method is as follows. A total of $1 \times 10^5$ cells were taken and centrifuged at low speed (300 g), and the supernatant was removed. The cells at the bottom of the centrifuge tube were rinsed once with the FACS buffer, then 200 $\mu$L of IC fixation solution (eBioscience, 00-8222) was added to the rinsed cells, and the mixture was incubated at 4 °C for 1 h. Next, the cultures were rinsed twice with a permeabilization buffer (eBioscience, 00-8333), and the aforementioned anti-CLDN18 antibody was added. The resulting mixture was incubated at 4 °C for 1 h. The cultures were rinsed additionally three times with the above-mentioned permeabilization buffer, then 0.5 $\mu$g of Alexa Fluor® 488 fluorescently-labelled donkey anti-rabbit IgG H&L (abeam, ab150073) was added, and the mixture was incubated at 4 °C for 1 h. Finally, the cells were detected by a flow cytometer (Beckman, CytoFLEX AOO-1-1102).

In addition, the binding of the IMAB362 antibody to human CLDN18.1-HEK293 and murine CLDN18.1-HEK293 cell lines was determined by using the method as described in section 1.3.1 above.

The results of flow cytometry identification are as shown in FIG. 1a. It shows that, the anti-CLDN18 antibody could recognize human CLDN18.2-HEK293T, human CLDN18.1-HEK293, murine CLDN18.2-HEK293, murine CLDN18.1-HEK293, HEK293 and HEK293T cells; and the antibody IMAB362 only recognized murine CLDN18.2-HEK293 and human CLDN18.2-HEK293T, but did not recognize murine CLDN18.1-HEK293 and human CLDN18.1-HEK293. This illustrated that the four cell lines, human CLDN18.2-HEK293T, human CLDN18.1-HEK293, murine CLDN18.2-HEK293 and murine CLDN18.1-HEK293, all successfully expressed corresponding CLDN18 proteins.

2. Construction and identification of overexpressing tumour cell lines

A gastric cancer cell line KATOIII overexpressing human CLDN18.2 (human CLDN18.2-KATOIII tumour cell line) was constructed by using lentiviral transfection and identified by the antibody IMAB362. The specific method was as follows.

A total of $5 \times 10^4$ human gastric cancer cells KATOIII (ATCC® HTB-103™) in good conditions were taken, and packaged lentiviruses containing human CLDN18.2 sequences (SEQ ID NO: 16) were added at an MOI ratio of 30 : 1, and the mixture was mixed fully and uniformly. Then, an IMDM complete culture medium (Gibco, 12440061) containing 8 $\mu$g/mL polybrene (Sigma, 107689) was added, and the mixture was mixed uniformly and incubated in a 37 °C, 5% $CO_2$ constant-temperature incubator for 20 h. Subsequently, the culture medium was removed and replaced by a fresh IMDM complete medium, and the mixture was incubated for another 24 h. Then, the transfected KATOIII cells were plated into a 96-well plate at a density of 0.5 cells/well on average, and puromycin at a final concentration of 2 $\mu$g/mL was added for pressurized resistance screening. The mixture was cultured in a 37 °C, 5% $CO_2$ constant-temperature incubator for 2-3 weeks, and clones were picked for identification.

The cell lines obtained through antibiotic screening were identified with the antibody IMAB362 by using flow cytometry according to the same method as that described in section 1.3.1 above.

The results of flow cytometry identification are as shown in FIG. 1b. As could be seen from FIG. 1b, KATOIII cells not transfected with CLDN18.2 were barely recognized by the antibody IMAB362, which illustrated that almost no CLDN18.2 or only a very small amount of CLDN18.2 was expressed on the KATOIII cell line; and human CLDN18.2-KATOIII tumour cell lines successfully constructed by lentiviral transfection could be recognized by the antibody IMAB362, which illustrated that the cell line was successfully constructed.

**Example 2**

**Animal immunization and immune titre detection in serum**

1. Immunization

In this example, alpaca immunization was used. The specific operation is as follows. The cell line human CLDN18.2-HEK293T (KYinno Biotechnology Co., Ltd., KC-0986) and hCLDN18.2-pLVX-puro plasmids containing human CLDN18.2 ECD1 (SEQ ID NO: 20) were used as immunogens. Alpacas (Nanchang Dajia Animal Raising Co., Ltd., alpaca code: NSY002) were immunized with $2 \times 10^7$ human CLDN18.2-HEK293T cells (subcutaneous multi-point injection) and 2 mg of plasmids (intramuscular multi-point injection) alternately by week, for a total of 8 immunizations. Finally, $2 \times 10^7$ human CLDN18.2-HEK293T cells were used for boosting immunization.

2. Immune titre determination in serum

Immune titre determination was performed by an ELISA method based on the signal of the immune serum on the recombinant antigenic protein CLDN18.2 (Genscript Biotech Corporation, CP0007). The specific method is as follows.

On the day before immune titre determination, the recombinant antigenic protein CLDN18.2 was diluted with PBS to obtain a 1 μg/mL diluent. The obtained diluent (30 μL) was added to an ELISA plate, and the plate was coated overnight at 4 °C. During the day of immune titre determination, the coated plate was rinsed twice with PBS, then blocked with PBST containing 5% by volume of skim milk powder at room temperature for two hours and subsequently rinsed twice with PBS. On another 96-well dilution plate, the unimmunized negative serum and the immunized serum were diluted with PBS, wherein the first well was subjected to 1000-fold dilution and then the following 7 wells were subjected to 2-fold gradient dilution. The diluted serum was correspondingly added to the first ELISA plate coated with the recombinant antigenic protein CLDN18.2; the mixture was incubated at 37 °C for 1 h and washed twice with PBS; and then a secondary antibody MonoRab™ rabbit anti-camelid VHH antibody (Genscript Biotech Corporation, A01862-200) was added at 1 : 5000 dilution. Finally, the OD values were read at a wavelength of 450 nm by a microplate reader (Molecular Devices, SpectraMax 190). As shown from the results in Table 1, the immune titre of alpaca was about 1 : 8000.

Table 1

| Serum dilution ratio | OD value | |
|---|---|---|
| | Serum of negative alpaca | Serum of immunized alpaca NSY002 |
| 1 : 1000 | 0.045 | 0.5618 |
| 1 : 2000 | 0.0457 | 0.2796 |
| 1 : 4000 | 0.0452 | 0.1471 |
| 1 : 8000 | 0.0458 | 0.1028 |
| 1 : 16000 | 0.0454 | 0.0694 |
| 1 : 32000 | 0.0459 | 0.0616 |
| 1 : 64000 | 0.045 | 0.0574 |
| 1 : 128000 | 0.0453 | 0.0501 |

**Example 3**

**Construction and screening of alpaca immune library**

After the completion of the animal immunization, 80 mL of blood was collected from the alpaca, and PBMCs were isolated with a Ficoll-Paque density gradient separation solution (GE, 17144003S) for construction of an alpaca immune library. The specific method is as follows.

A total of 15 mL of Ficoll-Paque density gradient separation solution was taken and slowly added to a 50-mL centrifuge tube, and then 15 mL of alpaca blood collected was slowly added so that the liquids maintain a clear separation interface. Centrifugation was performed at about 15 °C under the conditions of 400 g, an acceleration of 3 and a deceleration of 0 for 20 min. After the centrifugation, the entire liquid was divided into four layers, with the top layer being a plasma mixture, the bottom layer being red blood cells and granulocytes, the middle layer being Ficoll-Paque liquid, and the junction of the top and middle layers being a narrow zone of white misty layer mainly composed of PBMC, i.e., a PBMC cell layer. Firstly, the plasma mixture on the top layer was carefully pipetted with a sterile Pasteur pipette, and then the PBMCs were pipetted with a new sterile Pasteur pipette to obtain isolated PBMCs. The obtained PBMCs were rinsed twice with PBS, then centrifuged horizontally at 1500 rpm at 4 °C for 10 min, finally resuspended with 1.5 mL of PBS and counted by a cell counter (CountStar, CountStar Altair).

RNAs were extracted from the isolated PBMCs, and reverse-transcribed into cDNA by a reverse transcription kit (TaKaRa, 6210A). Since the molecular form of an alpaca antibody, which had no light chain and had a heavy chain without CH1, was different from that of a common antibody, two fragments of different sizes were obtained by designing primers at the front end of VH germline genes and on CH2 by means of PCR, and a smaller target fragment was recovered by gel extraction. A degenerate primer containing NcoI and NotI restriction sites was designed by aligning all sequences of all the V genes and J genes of a VHH antibody, and then all VHH genes were amplified by using the recovered DNA fragment product as a template. Finally, a target antibody gene fragment was inserted into a phage display vector by means of double enzyme digestion and ligation, wherein the VHH was fused to a GIII gene at the C terminus. The ligation product was recovered by a recovery kit (Omega, D6492-02) and finally transformed into competent *E. coli* SS320 (Lucigen, MC1061 F) by an electroporator (Bio-Rad, MicroPulser), and coated on an ampicillin-resistant 2-YT solid plate (formulated from 1.5% tryptone, 1% yeast extract, 0.5% NaCl and 1.5% agar, by mass/volume (g/mL)). To calculate the library size, the total number of all clones formed by electrotransformation, i.e., the library size, was calculated by using the clones formed on the plate from 1 μL of bacterial solution after dilution. The library size of this immune library was $1 \times 10^9$ cfu.

Based on the library size, 50 ODs (1 OD was $5 \times 10^8$ cfu) of alpaca library bacteria were picked and added to a fresh 2-YT liquid culture medium, so that the initial OD value was 0.05. The mixture was cultured at 37 °C and 220 rpm to a log growth phase. At this time, VSCM13 helper phages were added in an amount 5 times the number of bacteria, and the mixture was mixed uniformly, left to stand for 30 min, then cultured at 220 rpm for 1 h and centrifuged at 10000 rpm for 5 min. The supernatant was discarded, and the residues were transferred to a C+/K+ 2-YT culture medium, and cultured at 30 °C and 220 rpm overnight. On the next day, the resulting cultures were centrifuged at 13000 g for 10 min, and the supernatant thus obtained was precipitated by adding 20% PEG/NaCl (formulated from 20% PEG6000 and 2.5 M NaCl by volume concentration) to obtain phages corresponding to the alpaca library. The phages were rinsed once with PBS and then screened.

Antibodies against human CLDN18.2 were screened from the phage display library by using a cell screening method, with human CLDN18.2-HEK293T cell lines as screening antigens. The specific method was described as follows. Human CLDN18.2-HEK293T or human CLDN18.1-HEK293 was cultured in a T25 square culture flask. When the cultures grew to a density of about 90% (the growth state was the best at this time), the culture supernatant was removed, and the residues were rinsed once with PBS (Shanghai BasalMedia Technologies Co., LTD., B310KJ), then fixed with 5 mL of 4% paraformaldehyde (Sangon Biotech (Shanghai) Co., LTD., E672002-0500) for 1 h, and finally rinsed twice with PBS and then could be used as antigen materials for cell screening of phages. At the time of screening, the phages corresponding to the alpaca library were firstly incubated with the fixed human CLDN18.1-HEK293T cells in a square culture flask at room temperature for 1 h. Then, the phages in the supernatant obtained after adsorption were pipetted and incubated with the fixed human CLDN18.2-HEK293T cells in the square culture flask for 2 h. The resulting cultures were rinsed twice with PBS, and 3 mL of glycine-HCl (pH 2.0) was added, and the resulting mixture was mixed gently and uniformly for 10 min to elute the phages that specifically binded to the target membrane protein CLDN18.2. Next, SS320 bacterial cells (Lucigen, 60512-1) in a log phase were infected with the eluted supernatant, and the mixture was left to stand for 30 min. After that, the resulting mixture was cultured at 220 rpm for 1 h. VSCM13 helper phages were then added, and the mixture was left to stand for 30 min, cultured at 220 rpm for another 1 h, centrifuged and transferred to a C+/K+ 2-YT culture medium. The finally obtained phages were used for a second round of screening. The procedures were repeated as such, and sequence analysis was performed on 10 clones randomly selected in each round. The results showed that after three rounds of screening, sequence enrichment was obvious after the third round of screening.

The clones obtained after the third round of screening were picked and placed into a 96-well plate for preparing phage supernatant, and the positive clones against the CLDN18.2 recombinant protein were screened by phage ELISA. After that, all positive clones were picked and subjected to sequencing analysis, and then the phage supernatant prepared from the clone with the unique sequence was further verified at the level detected by flow cytometry, thereby obtaining candidate antibodies that specifically bind to human CLDN18.2 but not human CLDN18.1.

A specific verification method at the level detected by flow cytometry is as follows.

Firstly, $1 \times 10^5$ human CLDN18.2-HEK293T and human CLDN18.1-HEK293 cells were taken, respectively and centrifuged at a low speed of 500 g, and the supernatant was removed. The cells were rinsed with the FACS buffer as described in section 1.3.1, then the phages blocked with 10% FBS (Gibco, 15140-141) for 1 h were added to prepare a supernatant, and the resulting mixture was incubated at 4 °C for 1 h, and then rinsed twice with the FACS buffer. An anti-M13 murine-derived monoclonal antibody (Sino Biological, 11973-MM05T) was added at 1 : 50, and the resulting mixture was incubated at 4 °C for 1 h, and then rinsed twice with the FACS buffer. An APC-labelled anti-murine Fc secondary antibody (Jackson, 115136071) was added, and the resulting mixture was incubated at 4 °C for 1 h, rinsed twice with the FACS buffer, and finally detected by a flow cytometer (Beckman, CytoFLEX AOO-1-1102). The results showed that NA4-S, NA7-S and NA8-S could specifically bind to human CLDN18.2-HEK293T cells, but did not bind to human CLDN18.1-HEK293 cells. These antibodies were constructed in full length for further verification.

The amino acid sequences (SEQ ID NOs) of VHHs and CDR1, CDR2 and CDR3 defined using the IMGT or AbM numbering system of clones NA4-S, NA7-S and NA8-S are as shown in Table 2.

Table 2

| Clone no. | IMGT | | | AbM | | | VHH |
|---|---|---|---|---|---|---|---|
| | CDR1 | CDR2 | CDR3 | CDR1 | CDR2 | CDR3 | |
| NA4-S | 1 | 2 | 3 | 30 | 31 | 32 | 10 |
| NA7-S | 4 | 5 | 6 | 37 | 38 | 39 | 11 |
| NA8-S | 7 | 8 | 9 | 45 | 46 | 47 | 12 |

**Example 4**

**Generation and expression of chimeric VHH-Fc (hIgG1) antibody**

CLDN18.2 is highly expressed on cells of cancer such as gastric cancer, and antibody drugs against such tumour-related targets can kill tumours by means of complement-dependent cytotoxicity (CDC) and antibody-dependent cell-mediated cytotoxicity (ADCC). In this example, a chimeric antibody was designed on the basis of the candidate nanobodies obtained by screening in Example 3, and was expressed for use in subsequent CDC and ADCC experiments. Considering the specificity of the target, when genes of a candidate nanobody were constructed on a plasmid pcDNA3.4 (Thermofisher, A14697) for transient transfection and expression, a human IgG1 Fc fragment (SEQ ID NO: 21) was fused to the C terminus of the candidate nanobody, wherein this fragment included a linker region and a constant region of IgG1 for mediating the effects such as ADCC and CDC. In this example, candidate nanobodies NA4-S (SEQ ID NO: 10), NA7-S (SEQ ID NO: 11) and NA8-S (SEQ ID NO: 12) were selected and fused to human IgG1 Fc fragments on their respective bases, and Fc-fused chimeric antibodies NA4-S (also referred to as "candidate antibody NA4-S", "antibody NA4-S" or "NA4-S antibody" in the present disclosure, which was a chimeric antibody obtained by the fusion of the candidate nanobody NA4-S and the human IgG1 Fc), NA7-S (also referred to as "candidate antibody NA7-S", "antibody NA7-S" or "NA7-S antibody" in the present disclosure, which was a chimeric antibody obtained by the fusion of the candidate nanobody NA7-S and the human IgG1 Fc), and NA8-S (also referred to as "candidate antibody NA8-S", "antibody NA8-S" or "NA8-S antibody" in the present disclosure, which was a chimeric antibody obtained by the fusion of the candidate nanobody NA8-S and the human IgG1 Fc) were obtained, respectively.

The antibodies NA4-S, NA7-S and NA8-S were expressed by using an ExpiCHO transient transfection and expression system (Gibco, A29133). The specific method was as follows.

During the day of transfection, it was confirmed that the cell density was about $7 \times 10^6$ to $1 \times 10^7$ living cells/mL, and the cell viability was > 98%. At this time, cells were adjusted to a final concentration of $6 \times 10^6$ cells/mL by using 25 mL of fresh ExpiCHO expression culture medium preheated at 37 °C; a target plasmid (25 μg in total) was diluted with 1 mL of OptiPRO™ SFM precooled at 4°C; and meanwhile, 80 μL of ExpiFectamine™ CHO was diluted with 920 μL of OptiPRO™ SFM. Then, the two were mixed uniformly by gentle pipetting to prepare an ExpiFectamine™ CHO/plasmid DNA mixed solution. The mixed solution was incubated at room temperature for 1-5 min, then transferred to the prepared cell suspension by slow addition, which was gently shaken at the same time, and finally placed in a cell culture shaker and cultured under 37 °C, 8% $CO_2$ conditions.

ExpiCHO™ Enhancer and ExpiCHO™ Feed were added within 18-22 h after transfection, and the shake flask was placed in a 32 °C shaker under 5% $CO_2$ for further culture. On the fifth day after transfection, ExpiCHO™ Feed with the same volume was added slowly, and meanwhile the cell suspension was mixed gently and uniformly. On day 12-15 after transfection, the cell expression supernatant was centrifuged at a high speed (15000 g, 10 min), and the obtained supernatant was subjected to affinity purification with Protein A (Millipore, P2545). Subsequently, the target protein was eluted with 100 mM sodium acetate (pH 3.0) and then neutralized with 1 M Tris-HCl. And finally the resulting proteins (i.e., antibodies NA4-S, NA7-S and NA8-S) were transferred into the PBS buffer by means of centrifugal filter (Millipore, UFC901096).

**Example 5**

**Determination of specific binding and cross-species specific binding of candidate antibodies NA4-S, NA7-S and NA8-S**

By taking the candidate antibody NA4-S as an example, human CLDN18.2-HEK293T, HEK293T, HEK293, human CLDN18.1-HEK293, murine CLDN18.2-HEK293 and murine CLDN18.1-HEK293 cells in good growth conditions were firstly digested with Trypsin (Gibco, 25200-072) containing 0.25% EDTA. A total of $1 \times 10^5$ cells were taken and incubated with 10 μg/mL of the candidate antibody NA4-S for 1 h, and additionally, the hIgG1 isotype antibody was used as the control. The resulting mixture was rinsed twice with the FACS buffer, and then incubated with 0.5 $\mu$g of PE-labelled goat anti-human IgG-Fc secondary antibody (Abeam, ab98596) at 4 °C for 1 h. Subsequently, the resulting cultures were rinsed three times with the FACS buffer, and the binding of the candidate antibody on the cells was detected by a flow cytometer (Beckman, CytoFLEX AOO-1-1102).

The specific binding and cross-species specificity of candidate antibodies NA7-S and NA8-S were determined by using the same method as that against the NA4-S.

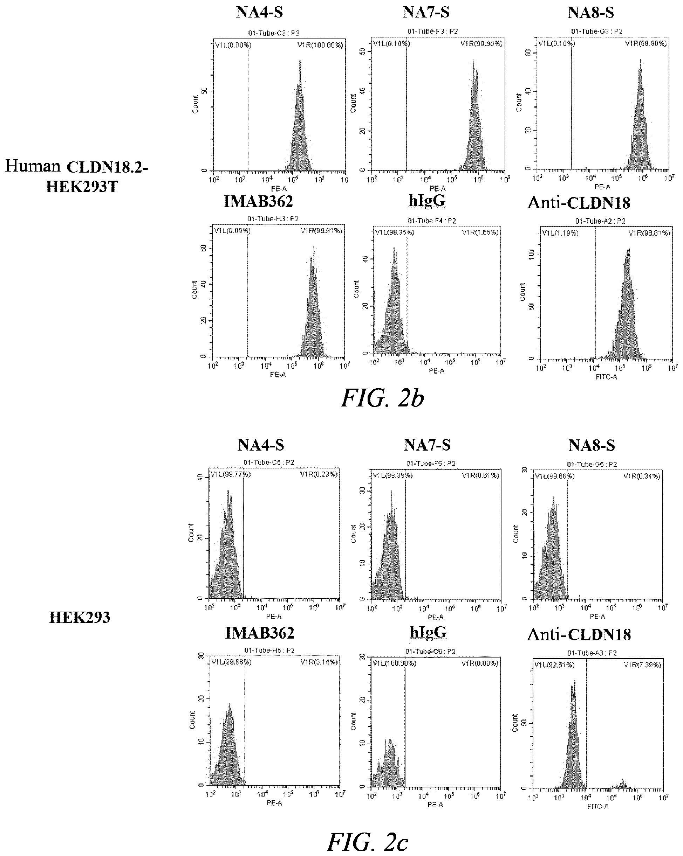

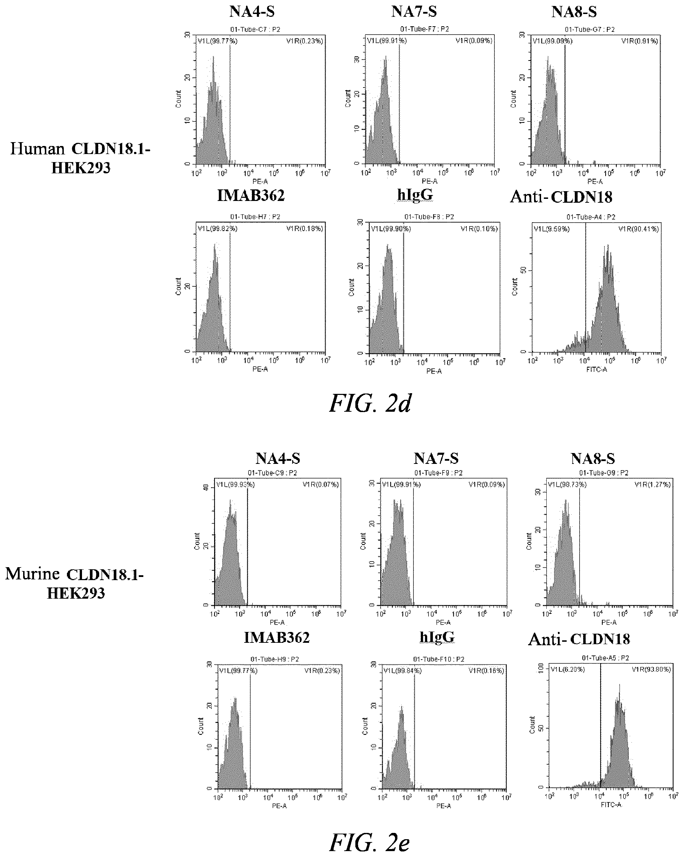

The results of flow cytometry detection (as shown in FIG. 2) showed that, similar to the control antibody IMAB362, the candidate antibodies NA4-S, NA7-S and NA8-S specifically binded to human CLDN18.2-HEK293T and murine CLDN18.2-HEK293, but did not bind to the HEK293T, HEK293, human CLDN18.1-HEK293 and murine CLDN18.1-HEK293 cells. This illustrated that the candidate antibodies NA4-S, NA7-S and NA8-S of the present disclosure could specifically bind to CLDN18.2 but did not bind to CLDN18.1, and performed cross recognition on human and murine CLDN18.2.

## Example 6

### Comparison of binding abilities of candidate antibodies NA4-S, NA7-S and NA8-S on human CLDN18.2-HEK293T cell lines and human CLDN18.2-KATOIII tumour cell lines

Well-cultured human CLDN18.2-HEK293T cells were taken and incubated with candidate antibodies NA4-S, NA7-S and NA8-S obtained after three-fold gradient dilution at 4 °C for 1 h, respectively. The resulting cultures were rinsed twice with the FACS buffer. Then, 0.5 $\mu$g of PE-labelled goat anti-human IgG-Fc antibody (Abeam, ab98596) was added, and the resulting mixture was incubated at 4 °C for 1 h, rinsed twice with the FACS buffer and then detected by a flow cytometer.

The results of flow cytometry detection are shown in FIG. 3a and FIG. 3b, and show that the candidate antibodies NA4-S, NA7-S and NA8-S all exhibited the cell binding activity comparable to or even better than that of the control antibody on human CLDN18.2-HEK293T cells.

The binding abilities of the candidate antibodies NA4-S, NA7-S and NA8-S on human CLDN18.2-KATOIII tumour cell lines were determined by using the same method. The results of flow cytometry detection are as shown in FIG. 3c, and the results show that, the candidate antibodies NA7-S and NA8-S exhibited the cell binding activity comparable to or even better than that of the control antibody on human CLDN18.2-KATOIII tumour cell lines; and the NA4-S hardly binded to human CLDN18.2-KATOIII tumour cell lines, which illustrated that CLDN18.2 proteins from two cells CLDN18.2-HEK293T and CLDN18.2-KATOIII were different in structure and post-translational modifications such as glycosylation.

## Example 7

### Complement-dependent cytotoxicity (CDC) of candidate antibodies NA4-S, NA7-S and NA8-S

The CDC cell killing effect of candidate antibodies was determined by using an MTS method. Candidate antibodies NA4-S, NA7-S and NA8-S could kill cells by means of CDC due to the inclusion IgG1 Fc fragments, and an MTS reagent could be reduced to a coloured compound by NADPH or NADH produced by living cells, so the shading of the colour represents the killing effect of the antibody-mediated CDC cell killing effect. The specific operation method is as follows.

By taking the candidate antibody NA4-S as an example, well-cultured human CLDN18.2-HEK293T cells were firstly digested with trypsin. A total of $5 \times 10^4$ cells were taken and mixed with rabbit serum subjected to 5-fold dilution, then the candidate antibody NA4-S and the control antibody IMAB362 subjected to gradient dilution were added, respectively, at 50 $\mu$L each, and the resulting mixture was incubated at 37°C for 3 h. Then, 30 $\mu$L of MTS reagent (Promega, G3580) was added to the candidate antibody NA4-S and the control antibody IMAB362, respectively, and the resulting mixture was mixed fully and uniformly, placed in a 37 °C, 5% $CO_2$ constant-temperature incubator and cultured for 4 h, during which period the colour change of the culture medium was observed and the OD value at a wavelength of 492 nm was measured by a microplate reader. 10% Triton X-100 plus target cells were used as the complete lysis control; target cells only were used as the blank negative control; and rabbit complements plus target cells were used as the background negative control.

The cell killing rate was calculated according to the following formula: cell killing rate (%) = (OD value of candidate antibody well - OD value of background well)/(OD value of complete lysis well - OD value of blank well) $\times$ 100%.

The results of CDC killing effect of the candidate antibody NA4-S on human CLDN18.2-HEK293T cells are as shown in FIG. 4a. As could be seen from this figure, the candidate antibody NA4-S at an equal molar concentration exhibited a CDC cell killing effect comparable to that of the control antibody IMAB362, and the $EC_{50}$ values of the CDC cell killing effects of NA4-S and IMAB362 were 0.7317 nM and 0.6125 nM, respectively. The CDC killing effects of the candidate antibodies NA7-S and NA8-S on human CLDN18.2-HEK293T cells were measured by using the same method, and the results were as shown in FIG. 4b and FIG. 4c. As could be seen from these figures, the candidate antibodies NA7-S and NA8-S at equal molar concentrations also exhibited CDC cell killing effects comparable to that of the control antibody IMAB362, and the $EC_{50}$ values of the CDC cell killing effects of NA7-S and IMAB362 were 0.5069 nM and 0.6285 nM, respectively; the $EC_{50}$ values of the CDC cell killing effects of NA8-S and IMAB362 were 0.4782 nM and 1.863 nM, respectively.

The CDC killing effects of the candidate antibodies NA7-S and NA8-S on human CLDN18.2-KATOIII tumour cells were measured by using the same method, and the results are as shown in FIGs. 4d and 4e.

As could be seen from FIG. 4d, NA7-S at an equal molar concentration exhibited a CDC cell killing effect stronger than that of the control antibody IMAB362, wherein the $EC_{50}$ value of the nanobody NA7-S was 6.853 nM, and the $EC_{50}$ value of the IMAB362 was 69.79 nM.

As could be seen from FIG. 4e, NA8-S at an equal molar concentration exhibited a CDC cell killing effect stronger than that of the control antibody IMAB362, wherein the $EC_{50}$ value of the nanobody NA8-S was 11.25 nM, and the $EC_{50}$ value of IMAB362 was 1865 nM.

In summary, the CDC killing effects mediated by the candidate antibodies NA4-S, NA7-S and NA8-S on CLDN18.2-HEK293T and CLDN18.2-KATOIII cells were better than that of the control antibody IMAB362.

**Example 8**

**Antibody-dependent cell-mediated cytotoxicity (ADCC)**

The ADCC effect was detected by using a lactate dehydrogenase (LDH) release method. The principle is that a variable region of an antibody binds to a target antigen on a target cell, and after an Fc fragment of the antibody binds to FcRIIIa (also known as CD16a) on NK effector cells in PBMC, the NK cells released perforin, granzymes, etc. to lyse the target cell; and then the release of lactate dehydrogenase in the cell supernatant can be detected by an LDH lactate dehydrogenase kit (Takara, MK401), so as to determine the killing degree of the target cell by the NK cells. The specific operation is as follows.

50 μL of human CLDN18.2-HEK293T cells at a density of $2 \times 10^5$ cells/mL were added to each well in a 96-well cell culture plate and incubated overnight (16-20 h) in a 37°C incubator. 50 μL of candidate antibody NA7-S or NA8-S subjected to gradient dilution was added, and the resulting mixture was mixed uniformly and incubated in a 37 °C incubator for 20 min. Then, the revived human PBMC cells (the ratio of effector cells/target cells being 50 : 1) were added at $5 \times 10^5$ cells/well, and the resulting mixture was incubated in a 37 °C incubator for 4 h and centrifuged at 300 g to obtain the supernatant. After that, an LDH detection reagent was added, and the resulting mixture was reacted for 60 min. Finally, the OD value at a wavelength of 492 nm was measured by a microplate reader (Molecular Devices, SpectraMax190) and the detection results were analysed. 10% Triton X-100 plus target cells were used as the complete lysis control; target cells only were used as the blank negative control; and PBMCs plus target cells were used as the background negative control.

The cell killing rate was calculated according to the following formula: killing rate (%) = (OD value of candidate antibody well - OD value of background well)/(OD value of complete lysis well - OD value of blank well) × 100%.

The results (FIGs. 5a and 5b) showed that the candidate antibody NA7-S or NA8-S at an equal molar concentration had a cell killing activity comparable to that of the control antibody IMAB362. As could be seen from FIG. 5a, the $EC_{50}$ values of the ADCC cell killing effects of NA7-S and IMAB362 were 0.1097 nM and 0.0862 nM, respectively; and as could be seen from FIG. 5b, the $EC_{50}$ values of the ADCC cell killing effects of NA8-S and IMAB362 were 0.0997 nM and 0.0636 nM, respectively.

The ADCC killing effect of the candidate antibody NA7-S or NA8-S on human CLDN18.2-KATOIII tumour cells was determined by using the same method. The results are as shown in FIGs. 5c and 5d. The candidate antibodies at equal molar concentrations exhibited ADCC cell killing effects stronger than that of the control antibody IMAB362, wherein in the comparison of the candidate antibody NA7-S and IMAB362, the NA7-S-mediated ADCC killing efficiency was nearly 50%, and the ADCC killing efficiency of the control antibody IMAB362 was only about 23%; and in the comparison of the candidate antibody NA8-S and IMAB362, the NA8-S-mediated ADCC killing efficiency was nearly 44%, and the ADCC killing efficiency of the control antibody IMAB362 was only about 24%.

**Example 9**

***In vivo* tumour suppression experiment (using human CLDN18.2-HEK293T as a tumourigenic cell line)**

6-8 weeks old, female SCID mice (body weight 24-26 g) were used in the experiment. The experimental mice were housed in independent ventilated boxes with constant temperature and humidity, the rearing chambers has a temperature of 21 °C-24 °C and a humidity of 30%-53%.

A total of $1 \times 10^7$ human CLDN18.2-HEK293T cells were subcutaneously injected into the right axilla. When the volume of the subcutaneous tumour reached 80-100 mm$^3$, the mouse samples with large differences in tumour volume were excluded, and the remaining samples were randomly grouped according to the tumour volume (8 mice per group) to obtain a PBS treatment group, an IMAB362 antibody treatment group, and a candidate antibody NA7-S or NA8-S treatment group, respectively. Antibody treatment began 8 days after cell inoculation (the candidate antibody NA7-S or NA8-S was given in a molar dose and a mass dose equal to that of the IMAB362 antibody based on the molecular weight, wherein the dose of the candidate antibody was 10 mg/kg, and the doses of the IMAB362 antibody were 20 mg/kg and 10 mg/kg). Administration was performed twice a week, in two ways, i.e., intravenous injection and intraperitoneal injection, alternately. The tumour length (mm) and width (mm) were observed and recorded, and the tumour growth volume (V) was calculated. The calculation formular was: V = (length $\times$ width$^2$)/2.

The results of tumour suppression by antibodies are as shown in FIGs. 6a and 6b, from which it could be seen that: at the same mass dose, the candidate antibody NA7-S or NA8-S was significantly better than the control antibody IMAB362 in inhibiting tumour growth.

**Example 10**

**Determination of binding epitopes of candidate antibodies on human CLDN18.2**

Both the candidate antibodies and the control antibody IMAB362 specifically bind to CLDN18.2 but do not to CLDN18.1, And the extramembranous regions of CLDN18.2 and CLDN18.1 only differ by eight amino acids in the ECD1 region. Therefore, it is speculated that the antigen-binding epitopes of the candidate antibodies NA7-S and NA8-S and the control antibody IMAB362 are within the ECD1 region. In order to confirm whether the antigen-binding positions of the candidate antibodies and IMAB362 are the same, this example adopts the method of competitive binding. The specific method is as follows.

CLDN18.2-HEK293T cells passaged 2-4 times and well-grown were used in the experiment. The cells were centrifuged at 4 °C, 300 g to remove the supernatant, then resuspended in an FACS buffer and counted, and then the cell density was adjusted to $2 \times 10^6$ cells/mL. The cells were added to a new 96-well round-bottom plate at 100 μL per well and centrifuged at 4°C, 300 g to remove the supernatant.

Meanwhile, an FACS buffer (1X PBS + 2% FBS) was formulated, and the competing antibodies NA7-S, NA8-S and the control antibody IMAB362 were subjected to gradient dilution by using the FACS buffer. Moreover, a biotin-labelled NA7-S (or IMAB362) protein was diluted to 13.4 nM by using the FACS buffer. 100 μL of gradient diluent and 100 μL of biotin-labelled antibody diluent were added to a 96-well plate, respectively, the resulting mixture was mixed uniformly by gentle pipetting with a multichannel pipette, and the 96-well plate was incubated at 4 °C for 1 h. The antibody and cell mixed solution obtained after incubation was centrifuged at 4 °C, 300 g, and the supernatant was removed. Subsequently, 200 μL of FACS buffer was added to each corresponding well, and the cells were resuspended and centrifuged at 4 °C, 300 g to remove the supernatant. This step was repeated twice. The PE-labelled streptavidin (eBioscience, 12-4317-87) was diluted at 1 : 200 by using an FACS buffer, and added to the corresponding cells at 200 μL/well with a multichannel pipette. The cells were gently pipetted, resuspended and then incubated at 4 °C for 30 min in the dark. After the incubation was complete, the cells were centrifuged at 4 °C, 300 g to remove the supernatant, and resuspended by adding the FACS buffer. This step was repeated twice. Finally, the cells were detected by a flow cytometer (Beckman, CytoFLEX AOO-1-1102).

The results are as shown in FIG. 7a. With the increase in the concentration of NA7-S, NA8-S or the control antibody IMAB362, the binding of IMAB362-biotin on CLDN18.2-HEK293T cells decreased. Similarly, the results in FIG. 7b showed that with the increase in the concentration of NA7-S, NA8-S or the control antibody IMAB362, the binding of NA7-S-biotin on CLDN18.2-HEK293T cells decreased, which illustrated that NA7-S, NA8-S and IMAB362 binded to similar or overlapped epitopes on CLDN18.2.

In order to further understand key amino acid sites to which NA7-S, NA8-S and IMAB362 binded on CLDN18.2, on the basis that CLDN18.2 and CLDN18.1 were different by eight amino acids in ECD1, eight mutant HEK293T cell lines were constructed in this example by mutating eight differential amino acids on CLDN18.2 into amino acids corresponding to CLDN18.1, and then the difference of the binding abilities of the antibodies to be tested on the wild-type lines and the mutant lines was determined so as to judge whether the amino acids were key amino acids for antibody binding. The specific method is as follows.

Firstly, the expression of CLDN18.2 in the wild-type and mutant huCLDN18.2-HEK293T cell lines was determined by using the method as described in section 1.3.2. The cells were fixed and permeabilized according to the method in the instruction of a cell permeabilization kit (eBioscience, 88-8824-00), and then the expression was determined according to the binding ability of the anti-CLDN18 antibody [34H14L15] to the C-terminal intracellular segment of CLDN18. The results are as shown in FIG. 7c. Each mutant cell line and the wild-type line had relatively high expression of CLDN18.2.

Based on the constructed cell lines, referring to the method in section 1.3.1, the binding intensities of candidate antibodies NA7-S or NA8-S, and IMAB362 on CLDN18.2 wild-type and mutant huCLDN18.2-HEK293T cell lines were determined. In addition, the binding intensities of the candidate antibodies were divided by the expression level of each cell line, and normalized by taking the binding of the candidate antibodies on the wild-type lines as 100%, and the normalized results were used as the final relative binding intensity. As shown in FIGs. 7d-7g, the epitopes of NA7-S and NA8-S were relatively close. One site (E56) on CLDN18.2 ECD1 was very critical for the binding of NA7-S and NA8-S on huCLDN18.2-HEK293T, and A42, E56 and G65 were three key amino acids for IMAB362 to bind to CLDN18.2.

## Example 11

### Candidate antibody-mediated Fab-ZAP cell killing effect

CLDN18.2 is highly expressed on various tumours, but is specifically expressed in the tight junction structures of gastric epithelial cells in normal tissues. Therefore, CLDN18.2 may become an ideal ADC drug target. In this experiment, the endocytic activities of antibodies were detected by the cytotoxicity of antibody-mediated Fab-ZAP endocytosis, wherein Fab-ZAP (Atsbio, IT-51-100) is an Fab fragment ligated to an anti-Fc region of saporin, and saporin is a ribosome inhibitor that can inhibit protein synthesis and cause cell death. The Fab-ZAP was incubated with the CLDN18.2 antibody so that the CLDN18.2 antibody carried the Fab-ZAP. When the CLDN18.2 antibody was endocytosed, the Fab-ZAP entered cells along with the antibody, thereby killing cells, and then the cell activity was detected by MTS (Promega, G3580) so as to detect and compare the endocytic activities of the candidate antibody and the control antibody. The specific method is as follows.

CLDN18.2-HEK293T cells in a log growth phase were taken and digested with trypsin (0.25% (w/v) Trypsin 0.53 mM EDTA) until there was no adhesion between the cells and gaps were formed, and the digestion was terminated with a complete culture medium. The cells were mixed fully and uniformly, then counted and determined for the viability. The cell density was adjusted to $4 \times 10^4$ cells/mL, and the cells were added to a cell culture plate at 50 $\mu$L per well and incubated in a 37 °C cell incubator for 16 h. In addition, the Fab-ZAP was diluted to 2 $\mu$g/mL with a DMEM complete culture medium, and the candidate antibodies NA7-S and NA8-S and the control antibody were further subjected to gradient dilution by using the diluted Fab-ZAP as a diluent. 50 $\mu$L of the resulting diluent was taken with a multichannel pipette, added to a cell culture plate and mixed uniformly with CLDN18.2-HEK293T by gentle pipetting. The cell culture plate was incubated in a 37°C cell incubator for another 72 h. Next, 20 $\mu$L of MTS was added to each well with a multichannel pipette, and the resulting mixture was mixed uniformly by gentle pipetting and then incubated at 37 °C for 2-4 h. Finally, the cell plate was centrifuged at 1000 rpm for 5 min in a desk centrifuge, and then the data were read in a microplate reader (Molecular Devices, SpectraMax190), with a detection wavelength of 492 nm.

The results are as shown in FIGs. 8a-8b. The candidate antibodies NA7-S and NA8-S could kill target cells CLDN18.2-HEK293T more effectively by means of Fab-ZAP relative to the control antibody IMAB362, wherein in the comparison of NA7-S and IMAB362, the $IC_{50}$ values were 0.0487 nM and 0.1284 nM, respectively; and in the comparison of NA8-S and IMAB362, the $IC_{50}$ values were 0.0492 nM and 0.1362 nM, respectively.

This fully demonstrates that the candidate antibodies can enter cells via CLDN18.2, they are superior to the control antibody IMAB362, and have greater potential to be used in the development of ADC antibody-conjugated drugs.

## Example 12

### Humanization of candidate antibodies NA4-S, NA7-S and NA8-S

Compared to murine-derived antibodies, alpaca-derived nanobodies had higher homology with human-derived antibodies, but their structures are special. Thus, during the humanization design of NA4-S, NA7-S and NA8-S, CDR sequences and framework region sequences were defined by using the AbM numbering system, and a human germline gene closest to a framework region sequence of each nanobody was selected. Moreover, when back mutation was performed, the structures of the antibodies remained unchanged, and a series of humanized antibodies were finally designed, wherein NA4-S-H7, NA7-S-H2 and NA8-S-H9 were the optimal humanized molecules corresponding to the parent antibodies (NA4-S, NA7-S and NA8-S), respectively.

The amino acid sequences (SEQ ID NOs) of VHHs and CDR1, CDR2 and CDR3 of the humanized antibodies NA4-S-H7, NA7-S-H2 and NA8-S-H9 are shown in Table 3.

Table 3

| Antibody name | CDR1 | CDR2 | CDR3 | VHH |
|---|---|---|---|---|
| NA4-S-H7 | 30 | 31 | 32 | 13 |
| NA7-S-H2 | 37 | 38 | 39 | 14 |
| NA8-S-H9 | 45 | 46 | 47 | 15 |

In order to investigate whether the affinities of NA4-S, NA7-S and NA8-S did not decrease after humanization, their binding abilities on human CLDN18.2-HEK293T cells were compared by using the method as described in section 1.3.1.

As shown in FIG. 9a, after NA4-S was subjected to humanization, NA4-S-H7 and the parent antibody had similar affinities; the $EC_{50}$ values of the molecule were 0.8822 $\mu$g/mL and 0.8771 $\mu$g/mL before and after humanization, respectively; and the binding intensities (mean fluorescence intensities, MFIs) of the antibodies at the highest concentration were 129181, 127633 and 82136, respectively, much better than that of the IMAB362 control antibody.

As shown in FIG. 10a, after NA7-S was subjected to humanization, NA7-S-H2 and the parent antibody had similar affinities; the $EC_{50}$ values of the molecule were 0.3790 $\mu$g/mL and 0.4824 $\mu$g/mL before and after humanization, respectively; and the binding intensities (MFIs) of the antibodies at the highest concentration were 163000, 184000 and 106000, respectively, much better than that of the IMAB362 control antibody.

As shown in FIG. 11a, only the affinity of NA8-S-H9 obtained after humanization of NA8-S was inferior to that of the parent antibody; the $EC_{50}$ values of the molecule were 0.09844 $\mu$g/mL and 0.1492 $\mu$g/mL before and after humanization, respectively; the binding intensity (MFI) of the antibody at the highest concentration for NA8-S-H9 was similar to that of the IMAB362 control antibody; and the MFIs of the three antibodies were 101714, 65195 and 76447, respectively.

## Example 13

### Binding specificity of candidate antibodies NA4-S, NA7-S and NA8-S at high concentrations before and after humanization

CLDN18.2 and CLDN18.1 are different by only eight amino acids in the candidate antibody binding region ECD1, and CLDN18.1 is expressed in lung epithelial cells. Therefore, if antibody drugs non-specifically binds to CLDN18.1, severe lung damage or toxicity would be caused and thus the clinical use is limited. Therefore, antibodies (including a high concentration of 100 $\mu$g/mL) at different concentrations were used to be incubated with human CLDN18.1-HEK293 *in vitro* in this example. The method was as described in section 1.3.1, wherein antibodies at different concentrations were incubated with target cells human CLDN18.1-HEK293 at 4 °C for 1 h. Next, the resulting cultures were rinsed three times with the above-mentioned FACS buffer. 0.5 $\mu$g (0.5 mg/ml) of PE-labelled goat anti-human IgG Fc antibody (Abeam, ab98596) was added, and the resulting mixture was incubated at 4 °C for 1 h, and then rinsed three times with the FACS buffer. The cells were resuspended by adding 200 $\mu$L of FACS buffer to the cells, and finally detected by a flow cytometer (Beckman, CytoFLEX AOO-1-1102), and the binding intensity and the positive rate of cell binding were recorded.

As shown in FIG. 9b, in terms of the binding intensity, the binding intensities of NA4-S, NA4-S-H7 and the control antibody IMAB362 on human CLDN18.1-HEK293 cells did not increase with the increase in the concentration, and were almost identical to that of the irrelevant control. In terms of the positive rate of cell binding in FIG. 9c, the positive rates of NA4-S and NA4-S-H7, even at a high concentration of 100 $\mu$g/mL, were very close to those of the control antibody IMAB362 and the irrelevant control hIgG1, and their positive rates were 0.434%, 0.247 %, 0.095% and 0.049%, respectively.

With regard to comparison of the specificity of NA7-S before and after humanization, as shown in FIG. 10b, in terms of the binding intensity, the binding intensities of NA7-S, NA7-S-H2 and the control antibody IMAB362 on human CLDN18.1-HEK293 cells were very weak, and were almost identical to that of the irrelevant control. In terms of the positive rate of cell binding in FIG. 10c, the positive rates of NA7-S, NA7-S-H2, the control antibody IMAB362, and the irrelevant control hIgG 1 at a high concentration of 100 $\mu$g/mL were 10.801%, 5.499%, 1.433% and 0.851%, respectively, wherein the positive rate or non-specificity of NA7-S-H2 was even better than that of the parent antibody NA7-S.

With regard to comparison of the specificity of NA8-S before and after humanization, as shown in FIG. 11b, in

32

terms of the binding intensity, the binding intensities of NA8-S, NA8-S-H9 and the control antibody IMAB362 on human CLDN18.1-HEK293 were very weak, but the binding intensities of NA8-S and NA8-S-H9 were significantly higher than that of the control antibody IMAB362. Moreover, in terms of the positive rate of cell binding in FIG. 11c, the positive rates of NA8-S, NA8-S-H9, the control antibody IMAB362 and the irrelevant control hIgG1 at a high concentration of 100 μg/mL were 14.438%, 5.459%, 0.070% and 0.067%, respectively, wherein the positive rate or non-specificity of NA8-S-H9 was even better than that of the parent antibody NA8-S.

**Example 14**

**Affinity maturation of humanized molecules**

In this example, affinity maturation was performed on humanized molecules NA4-S-H7, NA7-S-H2 and NA8-S-H9 to improve the affinity and the biological activity. The affinity maturation was based on the M13 phage display technology; CDR region mutations were introduced by using codon-based primers (in the primer synthesis process, a single codon was composed of NNK); and a total of four phage display libraries were constructed. Library 1 was CDR1 + CDR2 + CDR3 single-point combined mutation; library 2 was CDR1 + CDR2 double-point combined mutation; library 3 was CDR1 + CDR3 double-point combined mutation; and library 4 was CDR2 + CDR3 double-point combined mutation. A single CDR region mutant fragment was obtained by means of PCR with a humanized molecule as a template; then a full-length VHH fragment was obtained by means of Overlapping PCR; the point mutant antibody was ligated into a phage display vector by means of double digestion (HindIII and NotI) and double-sticky- end ligation; and finally the VHH sequence with a mutation site was transformed into *E. coli* SS320 by means of electrotransformation.

After the four constructed libraries were packaged into phages, solid-phase panning was performed. Antigens coated on an immunotube binded to the phages displaying the full-length VHH fragment, and antibodies with high affinity were subjected to pressure panning by reducing the mass of the antigens coated. After panning and elution, *E. coli* SS320 was infected, and the next cycle of panning was performed; and after 2-3 cycles of panning, monoclonal antibodies were picked for FACS detection. According to the affinity and sequence analysis, seven affinity-matured candidate molecules were selected, including NA7S-H2-418, NA7S-H2-420, NA7S-H2-20, NA7S-H2-30, NA4S-H7-10, NA4S-H7-3 and NA8S-H9-57. The above-mentioned affinity-matured molecules were selected for sample preparation, and the preparation method was detailed in Example 4.

The amino acid sequences (SEQ ID NOs) of VHHs and CDR1, CDR2 and CDR3 of the antibodies obtained by means of affinity maturation are shown in Table 4.

Table 4

| Antibody name | CDR1 | CDR2 | CDR3 | VHH |
|---|---|---|---|---|
| NA4S-H7-10 | 30 | 31 | 33 | 27 |
| NA4S-H7-3 | 34 | 35 | 36 | 28 |
| NA7S-H2-20 | 37 | 38 | 42 | 25 |
| NA7S-H2-30 | 37 | 43 | 44 | 26 |
| NA7S-H2-418 | 40 | 38 | 39 | 23 |
| NA7S-H2-420 | 41 | 38 | 39 | 24 |
| NA8S-H9-57 | 48 | 46 | 47 | 29 |

**Example 15**

**Specific binding assay of affinity-matured molecules**

The binding of affinity-matured molecules NA7S-H2-418, NA7S-H2-420, NA7S-H2-20, NA7S-H2-30, NA4S-H7-10, NA4S-H7-3 and NA8S-H9-57 on human CLDN18.1-HEK293 cells was detected by using the same detection method as that in Example 5.

With regard to comparison of the specificity of NA7-S-H2 before and after affinity maturation, the results are as shown in FIG. 12, wherein the affinity-matured molecules NA7S-H2-418, NA7S-H2-420, NA7S-H2-20 and NA7S-H2-30 did not bind to human CLDN18.1-HEK293 cells and had non-specificity better than that of NA7-S-H2. With regard to comparison of the specificity of NA4-S-H7 and NA8-S-H9 before and after affinity maturation, the results showed that the specificity was comparable to that of the parent molecules, so the results were not shown here.

**Example 16**

**Comparison of binding abilities of affinity-matured molecules on human CLDN18.2-HEK293T cell lines and human CLDN18.2-KATOIII tumour cell lines**

The binding abilities of affinity-matured molecules NA7S-H2-418, NA7S-H2-420, NA7S-H2-20, NA7S-H2-30, NA4S-H7-10, NA4S-H7-3 and NA8S-H9-57 on human CLDN18.2-HEK293T and human CLDN18.2 KATOIII cells were detected by using the same detection method as that in Example 6.

With regard to comparison of the binding abilities of NA7-S-H2 before and after affinity maturation, the results are as shown in FIG. 13a, wherein the affinity-matured molecules NA7S-H2-418, NA7S-H2-420, NA7S-H2-20, and NA7S-H2-30 all exhibited cell binding activities comparable to or even better than that of the control antibody on human CLDN18.2-HEK293T cells. With regard to comparison of the binding abilities of NA4-S-H7 before and after affinity maturation, the results are as shown in FIG. 13b, wherein the affinity-matured molecules NA4S-H7-10 and NA4S-H7-3 both exhibited cell binding activities comparable to or even better than that of the control antibody on human CLDN18.2-HEK293T cells. With regard to comparison of the binding abilities of NA8-S-H9 before and after affinity maturation, the results are as shown in FIG. 13c, wherein the affinity-matured molecule NA8S-H9-57 exhibited better cell binding activity on huCLDN18.2-HEK293T cells.

With regard to comparison of the binding abilities of NA7-S-H2 on human CLDN18.2-KATOIII cells before and after affinity maturation, the results are as shown in FIG. 14, wherein the binding ability of the affinity-matured molecule was significantly better than that of the parent molecule.

**Example 17**

**Complement-dependent cytotoxicity (CDC) of affinity-matured molecules**

The CDC cell killing effects of affinity-matured molecules NA7S-H2-418, NA7S-H2-420, NA7S-H2-20, NA7S-H2-30, NA4S-H7-10, NA4S-H7-3 and NA8S-H9-57 on human CLDN18.2-HEK293T cells were determined by using the same method as the MTS method in Example 7.

The CDC detection results are as follows. As shown in FIGs. 15a-15d, the CDC killing effects of all affinity-matured molecules on human CLDN18.2-HEK293T cells were comparable to or better than that of the parent molecule NA4-S-H7, NA7-S-H2 or NA8-S-H9.

**Example 18**

**ADCC activities of affinity-matured molecules**

After the Fc termini of affinity-matured molecules binds to CD16a (V158) and the VHH termini binds to target cells, the expression of NF-AT protein inside Jurkat cells is activated; the binding of NF-AT to its reaction element trigger the expression of its downstream luciferase; and stimulation with antibodies at different concentration gradients will result in a fluorescence reading curve with antibody concentration dependence, so that the ADCC activities of the antibodies can be evaluated.

huCLDN18.2-HEK293T cells or human CLDN18.2-KATOIII cells at $1 \times 10^4$ cells/well and Jurkat-NFAT-Luciferase-CD16a (V158) cells (the stably transfected cell lines obtained by stably transfecting CD16a (V158) sequences (UniProtKB-P08637) and pGL4.30 plasmids (promega, #E8481) containing NF-AT-re nucleic acid sequences into Jurkat cells (ATCC®TIB-152)) at $1 \times 10^5$ cells/well, with a total volume of 50 $\mu$L, were added to each well in a 96-well cell culture plate. 50 $\mu$L of to-be-tested sample obtained after gradient dilution was added, and the mixture was incubated in a 37°C incubator for 6 h. 50 $\mu$L of Bright-Lite (vazyme, Cat. No. DD1204-03) was added to each well; the mixture was incubated in the dark for 10 min; and the fluorescence signal was detected.

The ADCC effect detection results on huCLDN18.2-HEK293T cells are as follows. As shown in FIG. 16a and FIG. 16b, the ADCC killing effects of affinity-matured molecules NA7S-H2-418, NA7S-H2-420 and NA7S-H2-20 were better than that of the parent molecule NA7-S-H2; and the ADCC killing effect of the affinity-matured molecule NA7S-H2-30 was comparable to that of the parent molecule NA7-S-H2.

The detection results on human CLDN18.2-KATOIII cells are as follows. As shown in FIG. 17a, the ADCC killing effects of affinity-matured molecules NA4S-H7-10 and NA4S-H7-3 were better than that of the parent molecule NA4-S-H7; and as shown in FIG. 17b, the ADCC killing effect of the affinity-matured molecule NA8S-H9-57 was better than that of the parent molecule NA8-S-H9.

**Overview of the Sequence Listing**

| SEQ ID NO. | Sequence name | Sequence description | Sequence information |
|---|---|---|---|
| 1 | NA4-S-CDR1 | CDR1 | GSIFSINV |
| 2 | NA4-S-CDR2 | CDR2 | ISTGGTT |
| 3 | NA4-S-CDR3 | CDR3 | NADLATATSWPVRYLEV |
| 4 | NA7-S-CDR1 | CDR1 | GSFFRIVA |
| 5 | NA7-S-CDR2 | CDR2 | ITRGGST |
| 6 | NA7-S-CDR3 | CDR3 | NVRVEVPFMQPNDY |
| 7 | NA8-S-CDR1 | CDR1 | GRTFSNYA |
| 8 | NA8-S-CDR2 | CDR2 | ITWSGGST |
| 9 | NA8-S-CDR3 | CDR3 | AGGFFATSYSGNYGY |
| 10 | NA4-S | VHH sequence | EVQVVESGGGLVQPGGSLRLSCAASGSIFSINVMGWYRQAPGKQRELVAGISTGGTTRYADSAKGRFTISRDNAKNTVYLQMNSLKPEDTAVYYCNADLATATSWPVRYLEVWGQGTLVTVSA |
| 11 | NA7-S | VHH sequence | EVQVQESGGGLVQPGGSLRLSCAASGSFFRIVAMGWYRQAPGSQRELVATITRGGSTYYADSMKGRSTISRDNAKNTVYLQMNSLKPEDTAVYYCNVRVEVPFMQPNDYWGHGTQVTVSS |
| 12 | NA8-S | VHH sequence | EVQVQESGGGLVQAGGSLRLSCAASGRTFSNYAVGWFRQAPGKEREFVAAITWSGGSTNYADSVKGRFTISRDNAKNTAWLQMNSLKPEDTAVYYCAGGFFATSYSGNYGYWGQGTQVTVSS |
| 13 | NA4-S-H7 | Humanized VHH sequence | EVQLVESGGGLVQPGGSLRLSCAASGSIFSINVMGWYRQAPGKQRELVAGISTGGTTRYADSVKGRFTISRDNAKNTLYLQMNSLRAEDTAVYYCNADLATATSWPVRYLEVWGQGTLVTVSS |
| 14 | NA7-S-H2 | Humanized VHH sequence | EVQLVESGGGLVQPGGSLRLSCAASGSFFRIVAMGWYRQAPGKGRELVATITRGGSTYYADSMKGRSTISRDNAKNTVYLQMNSLKPEDTAVYYCNVRVEVPFMQPNDYWGQGTLVTVSS |
| 15 | NA8-S-H9 | Humanized VHH sequence | EVQLVESGGGLVQPGGSLRLSCAASGRTFSNYAVGWFRQAPGKGLEFVAAITWSGGSTNYADSVKGRFTISRDNAKNTLYLQMNSLRAEDTAVYYCAGGFFATSYSGNYGYWGQGTLVTVSS |
| 16 | Human CLDN 18.2 | Full-length sequence of human CLDN18.2 | MAVTACQGLGFVVSLIGIAGIIAATCMDQWSTQDLYNNPVTAVFNYQGLWRSCVRESSGFTECRGYFTLLGLPAMLQAVRALMIVGIVLGAIGLLVSIFALKCIRIGSMEDSAKANMTLTSGIMFIVSGLCAIAGVSVFANMLVTNFWMSTANMYTGMGGMVQTVQTRYTFGAALFVGWVAGGLTLIGGVMMCIACRGLAPEETNYKAVSYHASGHSVAYKPGGFKASTGFGSNTKNKKIYDGGARTEDEVQSYPSKHDYV |

(continued)

| SEQ ID NO. | Sequence name | Sequence description | Sequence information |
|---|---|---|---|
| 17 | Human CLDN18.1 | Full-length sequence of human CLDN 18.1 | MSTTTCQVVAFLLSILGLAGCIAATGMDMWSTQDLYDNPVTS VFQYEGLWRSCVRQSSGFTECRPYFTILGLPAMLQAVRALMI VGIVLGAIGLLVSIFALKCIRIGSMEDSAKANMTLTSGIMFIVS GLCAIAGVSVFANMLVTNFWMSTANMYTGMGGMVQTVQTR YTFGAALFVGWVAGGLTLIGGVMMCIACRGLAPEETNYKAV SYHASGHSVAYKPGGFKASTGFGSNTKNKKIYDGGARTEDEV QSYPSKHDYV |
| 18 | Murine CLDN18.2 | Full-length sequence of murine CLDN18.2 | MSVTACQGLGFVVSLIGFAGIIAATCMDQWSTQDLYNNPVTA VFNYQGLWRSCVRESSGFTECRGYFTLLGLPAMLQAVRALMI VGIVLGVIGILVSIFALKCIRIGSMDDSAKAKMTLTSGILFIISGI CAIIGVSVFANMLVTNFWMSTANMYSGMGGMGGMVQTVQT RYTFGAALFVGWVAGGLTLIGGVMMCIACRGLTPDDSNFKA VSYHASGQNVAYRPGGFKASTGFGSNTRNKKIYDGGARTED DEQSHPTKYDYV |
| 19 | Murine CLDN 18.1 | Full-length sequence of murine CLDN18.1 | MATTTCQVVGLLLSLLGLAGCIAATGMDMWSTQDLYDNPVT AVFQYEGLWRSCVQQSSGFTECRPYFTILGLPAMLQAVRALM IVGIVLGVIGILVSIFALKCIRIGSMDDSAKAKMTLTSGILFIISGI CAIIGVSVFANMLVTNFWMSTANMYSGMGGMGGMVQTVQT RYTFGAALFVGWVAGGLTLIGGVMMCIACRGLTPDDSNFKA VSYHASGQNVAYRPGGFKASTGFGSNTRNKKIYDGGARTED DEQSHPTKYDYV |
| 20 | Human CLDN18.2 ECD1 | Extracellul ar domain 1 of human CLDN18.2 | METDTLLLWVLLLWVPGSTGDAAQPARRARRTKLGTELGST PVWWNSADGRMDQWSTQDLYNNPVTAVFNYQGLWRSCVR ESSGFTECRGYFTLLGLPAMLQAVRAAIQHSGGRSRRARTKT HLRRGSE |
| 21 | Human IgG1 Fc | Human IgG1 Fc sequence for fusion to C terminus of candidate antibody | EPKSCDKTHTCPPCPAPELLGGPSVFLFPPKPKDTLMISRTPEV TCVVVDVSHEDPEVKFNWYVDGVEVHNAKTKPREEQYNSTY RVVSVLTVLHQDWLNGKEYKCKVSNKALPAPIEKTISKAKGQ PREPQVYTLPPSRDELTKNQVSLTCLVKGFYPSDIAVEWESNG QPENNYKTTPPVLDSDGSFFLYSKLTVDKSRWQQGNVFSCSV MHEALHNHYTQKSLSLSPGK |
| 22 | C-terminal intracellular segment of CLDN18 | Intracellula r antibody recognition segment of CLDN18 | GFKASTGFGSNTKN |
| 23 | NA7S-H2-418 | Affinity- matured VHH sequence | EVQLVESGGGLVQPGGSLRLSCAASGSFFRIVAMEWYRQAPG KGRELVATITRGGSTYYADSMKGRSTISRDNAKNTVYLQMNS LKPEDTAVYYCNVRVEVPFMQPNDYWGQGTLVTVSS |

(continued)

| SEQ ID NO. | Sequence name | Sequence description | Sequence information |
|---|---|---|---|
| 24 | NA7S-H2-420 | Affinity-matured VHH sequence | EVQLVESGGGLVQPGGSLRLSCAASGSFFRIVMYGWYRQAPG KGRELVATITRGGSTYYADSMKGRSTISRDNAKNTVYLQMNS LKPEDTAVYYCNVRVEVPFMQPNDYWGQGTLVTVSS |
| 25 | NA7S-H2-20 | Affinity-matured VHH sequence | EVQLVESGGGLVQPGGSLRLSCAASGSFFRIVAMGWYRQAPG KGRELVATITRGGSTYYADSMKGRSTISRDNAKNTVYLQMNS LKPEDTAVYYCNVRVEVPFMAPNDYWGQGTLVTVSS |
| 26 | NA7S-H2-30 | Affinity-matured VHH sequence | EVQLVESGGGLVQPGGSLRLSCAASGSFFRIVAMGWYRQAPG KGRELVATITRGGSSYYADSMKGRSTISRDNAKNTVYLQMNS LKPEDTAVYYCNVRVEVPFFQPNDYWGQGTLVTVSS |
| 27 | NA4S-H7-10 | Affinity-matured VHH sequence | EVQLVESGGGLVQPGGSLRLSCAASGSIFSINVMGWYRQAPG KQRELVAGISTGGTTRYADSVKGRFTISRDNAKNTLYLQMNS LRAEDTAVYYCNADLATSYSWPVRYLEVWGQGTLVTVSS |
| 28 | NA4S-H7-3 | Affinity-matured VHH sequence | EVQLVESGGGLVQPGGSLRLSCAASGSIFSINVMSWYRQAPG KQRELVAGISAGGTTRYADSVKGRFTISRDNAKNTLYLQMNS LRAEDTAVYYCNADLAEATSWPVRYLEVWGQGTLVTVSS |
| 29 | NA8S-H9-57 | Affinity-matured VHH sequence | EVQLVESGGGLVQPGGSLRLSCAASGLIFSNYAVGWFRQAPG KGLEFVAAITWSGGSTNYADSVKGRFTISRDNAKNTLYLQMN SLRAEDTAVYYCAGGFFATSYSGNYGYWGQGTLVTVSS |
| 30 | NA4-S/NA4-S-H7/NA4S-H7-10 CDR1 | CDR1 | GSIFSINVMG |
| 31 | NA4-S/NA4-S-H7/NA4S-H7-10 CDR2 | CDR2 | GISTGGTTR |
| 32 | NA4-S/NA4-S-H7 CDR3 | CDR3 | DLATATSWPVRYLEV |
| 33 | NA4S-H7-10-CDR3 | CDR3 | DLATSYSWPVRYLEV |
| 34 | NA4S-H7-3-CDR1 | CDR1 | GSIFSINVMS |
| 35 | NA4S-H7-3-CDR2 | CDR2 | GISAGGTTR |
| 36 | NA4S-H7-3-CDR3 | CDR3 | DLAEATSWPVRYLEV |
| 37 | NA7-S/NA7-S-H2/NA7S-H2-20/NA7S-H2-30 CDR1 | CDR1 | GSFFRIVAMG |
| 38 | NA7-S/NA7-S-H2/NA7S-H2-20/NA7S-H2-418/NA7S-H2-420 CDR2 | CDR2 | TITRGGSTY |

(continued)

| SEQ ID NO. | Sequence name | Sequence description | Sequence information |
|---|---|---|---|
| 39 | NA7-S/NA7-S-H2/ NA7S-H2-418/NA7S-H2-420 CDR3 | CDR3 | RVEVPFMQPNDY |
| 40 | NA7S-H2-418-CDR1 | CDR1 | GSFFRIVAME |
| 41 | NA7S-H2-420-CDR1 | CDR1 | GSFFRIVMYG |
| 42 | NA7S-H2-20-CDR3 | CDR3 | RVEVPFMAPNDY |
| 43 | NA7S-H2-30-CDR2 | CDR2 | TITRGGSSY |
| 44 | NA7S-H2-30-CDR3 | CDR3 | RVEVPFFQPNDY |
| 45 | NA8-S/NA8-S-H9 CDR1 | CDR1 | GRTFSNYAVG |
| 46 | NA8-S/NA8-S-H9/NA8S-H9-57 CDR2 | CDR2 | AITWSGGSTN |
| 47 | NA8-S/NA8-S-H9/NA8S-H9-57 CDR3 | CDR3 | GFFATSYSGNYGY |
| 48 | NA8S-H9-57-CDR1 | CDR1 | GLIFSNYAVG |

Those skilled in the art will further appreciate that the present invention may be implemented in other specific forms without departing from its spirit or centre features. Since the foregoing description of the present disclosure only discloses its exemplary embodiments, it should be understood that other variations are considered as falling within the scope of the present invention. Accordingly, the present invention is not limited to the particular embodiments described in detail herein. Rather, reference should be made to the appended claims as indicative of the scope and content of the present invention.

## Claims

1. A CLDN18.2 binding molecule, comprising at least one immunoglobulin single variable domain, wherein the immunoglobulin single variable domain comprises CDR1, CDR2 and CDR3, and wherein

(1) an amino acid sequence of CDR1 differs from a sequence as shown in SEQ ID NO: 1, 4 or 7 by addition, deletion or substitution of no more than 2 amino acids; an amino acid sequence of CDR2 differs from a sequence as shown in SEQ ID NO: 2, 5 or 8 by addition, deletion or substitution of no more than 2 amino acids; and/or an amino acid sequence of CDR3 differs from a sequence as shown in SEQ ID NO: 3, 6 or 9 by addition, deletion or substitution of no more than 2 amino acids; or
(2) an amino acid sequence of CDR1 differs from a sequence as shown in SEQ ID NO: 30, 37 or 45 by addition, deletion or substitution of no more than 2 amino acids; an amino acid sequence of CDR2 differs from a sequence as shown in SEQ ID NO: 31, 38 or 46 by addition, deletion or substitution of no more than 2 amino acids; and/or an amino acid sequence of CDR3 differs from a sequence as shown in SEQ ID NO: 32, 39 or 47 by addition, deletion or substitution of no more than 2 amino acids.

2. The CLDN18.2 binding molecule of claim 1, wherein the CLDN18.2 binding molecule is an antibody or an antigen-binding fragment thereof against CLDN18.2.

3. The CLDN18.2 binding molecule of claim 1 or 2, wherein the immunoglobulin single variable domain is VHH, such as VHH from camelids (e.g., an alpaca).

4. The CLDN18.2 binding molecule of any one of claims 1-3, wherein the immunoglobulin single variable domain comprises:

   i) CDR1 having an amino acid sequence as shown in SEQ ID NO: 1, 4, 7, 30, 34, 37, 40, 41, 45 or 48;
   ii) CDR2 having an amino acid sequence as shown in SEQ ID NO: 2, 5, 8, 31, 35, 38, 43 or 46; and
   iii) CDR3 having an amino acid sequence as shown in SEQ ID NO: 3, 6, 9, 32, 33, 36, 39, 42, 44 or 47.

5. The CLDN18.2 binding molecule of any one of claims 1-4, wherein the immunoglobulin single variable domain comprises:

   (a) CDR1 having the amino acid sequence as shown in SEQ ID NO: 1, CDR2 having the amino acid sequence as shown in SEQ ID NO: 2 and CDR3 having the amino acid sequence as shown in SEQ ID NO: 3;
   (b) CDR1 having the amino acid sequence as shown in SEQ ID NO: 4, CDR2 having the amino acid sequence as shown in SEQ ID NO: 5 and CDR3 having the amino acid sequence as shown in SEQ ID NO: 6;
   (c) CDR1 having the amino acid sequence as shown in SEQ ID NO: 7, CDR2 having the amino acid sequence as shown in SEQ ID NO: 8 and CDR3 having the amino acid sequence as shown in SEQ ID NO: 9;
   (d) CDR1 having the amino acid sequence as shown in SEQ ID NO: 30, CDR2 having the amino acid sequence as shown in SEQ ID NO: 31 and CDR3 having the amino acid sequence as shown in SEQ ID NO: 32;
   (e) CDR1 having the amino acid sequence as shown in SEQ ID NO: 30, CDR2 having the amino acid sequence as shown in SEQ ID NO: 31 and CDR3 having the amino acid sequence as shown in SEQ ID NO: 33;
   (f) CDR1 having the amino acid sequence as shown in SEQ ID NO: 34, CDR2 having the amino acid sequence as shown in SEQ ID NO: 35 and CDR3 having the amino acid sequence as shown in SEQ ID NO: 36;
   (g) CDR1 having the amino acid sequence as shown in SEQ ID NO: 37, CDR2 having the amino acid sequence as shown in SEQ ID NO: 38 and CDR3 having the amino acid sequence as shown in SEQ ID NO: 39;
   (h) CDR1 having the amino acid sequence as shown in SEQ ID NO: 40, CDR2 having the amino acid sequence as shown in SEQ ID NO: 38 and CDR3 having the amino acid sequence as shown in SEQ ID NO: 39;
   (i) CDR1 having the amino acid sequence as shown in SEQ ID NO: 41, CDR2 having the amino acid sequence as shown in SEQ ID NO: 38 and CDR3 having the amino acid sequence as shown in SEQ ID NO: 39;
   (j) CDR1 having the amino acid sequence as shown in SEQ ID NO: 37, CDR2 having the amino acid sequence as shown in SEQ ID NO: 38 and CDR3 having the amino acid sequence as shown in SEQ ID NO: 42;
   (k) CDR1 having the amino acid sequence as shown in SEQ ID NO: 37, CDR2 having the amino acid sequence as shown in SEQ ID NO: 43 and CDR3 having the amino acid sequence as shown in SEQ ID NO: 44;
   (l) CDR1 having the amino acid sequence as shown in SEQ ID NO: 45, CDR2 having the amino acid sequence as shown in SEQ ID NO: 46 and CDR3 having the amino acid sequence as shown in SEQ ID NO: 47;
   (m) CDR1 having the amino acid sequence as shown in SEQ ID NO: 48, CDR2 having the amino acid sequence as shown in SEQ ID NO: 46 and CDR3 having the amino acid sequence as shown in SEQ ID NO: 47.

6. The CLDN18.2 binding molecule of any one of claims 1-5, wherein the immunoglobulin single variable domain comprises or consists of the following sequences:

   (A) an amino acid sequence as shown in SEQ ID NO: 10, 11, 12, 13, 14, 15, 23, 24, 25, 26, 27, 28 or 29;
   (B) an amino acid sequence that is at least 80%, 85%, 90%, 95%, 98% or 99% identical to the amino acid sequence as shown in SEQ ID NO: 10, 11, 12, 13, 14, 15, 23, 24, 25, 26, 27, 28 or 29; or
   (C) an amino acid sequence having addition, deletion and/or substitution of one or more (e.g., 1, 2, 3, 4, 5, 6, 7, 8, 9 or 10) amino acids in comparison with the amino acid sequence as shown in SEQ ID NO: 10, 11, 12, 13, 14, 15, 23, 24, 25, 26, 27, 28 or 29, wherein preferably, the addition, deletion and/or substitution do/does not occur in a CDR region.

7. The CLDN18.2 binding molecule of any one of claims 1-6, wherein the CLDN18.2 binding molecule is a single domain antibody, such as a heavy chain single domain antibody; a chimeric antibody; or a humanized antibody.

8. The CLDN18.2 binding molecule of any one of claims 1-7, wherein the immunoglobulin single variable domain is fused to an additional molecule, and the additional molecule is, for example, an Fc domain of an immunoglobulin (e.g., IgG) or a fluorescent protein.

9. The CLDN18.2 binding molecule of claim 8, wherein the CLDN18.2 binding molecule is a chimeric antibody comprising a VHH from camelid and an Fc domain of human IgG (e.g., human IgG1 or IgG4).

10. The CLDN18.2 binding molecule of claim 9, wherein the CLDN18.2 binding molecule is a chimeric antibody comprising a VHH from alpaca and an Fc domain of human IgG1.

11. The CLDN18.2 binding molecule of any one of claims 1-10, wherein the CLDN18.2 binding molecule binds to extracellular domain 1 (ECD1) of human CLDN18.2.

12. A CLDN18.2 binding molecule, competing with the CLDN18.2 binding molecule of any one of claims 1-11 for the same epitope.

13. The CLDN18.2 binding molecule of any one of claims 1-12, specifically binding to CLDN18.2, but not binding to CLDN18.1.

14. An isolated nucleic acid molecule, encoding the CLDN18.2 binding molecule of any one of claims 1-13.

15. An expression vector, comprising the isolated nucleic acid molecule of claim 14.

16. A host cell, comprising the expression vector of claim 15.

17. A pharmaceutical composition, comprising at least one CLDN18.2 binding molecule of any one of claims 1-13 and a pharmaceutically acceptable carrier.

18. A method for preparing the CLDN18.2 binding molecule of any one of claims 1-13, comprising the following steps:

   - expressing the CLDN18.2 binding molecule of any one of claims 1-13 in the host cell of claim 16; and
   - isolating the CLDN18.2 binding molecule from the host cell.

19. Use of the CLDN18.2 binding molecule of any one of claims 1-13 in the preparation of a drug for treating or preventing a condition associated with CLDN18.2.

20. A kit for treating or diagnosing a condition associated with CLDN18.2, comprising a container, wherein the container comprises the CLDN18.2 binding molecule of any one of claims 1-13.

*FIG. 1a*

*FIG. 1b*

*FIG. 2a*

*FIG. 2b*

*FIG. 2c*

*FIG. 2d*

*FIG. 2e*

*FIG. 2f*

*FIG. 3a*

*FIG. 3b*

*FIG. 3c*

FIG. 4a

FIG. 4b

FIG. 4c

FIG. 4d

FIG. 4e

*FIG. 5a*

*FIG. 5b*

FIG. 5c

FIG. 5d

*FIG. 6a*

*FIG. 6b*

FIG. 7a

FIG. 7b

*FIG. 7c*

*FIG. 7d*

FIG. 7e

FIG. 7f

*FIG. 7g*

*FIG. 8a*

*FIG. 8b*

*FIG. 9a*

*FIG. 9b*

*FIG. 9c*

*FIG. 10a*

*FIG. 10b*

*FIG. 10c*

EP 4 253 411 A1

FIG. 11a

FIG. 11b

FIG. 11c

*FIG. 12*

*FIG. 13a*

*FIG. 13b*

*FIG. 13c*

*FIG. 14*

*FIG. 15a*

*FIG. 15b*

*FIG. 15c*

*FIG. 15d*

*FIG. 16a*

FIG. 16b

FIG. 17a

*FIG. 17b*

## INTERNATIONAL SEARCH REPORT

| | International application No. |
|---|---|
| | **PCT/CN2021/132218** |

| A. CLASSIFICATION OF SUBJECT MATTER |
|---|
| C07K 16/18(2006.01)i;  C12N 15/13(2006.01)i;  G01N 33/574(2006.01)i;  A61K 39/395(2006.01)i;  A61P 35/00(2006.01)i |
| According to International Patent Classification (IPC) or to both national classification and IPC |

| B. FIELDS SEARCHED |
|---|
| Minimum documentation searched (classification system followed by classification symbols) |
| C07K; C12N; G01N; A61K; A61P |
| Documentation searched other than minimum documentation to the extent that such documents are included in the fields searched |
| |
| Electronic data base consulted during the international search (name of data base and, where practicable, search terms used) |
| CNABS, DWPI, VEN, SIPOABS, CNTXT, TWTXT, GBTXT, SGTXT, EPTXT, USTXT, CHTXT, WOTXT, CATXT, ATTXT, KRTXT, LEXTXT, RUTXT, DETXT, JPTXT, CNKI, 万方, WANFANG, ISI Web of Science, PubMed, 百度学术, BAIDU, GenBank, EMBL, DDBJ, 中国专利生物序列检索系统, Chinese Patent Biological Sequence Retrieval System, STN: CLDN18.2, CLD18A2, CNDN18, 免疫球蛋白单可变结构域, 单结构域抗体, 嵌合抗体, 结合分子, 抗原结合片段, 人源化抗体, 单域抗体, 融合, 免疫球蛋白, 荧光蛋白, 胞外结构域, IgG, Fc, NA4-S, VHH, Claudin, single domain antibody, sdAb, ECD, CDR1, CDR2, CDR3, SEQ ID NOs: 1-3, 10. |

| C. DOCUMENTS CONSIDERED TO BE RELEVANT | | |
|---|---|---|
| Category* | Citation of document, with indication, where appropriate, of the relevant passages | Relevant to claim No. |
| PX | CN 112480248 A (SANYOU BIOPHARMACEUTICALS CO., LTD.) 12 March 2021 (2021-03-12)<br>claims 1-20, and description, paragraphs 4-321 | 1-20 (all in part) |
| X | EP 2852408 A1 (GANYMED PHARMACEUTICALS AG et al.) 01 April 2015 (2015-04-01)<br>clams 1-18, and description, paragraphs 25-297 | 12-20 (all in part) |
| X | CN 107667118 A (GANYMED PHARMACEUTICALS AG; TRON-TRANSLATIONALE ONKOLOGIE AN DER UNIVERSIT TSMEDIZIN DER JOHANNES GUTENBERG-UNIVERSIT MAINZ) 06 February 2018 (2018-02-06)<br>claims 1-68, and description, paragraphs 7-529 | 12-20 (all in part) |
| X | CN 111961135 A (ETINPRO (BEIJING) CO., LTD.) 20 November 2020 (2020-11-20)<br>claims 1-9, and description, paragraphs 6-91 | 12-20 (all in part) |
| X | CN 109762067 A (BEIJING MABWORKS BIOTECH CO., LTD.) 17 May 2019 (2019-05-17)<br>claims 1-18, and description, paragraphs 13-257 | 12-20 (all in part) |

| ☑ Further documents are listed in the continuation of Box C. | ☑ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **25 January 2022** | **15 February 2022** |

| Name and mailing address of the ISA/CN | Authorized officer |
|---|---|
| **China National Intellectual Property Administration (ISA/ CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| | International application No. |
|---|---|
| | **PCT/CN2021/132218** |

### C.     DOCUMENTS CONSIDERED TO BE RELEVANT

| Category* | Citation of document, with indication, where appropriate, of the relevant passages | Relevant to claim No. |
|---|---|---|
| X | CN 111836644 A (PHANES THERAPEUTICS, INC.) 27 October 2020 (2020-10-27) claims 1-23, and description, paragraphs 9-375 | 12-20 (all in part) |
| X | CN 111848809 A (L&L BIOPHARMA, CO., LTD.) 30 October 2020 (2020-10-30) claims 1-19, and description, paragraphs 5-201 | 12-20 (all in part) |
| X | CN 111518214 A (L&L BIOPHARMA, CO., LTD.) 11 August 2020 (2020-08-11) claims 1-17, and embodiments 1-13 | 12-20 (all in part) |
| X | CN 111867630 A (L&L BIOPHARMA, CO., LTD.) 30 October 2020 (2020-10-30) claims 1-33, and embodiments 1-11 | 12-20 (all in part) |
| X | WOLL, S. et al. "Claudin 18.2 is a target for IMAB362 antibody in pancreatic neoplasms" *Int J Cancer,* Vol. 3, No. 134, 01 February 2014 (2014-02-01), ISSN: 1097-0215, abstract, p. 731, right column, paragraph 2 to p. 738, lef column, paragraph 2 | 12-20 (all in part) |
| A | 徐良额 等 (XU, Liang'e et al.). "CLDN18.2蛋白在恶性肿瘤治疗中的研究进展 (Advances of CLDN18.2 Protein in the Therapy of Malignant Tumors)" 中国肿瘤临床 *(Chinese Journal of Clinical Oncology),* Vol. 46, No. 6, 30 March 2019 (2019-03-30), abstract, p. 311, right column, paragraph 2 to p. 314, right column, paragraph 2 | 1-20 (all in part) |

Form PCT/ISA/210 (second sheet) (January 2015)

70

## INTERNATIONAL SEARCH REPORT

International application No.

**PCT/CN2021/132218**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed:

   ☑ in the form of an Annex C/ST.25 text file.

   ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

   ☐ in the form of an Annex C/ST.25 text file (Rule 13*ter*.1(a)).

   ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

71

## INTERNATIONAL SEARCH REPORT

International application No.

**PCT/CN2021/132218**

| Box No. III | Observations where unity of invention is lacking (Continuation of item 3 of first sheet) |

This International Searching Authority found multiple inventions in this international application, as follows:

[1] Invention 1: claims 1-20 (all in part), relating to a CLDN18.2 binding molecule and a corresponding chimeric antibody, nucleic acid, expression vector, host cell, pharmaceutical composition, kit, and preparation method, and a pharmaceutical use thereof, wherein the antibody contains at least one immunoglobulin single variable domain, and the immunoglobulin single variable domain contains CDR1 as shown in SEQ ID NO. 1, CDR2 as shown in SEQ ID NO. 2, and CDR3 as shown in SEQ ID NO. 3, or CDR1 as shown in SEQ ID NO. 30, CDR2 as shown in SEQ ID NO. 31, and CDR3 as shown in SEQ ID NO. 32.

[2] Invention 2: claims 1-20 (all in part), relating to a CLDN18.2 binding molecule and a corresponding chimeric antibody, nucleic acid, expression vector, host cell, pharmaceutical composition, kit, and preparation method, and a pharmaceutical use thereof, wherein the antibody contains at least one immunoglobulin single variable domain, and the immunoglobulin single variable domain contains CDR1 as shown in SEQ ID NO. 4, CDR2 as shown in SEQ ID NO. 5, and CDR3 as shown in SEQ ID NO. 6, or CDR1 as shown in SEQ ID NO. 37, CDR2 as shown in SEQ ID NO. 38, and CDR3 as shown in SEQ ID NO. 39.

[3] Invention 3: claims 1-20 (all in part), relating to a CLDN18.2 binding molecule and a corresponding chimeric antibody, nucleic acid, expression vector, host cell, pharmaceutical composition, kit, and preparation method, and a pharmaceutical use thereof, wherein the antibody contains at least one immunoglobulin single variable domain, and the immunoglobulin single variable domain contains CDR1 as shown in SEQ ID NO. 7, CDR2 as shown in SEQ ID NO. 8, and CDR3 as shown in SEQ ID NO. 9, or CDR1 as shown in SEQ ID NO. 45, CDR2 as shown in SEQ ID NO. 46, and CDR3 as shown in SEQ ID NO. 47.

[4] Invention 4: claims 1-20 (all in part), relating to a CLDN18.2 binding molecule and a corresponding chimeric antibody, nucleic acid, expression vector, host cell, pharmaceutical composition, kit, and preparation method, and a pharmaceutical use thereof, wherein the antibody contains at least one immunoglobulin single variable domain, and the immunoglobulin single variable domain contains CDR1 as shown in SEQ ID NO. 30, CDR2 as shown in SEQ ID NO. 31, and CDR3 as shown in SEQ ID NO. 33.

[5] Invention 5: claims 1-20 (all in part), relating to a CLDN18.2 binding molecule and a corresponding chimeric antibody, nucleic acid, expression vector, host cell, pharmaceutical composition, kit, and preparation method, and a pharmaceutical use thereof, wherein the antibody contains at least one immunoglobulin single variable domain, and the immunoglobulin single variable domain contains CDR1 as shown in SEQ ID NO. 34, CDR2 as shown in SEQ ID NO. 35, and CDR3 as shown in SEQ ID NO. 36.

[6] Invention 6: claims 1-20 (all in part), relating to a CLDN18.2 binding molecule and a corresponding chimeric antibody, nucleic acid, expression vector, host cell, pharmaceutical composition, kit, and preparation method, and a pharmaceutical use thereof, wherein the antibody contains at least one immunoglobulin single variable domain, and the immunoglobulin single variable domain contains CDR1 as shown in SEQ ID NO. 37, CDR2 as shown in SEQ ID NO. 38, and CDR3 as shown in SEQ ID NO. 42.

[7] Invention 7: claims 1-20 (all in part), relating to a CLDN18.2 binding molecule and a corresponding chimeric antibody, nucleic acid, expression vector, host cell, pharmaceutical composition, kit, and preparation method, and a pharmaceutical use thereof, wherein the antibody contains at least one immunoglobulin single variable domain, and the immunoglobulin single variable domain contains CDR1 as shown in SEQ ID NO. 37, CDR2 as shown in SEQ ID NO. 43, and CDR3 as shown in SEQ ID NO. 44.

[8] ...

[9] Invention 51: claims 1-20 (all in part), relating to a CLDN18.2 binding molecule and a corresponding chimeric antibody, nucleic acid, expression vector, host cell, pharmaceutical composition, kit, and preparation method, and a pharmaceutical use thereof, wherein the antibody contains at least one immunoglobulin single variable domain, and the immunoglobulin single variable domain contains CDR1 as shown in SEQ ID NO. 45, CDR2 as shown in SEQ ID NO. 46, and CDR3 as shown in SEQ ID NO. 39.

[10] Invention 52: claims 1-20 (all in part), relating to a CLDN18.2 binding molecule and a corresponding chimeric antibody, nucleic acid, expression vector, host cell, pharmaceutical composition, kit, and preparation method, and a pharmaceutical use thereof, wherein the antibody contains at least one immunoglobulin single variable domain, and the immunoglobulin single variable domain contains a difference of amino acid addition, deletion or substitution of no more than 2 amino acids on the sequences shown in the above CDR1-3 combination.

[11] The common technical feature between the inventions is the CLDN18.2 binding molecule. However, the prior art document 1 (CN109762067Ac, published on 17 May 2019) discloses an antibody binding to human

Form PCT/ISA/210 (continuation of first sheet) (January 2015)

## INTERNATIONAL SEARCH REPORT

International application No.

**PCT/CN2021/132218**

| Box No. III | Observations where unity of invention is lacking (Continuation of item 3 of first sheet) |
|---|---|

Claudin18.2 (see claims 1-18); the prior art document 2 (CN111961135A, published on 20 November 2020) discloses an antibody specifically binding to human dense protein 18.2 (see claims 1-9); and the prior art document 3 (CN111836644A, published on 27 October 2020) discloses an anti- CLDN18.2 antibody (see claims 1-23). Hence, the common technical feature is disclosed in the prior art. Therefore, the inventions do not share a same or corresponding special technical feature that makes a contribution over the prior art, and therefore, said claims do not comply with the requirement of unity of invention as defined in PCT Rule 13.1. Considering that the applicant's response to the notice of payment of additional fees and objection fees as applicable dated 23 December 2021 expects the examiner to make a search report for CDR1 as shown in SEQ ID NO. 1 and CDR2 as shown in SEQ ID NO. 2, and the CLDN18.2 binding molecule of CDR3 as shown in SEQ ID NO. 3, the examiner made this search report for this group of inventions according to principles of a request.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-20 (all in part)**

**Remark on Protest** ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

<div align="center">

**INTERNATIONAL SEARCH REPORT**
Information on patent family members

</div>

International application No.

**PCT/CN2021/132218**

| Patent document cited in search report | | | Publication date (day/month/year) | Patent family member(s) | | | Publication date (day/month/year) |
|---|---|---|---|---|---|---|---|
| CN | 112480248 | A | 12 March 2021 | None | | | |
| EP | 2852408 | A1 | 01 April 2015 | PL | 3254695 | T3 | 06 April 2021 |
| | | | | DK | 3254695 | T3 | 30 November 2020 |
| | | | | HR | P20171218 | T1 | 20 October 2017 |
| | | | | PL | 2852408 | T3 | 29 December 2017 |
| | | | | CY | 1119549 | T1 | 07 March 2018 |
| | | | | ME | 02932 | B | 20 April 2018 |
| | | | | EP | 2852408 | B1 | 17 May 2017 |
| | | | | EP | 3791896 | A1 | 17 March 2021 |
| | | | | SI | 2852408 | T1 | 30 October 2017 |
| | | | | EP | 3254695 | A1 | 13 December 2017 |
| | | | | EP | 3254695 | B1 | 09 September 2020 |
| | | | | RS | 56187 | B1 | 30 November 2017 |
| CN | 107667118 | A | 06 February 2018 | MA | 41937 | A | 28 February 2018 |
| | | | | IL | 254085 | D0 | 31 October 2017 |
| | | | | SG | 10202105142V | A | 29 June 2021 |
| | | | | HK | 1247210 | A1 | 21 September 2018 |
| | | | | JP | 2021038255 | A | 11 March 2021 |
| | | | | KR | 20180082325 | A | 18 July 2018 |
| | | | | RU | 2017139490 | A | 15 May 2019 |
| | | | | RU | 2017139490 | A3 | 17 January 2020 |
| | | | | ZA | 201705923 | B | 26 June 2019 |
| | | | | JP | 2018513146 | A | 24 May 2018 |
| | | | | JP | 6800883 | B2 | 16 December 2020 |
| | | | | SG | 11201708271 T | A | 29 November 2017 |
| | | | | CA | 2982401 | A1 | 20 October 2016 |
| | | | | WO | 2016165762 | A1 | 20 October 2016 |
| | | | | AU | 2016249782 | A1 | 02 November 2017 |
| | | | | US | 2018117174 | A1 | 03 May 2018 |
| | | | | WO | 2016166122 | A1 | 20 October 2016 |
| | | | | EP | 3283521 | A1 | 21 February 2018 |
| | | | | MX | 2017013075 | A | 19 February 2018 |
| | | | | BR | 112017018521 | A2 | 17 April 2018 |
| CN | 111961135 | A | 20 November 2020 | None | | | |
| CN | 109762067 | A | 17 May 2019 | CA | 3104383 | A1 | 23 July 2020 |
| | | | | AU | 2019422603 | A1 | 07 January 2021 |
| | | | | CN | 112399974 | A | 23 February 2021 |
| | | | | WO | 2020147321 | A1 | 23 July 2020 |
| | | | | KR | 20210116207 | A | 27 September 2021 |
| | | | | US | 10421817 | B1 | 24 September 2019 |
| | | | | EP | 3683239 | A1 | 22 July 2020 |
| | | | | JP | 2021509889 | A | 08 April 2021 |
| | | | | JP | 6927639 | B2 | 01 September 2021 |
| CN | 111836644 | A | 27 October 2020 | EP | 3762031 | A1 | 13 January 2021 |
| | | | | EP | 3762031 | A4 | 22 December 2021 |
| | | | | US | 2020399364 | A1 | 24 December 2020 |
| | | | | SG | 11202007055 Q | A | 29 September 2020 |
| | | | | KR | 20200129116 | A | 17 November 2020 |
| | | | | CA | 3089653 | A1 | 12 September 2019 |

Form PCT/ISA/210 (patent family annex) (January 2015)

**INTERNATIONAL SEARCH REPORT**
Information on patent family members

International application No.

**PCT/CN2021/132218**

| Patent document cited in search report | | | Publication date (day/month/year) | Patent family member(s) | | | Publication date (day/month/year) |
|---|---|---|---|---|---|---|---|
| | | | | BR | 112020015479 | A2 | 08 December 2020 |
| | | | | WO | 2019173420 | A1 | 12 September 2019 |
| | | | | AU | 2019232762 | A1 | 27 August 2020 |
| | | | | JP | 2021514664 | A | 17 June 2021 |
| | | | | IL | 276830 | D0 | 29 October 2020 |
| CN | 111848809 | A | 30 October 2020 | None | | | |
| CN | 111518214 | A | 11 August 2020 | None | | | |
| CN | 111867630 | A | 30 October 2020 | JP | 2021527441 | A | 14 October 2021 |
| | | | | US | 2021230272 | A1 | 29 July 2021 |
| | | | | WO | 2019242505 | A1 | 26 December 2019 |
| | | | | EP | 3808376 | A1 | 21 April 2021 |
| | | | | EP | 3808376 | A4 | 01 September 2021 |

Form PCT/ISA/210 (patent family annex) (January 2015)

## REFERENCES CITED IN THE DESCRIPTION

### Patent documents cited in the description

- CN 202011332200
- WO 03048731 A
- WO 9404678 A
- US 5500362 A
- US 5821337 A
- US 4399216 A
- US 4634665 A
- US 5179017 A
- US 4767704 A
- US 4657866 A
- US 4927762 A
- US 4560655 A
- US 5122469 A
- WO 9003430 A
- WO 8700195 A
- US 30985 A
- US 20180127489 A1

### Non-patent literature cited in the description

- **HAMERS-CASTERMAN C ; ATARHOUCH T ; MUYLDERMANS S ; ROBINSON G ; HAMERS C ; SONGA EB ; BENDAHMAN N ; HAMERS R.** Naturally occurring antibodies devoid of light chains. *Nature,* 1993, vol. 363 (6428), 446-448
- **ABBAS et al.** Cellular and Molecular Immunology. W.B. Saunders Company, 2010
- **SAMBROOK J ; RUSSELL D.** Molecular Cloning: A Laboratory Manual,. Cold Spring Harbour Laboratory Press, 2000
- **AUSUBEL et al.** Short Protocols in Molecular Biology: A Compendium of Methods from Current Protocols in Molecular Biology. Wiley, John & Sons, Inc, 2002
- **HARLOW ; LANE.** Using Antibodies: A Laboratory Manual. Cold Spring Harbour Laboratory Press, 1998
- **COLIGAN et al.** Short Protocols in Protein Science. Wiley, John & Sons, Inc, 2003
- **KABAT.** Sequences of Proteins of Immunological Interest. National Institutes of Health, 1987
- **CHOTHIA ; LESK.** *J. Mol. Biol.,* 1987, vol. 196, 901-917
- **CHOTHIA et al.** *Nature,* 1989, vol. 342, 878-883
- Epitope Mapping Protocols in Methods in Molecular Biology. 1996, vol. 66
- **MAKRIDES, S.C.** *Protein Expr. Purif.,* 1999, vol. 17, 183-202
- **GEISSE, S et al.** *Protein Expr. Purif.,* 1996, vol. 8, 271-282
- **KAUFMAN, R.J.** *Mol. Biotechnol,* 2000, vol. 16, 151-160
- **R. G.** *Drug Res,* 1998, vol. 48, 870-880
- Computational Molecular Biology. Oxford University Press, 1988
- Biocomputing Informatics and Genome Projects. Academic Press, 1993
- Computer Analysis of Sequence Data. Humana Press, 1994
- **VON HEINJE G.** Sequence Analysis in Molecular Biology. Academic Press, 1987
- Sequence Analysis Primer. M. Stockton Press, 1991
- **CARILLO et al.** SIAMJ. Applied Math. 1988, vol. 48, 1073
- **GRAHAM et al.** *Virology,* 1973, vol. 52, 456
- **SAMBROOK et al.** Molecular Cloning: A Laboratory Manual. 2001
- **DAVIS et al.** Basic Methods in Molecular Biology. Elsevier, 1986
- **CHU et al.** *Gene,* 1981, vol. 13, 197
- **FLOWMETRIC.** *Sorting Out Fluorescence Activated Cell Sorting,* 09 November 2017
- Remington's Pharmaceutical Sciences. Mack Publishing Company, 1995
- **BARBAS et al.** *Proc. Natl. Acad. Sci. USA,* 1991, vol. 88, 7978-7982
- **MARKS et al.** *Bio/Technology,* 1992, vol. 10, 779-783
- **BARBAS et al.** *Proc. Nat. Acad. Sci. USA,* 1994, vol. 91, 3809-3813
- **SCIER et al.** *Gene,* 1995, vol. 169, 147-155
- **HAWKINS et al.** *J. Mol. Biol.,* 1992, vol. 226, 889-896
- **WARD et al.** *Nature,* 1989, vol. 341, 544-546
- **WESOLOWSKI et al.** Single Domain Antibodies: Promising Experimental and Therapeutic Tools in Infection and Immunity. *Med Microbiol Immunol,* 2009, vol. 198, 157-174
- **R. VAN DER LINDEN et al.** *Journal of Immunological Methods,* 2000, vol. 240, 185-195

- **LI et al.** *J Biol Chem.,* 2012, vol. 287, 13713-13721
- **DEFFAR et al.** *African Journal of Biotechnology,* 17 June 2009, vol. 8 (12), 2645
- **RAVETCH ; KINET.** *Annu. Rev. Immunol,* 1991, vol. 9, 457-92
- **CLYNES et al.** *PNAS (USA),* 1998, vol. 95, 652-656
- **GAZZANO-SANTORO et al.** *J. Immunol. Methods,* 1996, vol. 202, 163
- **CHOTHIA et al.** *Nature,* 1989, vol. 342, 877-883
- **AL-LAZIKANI et al.** Standard Conformations for the Canonical Structures of Immunoglobulins. *Journal of Molecular Biology,* 1997, vol. 273, 927-948
- **KABAT et al.** Sequences of Proteins of Immunological Interest. Department of Health and Human Services, 1987
- Antibody Engineering. Springer, 2001
- **DINARELLO et al.** Current Protocols in Immunology. John Wiley and Sons Inc, 2000
- **A.C. MARTIN.** *Department of Biochemistry & Molecular Biology University College London, London, England*
- **RETTER et al.** *Nucl. Acids Res,* 2005, vol. 33, D671-D674
- Protein Sequence and Structure Analysis of Antibody Variable Domains. Antibody Engineering Lab Manual. Springer-Verlag
- **E. MEYERS ; W. MILLER.** *Comput. Appl. Biosci.,* 1988, vol. 4, 11-17
- **NEEDLEMAN ; WUNSCH.** *J. Mol. Biol.,* 1970, vol. 48
- **ALTSCHUL et al.** *J. Mol. Biol.,* 1990, vol. 215, 403-10
- **ALTSCHUL et al.** *Nucleic Acids Res.,* 1997, vol. 25 (17), 3389-3402
- **BRUMMELL et al.** *Biochem.,* 1993, vol. 32, 1180-1187
- **KOBAYASHI et al.** *Protein Eng.,* 1999, vol. 12 (10), 879-884
- **BURKS et al.** *Proc. Natl. Acad. Sci. USA,* 1997, vol. 94, 412-417
- **BRUMMELL et al.** *Biochem,* 1993, vol. 32, 1180-8
- **DE WILDT et al.** *Prot. Eng,* 1997, vol. 10, 835-41
- **KOMISSAROV et al.** *J. Biol. Chem.,* 1997, vol. 272, 26864-26870
- **HALL et al.** *J. Immunol.,* 1992, vol. 149, 1605-12
- **KELLEY ; O' CONNELL.** *Biochem.,* 1993, vol. 32, 6862-35
- **ADIB-CONQUY et al.** *Int. Immunol.,* 1998, vol. 10, 341-6
- **BEERS et al.** *Clin. Can. Res,* 2000, vol. 6, 2835-43
- **HAM et al.** *Meth. Enz.,* 1979, vol. 58, 44
- **BARNES et al.** *Anal. Biochem,* 1980, vol. 102, 255
- **CARTER et al.** *Bio/Technology,* 1992, vol. 10, 163-167